# EUROPÄISCHE PATENTSCHRIFT

(11) **EP 0 771 813 B1**
(45) Veröffentlichungstag und Bekanntmachung des Hinweises auf die Patenterteilung: **16.01.2002**
(21) Anmeldenummer: 96116861.4
(22) Anmeldetag: 21.10.1996
(51) Int. Cl.: C07F 9/6561, A61K 31/675

(54) **Alkylxanthinphosphonate und Alkylxanthinphosphinoxide und deren Verwendung als Arzneimittel**
Alkylxanthine phosphonates and alkylxanthine phosphine oxydes and their use as medicines
Phosphonates d'alkylxanthines et oxydes de phosphine d'alkylxanthines et leur utilisation comme médicaments

(30) Priorität: 02.11.1995 DE 19540798
(43) Veröffentlichungstag der Anmeldung: 07.05.1997
(73) Patentinhaber: HOECHST AKTIENGESELLSCHAFT, 65929 Frankfurt am Main (DE)
(72) Erfinder: Billen, Günter, Dr., 65527 Niedernhausen (DE); Okyayuz-Baklouti, Ismahan, Dr., 65197 Wiesbaden (DE); Anagnostopulos, Hiristo, DB., 65193 Wiesbaden (DE); Müllner, Stefan, Dr., 65239 Hochheim (DE)

(56) Entgegenhaltungen:
- EP-A- 0 343 133
- EP-A- 0 557 876
- EP-A- 0 632 048
- WO-A-87/00523

## Beschreibung

Die vorliegende Erfindung betrifft Xanthinderivate mit mindestens einer Alkylphosphonat- oder Alkylphosphinoxidgruppe in 1- oder 7-Stellung. Diese Verbindungen eignen sich zur Behandlung von Muskelatrophie, Muskeldystrophie, Kachexie, Sepsis, septischer Schock, endotoxischer Schock, Systemic Inflammation Response Syndrome (SIRS), Adult Respiratory Distress Syndrome (ARDS), zerebrale Malaria, chronische Lungenentzündung, Lungensarkoidose, Reperfusionsschäden, Narbenbildung, Darmentzündungen, Kolitis Ulcerosa, Kachexie, infolge von Infektionen, Acquired Immune Deficiency Syndrom (AIDS), Krebs, Trauma und anderen Erkrankungen mit erhöhtem Proteinverlust, peripheren Durchblutungsstörungen, Erkrankungen mit gestörter Leukozyten-Adhäsion, sowie Erkrankungen, die mit einer erhöhten oder unregulierten Tumor Nekrose Faktor (TNF) - Produktion einhergehen wie rheumatoide Arthritis, rheumatoide Spondylitis, Osteoarthritis und andere arthritische Erkrankungen. Ferner werden Verfahren zur Herstellung der erfindungsgemäßen Xanthinderivate beschrieben.

Einige Oxoalkyl- und Hydroxyalkylxanthine wirken durchblutungsfördernd und können bei mitochondrialen Myopathien eingesetzt werden (WO 87/00523).

Bisher sind keine Arzneimittel bekannt, die es ermöglichen den bei vielen Erkrankungen auftretenden Funktionsverlust der Muskulatur zu reduzieren. Bekannte Arzneimittel weisen eine Erhöhung der Proteinmasse oder Hemmung des Proteinabbaus auf (EP 464 932; EP 516 594; Drugs of the Future 11: 927 - 930, 1986) oder zeigen eine Verschiebung des Protein/Fett Verhältnisses zugunsten des Proteinanteils (EP 375 791; EP 290 122; EP 254 856). Dies läßt jedoch keinen direkten Rückschluß auf die Funktion der betroffenen Muskeln zu und ist auch dann zu beobachten, wenn keine Erkrankung zugrunde liegt.

Einige der erfindungsgemäß verwendbaren Verbindungen sind bekannt, doch ist ihre Verwendung als Arzneimittel nicht erkannt worden (J. Am. Chem. Soc. 77, Seiten 2386 bis 2388 (1955)).

Es wurde nun gefunden, daß die Verbindung der Formel I, den bei verschiedenen Erkrankungen auftretenden Funktionsverlust der Muskulatur reduziert und die Mortalität durch Lipopolysaccharid (LPS) induzierten Endotoxin Schock deutlich verringert. Ferner wurde gefunden, daß die Verbindung der Formel I die Effekte der Entzündungsmediatoren Substanz P (SP), Leukotrien D₄ (LTD₄) und Platelet activating factor (PAF) antagonisiert, sowie eine Phosphodiesterase Hemmung ausübt und sich daher zur Prophylaxe und/oder Therapie der oben genannten Erkrankungen besonders eignet.

Die Erfindung betrifft eine Verbindung der Formel I 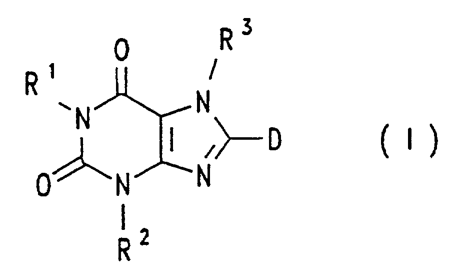 und/oder ein physiologisch verträgliches Salz der Verbindung der Formel I und/oder eine gegebenenfalls stereoisomere Form der Verbindung der Formel I,
wobei
R¹ und R³ gleich oder verschieden sind und mindestens einer der Reste R¹ und
R³ für
   a) einen Rest der Formel XI steht, 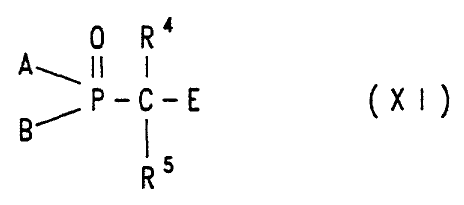 worin R⁴
      und R⁵ gleich oder verschieden sind und unabhängig voneinander
      1.1 Wasserstoffatom,
      1.2 Hydroxyl oder
      1.3 (C₁-C₆)-Alkyl darstellen,
      A und B gleich oder verschieden sind und unabhängig voneinander
      2.1 (C₁-C₄)-Alkyl,
      2.2 (C₁-C₆)-Alkoxy,
      2.3 Hydroxyl oder
      2.4 Benzyloxy darstellen,
      E eine kovalente Bindung oder ein geradkettiges oder verzweigtes Alkyl mit 1 bis 5 C-Atomen darstellt, und
   b) falls nur einer der Reste R¹ oder R³ die unter a) genannte Bedeutung hat, der andere Rest R¹ oder R³ für
      1) Wasserstoffatom,
      2) (C₁-C₆)-Alkyl, geradkettig oder verzweigt,
      3) (C₂-C₆)-Alkyl, geradkettig oder verzweigt, wobei die Kohlenstoffkette durch 1 oder 2 Sauerstoffatome unterbrochen ist und 2 aneinander gebundene Sauerstoffatome ausgeschlossen sind,
      4) (C₁-C₈)-Alkyl, geradkettig oder verzweigt, substituiert durch
         4.1 eine Oxogruppe oder
         4.2 eine oder zwei Hydroxylgruppen,
      5) (C₂-C₆)-Alkenyl, geradkettig oder verzweigt,
      6) Benzyl,
      7) Benzyl, ein- bis fünffach substituiert durch
         7.1 (C₁-C₄)-Alkyl oder
         7.2 (C₁-C₄)-Alkoxy,
      8) (C₃-C₆)-Cycloalkyl oder
      9) (C₁-C₄)-Alkyl-(C₃-C₆)-cycloalkyl, steht, und
      R² für
      1) Wasserstoffatom,
      2) (C₁-C₆)-Alkyl, geradkettig oder verzweigt,
      3) (C₂-C₆)-Alkyl, geradkettig oder verzweigt, wobei die Kohlenstoffkette durch 1 oder 2 Sauerstoffatome unterbrochen ist und 2 aneinander gebundene Sauerstoffatome ausgeschlossen sind,
      4) Benzyl,
      5) Benzyl, ein- bis fünffach substituiert durch
         5.1 (C₁-C₄)-Alkyl oder
         5.2 (C₁-C₄)-Alkoxy,
      6) Phenyl,
      7) (C₃-C₆)-Cycloalkyl oder
      8) (C₁-C₄)-Alkyl-(C₃-C₆)-Cycloalkyl, steht, und
      D für
      1) Wasserstoffatom oder
      2) Fluor-, Chlor-, Brom- oder Jodatom, steht,
wobei die Verbindungen [4-(1,3-Dimethylxanthin-7-yl)-butyl]-phosphonsäurediethylester, 5-(1,3-Dimethoxyxanthin-7-yl)-pentylphosphonsäure, 4-(1,3-Dimethylxanthin-7-yl)-butyl-phosphonsäure und 3-(1,3-Dimethylxanthin-7-yl)-propylphosphonsäure ausgeschlossen sind.

Bevorzugt ist eine Verbindung der Formel I und/oder ein physiologisch verträgliches Salz der Verbindung der Formel I und/oder eine gegebenenfalls stereoisomere Form der Verbindung der Formel I, wobei
A und B für
1) Hydroxyl,
2) C₂-Alkoxy oder
3) Methyl stehen.

Insbesondere bevorzugt ist eine Verbindung der Formel I, wobei R² für
1) Wasserstoffatom,
2) (C₂-C₆)-Alkyl, geradkettig oder verzweigt, wobei die Kohlenstoffkette durch 1 Sauerstoffatom unterbrochen ist,
3) Benzyl,
4) Cyclopropyl oder
5) -CH₂-Cyclopropyl, steht.

Ferner ist bevorzugt eine Verbindung der Formel I, wobei einer der Reste R¹ oder R³ für einen Rest der Formel XI steht und der andere Rest R¹ oder R³ für
1) Wasserstoffatom,
2) (C₂-C₆)-Alkyl, geradkettig oder verzweigt, wobei die Kohlenstoffkette durch 1 oder 2 Sauerstoffatome unterbrochen ist und 2 aneinander gebundene Sauerstoffatome ausgeschlossen sind,
3) Benzyl oder
4) -CH₂-Cyclopropyl, steht.

Die Erfindung betrifft weiterhin Verfahren zur Herstellung der Verbindung der Formel I, wobei eine Ausführungsform darin besteht, daß man
A) eine Verbindung der Formel III, 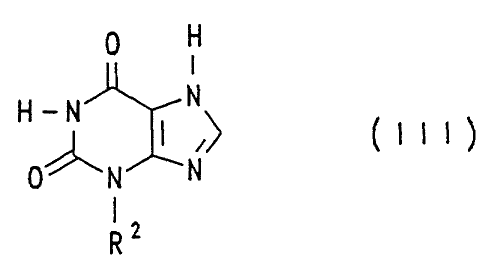 wobei R² die in Formel I genannte Bedeutung hat,
   in Gegenwart basischer Mittel mit einem Alkylierungsmittel der Formel II, 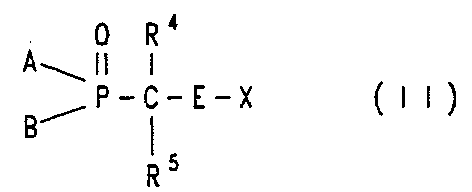 wobei X Chlor, Brom, Jod oder Sulfonsäureesterrest bedeutet und R⁴, R⁵, A, B und E die in Formel XI genannte Bedeutung haben,
   zu einer Verbindung der Formel I umsetzt,
   wobei R¹ Wasserstoffatom bedeutet und R³ ein Rest der Formel XI 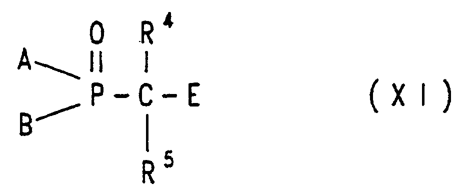 ist, und R² die in Formel I genannte Bedeutung hat, oder
B) eine gemäß A) hergestellte Verbindung der Formel 1 in Gegenwart basischer Mittel mit einem Alkylierungsmittel der Formel II, wobei X, R⁴, R⁵, A, B und E wie in Verfahren A) definiert sind, zu einer Verbindung der Formel I umsetzt, wobei R¹ und R³ gleich oder verschieden sind und für einen Rest der Formel XI stehen, oder
C) eine gemäß A) hergestellte Verbindung der Formel I in Gegenwart basischer Mittel mit einer Verbindung R⁶-X, wobei
   R⁶ für
   1) Wasserstoffatom,
   2) (C₁-C₆)-Alkyl, geradkettig oder verzweigt,
   3) (C₂-C₆)-Alkyl, geradkettig oder verzweigt, wobei die Kohlenstoffkette durch 1 oder 2 Sauerstoffatome unterbrochen ist und 2 aneinander gebundene Sauerstoffatome ausgeschlossen sind,
   4) (C₁-C₆)-Alkyl, geradkettig oder verzweigt, substituiert durch
      4.1 eine Oxogruppe oder
      4.2 eine oder zwei Hydroxylgruppen,
   5) (C₂-C₆)-Alkenyl, geradkettig oder verzweigt,
   6) Benzyl,
   7) Benzyl, ein- bis fünffach substituiert durch
      7.1 (C₁-C₄)-Alkyl oder
      7.2 (C₁-C₄)-Alkoxy,
   8) (C₃-C₆)-Cycloalkyl oder
   9) (C₁-C₄)-Alkyl-(C₃-C₆)-cycloalkyl, steht, und
   X die unter A) genannte Bedeutung hat, zu einer Verbindung der Formel I umsetzt, wobei
   R¹ die Bedeutung von R⁶ hat, R² gemäß der Verbindung der Formel I definiert ist und R³ die Bedeutung der Formel II hat, oder
D) eine Verbindung der Formel IV, 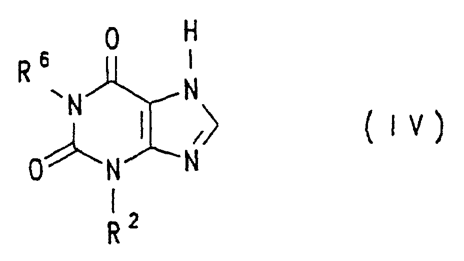 wobei R⁶ die unter C) genannte Bedeutung hat und R² die in Formel I genannte Bedeutung hat, in Gegenwart basischer Mittel mit einem Alkylierungsmittel der Formel II zu einer Verbindung der Formel I umsetzt, wobei R¹ die Bedeutung von R⁶ hat, sowie R² die in Formel I und R³ die in Formel XI genannte Bedeutung haben, oder
E) eine Verbindung der Formel V 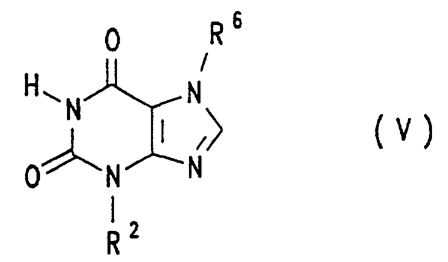 wobei R⁶ die unter C) genannte Bedeutung und R² die in Formel I genannte Bedeutung haben, in Gegenwart basischer Mittel mit einem Alkylierungsmittel der Formel II zu einer Verbindung der Formel I umsetzt, wobei R¹ die in Formel XI genannte Bedeutung hat, R² die in Formel I genannte Bedeutung hat und R³ die Bedeutung von R⁶ hat, oder
F) eine Verbindung der Formel I, wobei mindestens ein R¹, R² und R³ ein Benzyl-, Alkoxymethyl- oder Alkoxyalkoxymethylrest ist und mindestens ein R¹ und R³ ein Rest der Formel XI ist, unter reduzierenden oder hydrolytischen Bedingungen den Benzyl-, Alkoxymethyl- oder Alkoxyalkoxymethylrest der Formel I abspaltet, oder
G) eine Verbindung der Formel VI, 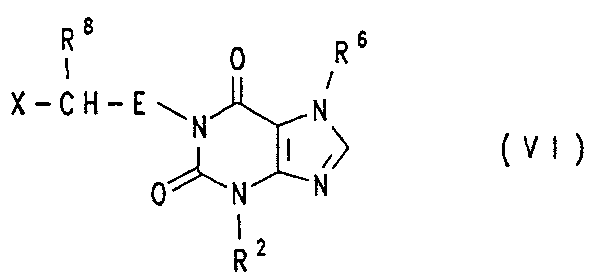 wobei
   - R⁶: die unter C) genannte Bedeutung hat,
   - R²: die in Formel I genannte Bedeutung hat,
   - E: die in Formel XI genannte Bedeutung hat,
   - R⁸: Wasserstoffatom oder (C₁-C₄)-Alkyl ist und
   - X: Chlor, Brom, Jod oder ein Sulfonsäureesterrest ist,
   in Gegenwart einer starken Base wie Natriumhydrid, Butyllithium oder Lithiumdiisopropylamid in einem inerten Lösungsmittel mit einer Verbindung der Formel VIII, 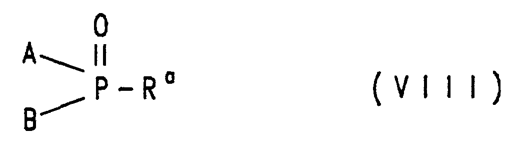 wobei A und B die in Formel I genannte Bedeutung haben und
   R^{a} Wasserstoffatom oder (C₁-C₄)-Alkyl ist, zu einer Verbindung der Formel I umsetzt, wobei R¹ die in Formel XI genannte Bedeutung hat, R² die in Formel I genannte Bedeutung hat und R³ die Bedeutung von R⁶ hat, oder
H) eine Verbindung der Formel VI, wobei R², R⁶, R⁸, E und X die unter G) genannte Bedeutung haben mit einer Verbindung der Formel IX,

   P(OR¹⁰)₃ (IX)

   wobei P Phosphor, O Sauerstoff und R¹⁰ ein (C₁-C₄)-Alkyl sind, in eine Verbindung der Formel I umsetzt, wobei R¹ die in Formel XI genannte Bedeutung hat, R² die in Formel I genannte Bedeutung hat und R³ die Bedeutung von R⁶ hat, oder
I) eine Verbindung der Formel I, wobei R¹, R², R³, E und D die in Formel I genannte Bedeutung haben und A und B für (C₁-C₄)-Alkyl oder (C₁-C₄)-Alkoxy stehen, mit Mitteln wie Mineralsäuren oder Silylhalogenide in die entsprechenden Phosphonsäurederivate der Formel I überführt, oder
K) eine Verbindung der Formel I, wobei R¹, R², R³, E und D die in Formel I genannte Bedeutung haben und A oder B für eine Benzyloxygruppe steht, selektiv in den entsprechenden Phosphonsäurehalbester überführt, oder
L) eine Verbindung der Formel I, wobei R¹, R², R³, E, A und B die in Formel I genannte Bedeutung haben und D für Wasserstoffatom steht halogeniert, wobei eine entsprechende Verbindung der Formel I entsteht in der D für Fluor, Chlor, Brom oder Jod steht, oder
M) die nach Verfahren A) bis L) hergestellte Verbindung der Formel I entweder in freier Form isoliert oder im Falle des Vorliegens von sauren oder basischen Gruppen gegebenenfalls in physiologisch verträgliche Salze umwandelt.

Die Herstellung der als Ausgangsstoffe verwendeten 3-Alkyl-, 1,3-Dialkyl oder 3,7-Dialkylxanthine ist größtenteils bekannt oder sie lassen sich nach literaturbekannten Methoden leicht darstellen (Ken-ichi Miyamoto et al., J. Med. Chem. 35 (1992), 4039 - 4044).
Die Umsetzung der mono- oder disubstituierten Xanthinderivate der Formel III, IV oder V mit den betreffenden Alkylierungsmitteln der Formel II oder R6-X erfolgt gewöhnlich an einem gegenüber den Reaktionsteilnehmern inerten Verteilungs- oder Lösungsmittel. Als solche kommen vor allem dipolar aprotische Solvenzien, beispielsweise Formamid, Dimethylformamid, Dimethylacetamid, Dimethylsulfoxid, Aceton oder Butanol in Frage; es können aber auch Alkohole wie Methanol, Ethylenglykol und dessen Mono- oder Di(C₁-C₄)alkylether, Ethanol, Propanol, Isopropanol und die verschiedenen Butanole; Kohlenwasserstoffe wie Benzol, Toluol oder Xylol; halogenierte Kohlenwasserstoffe wie Dichlormethan, oder Chloroform; Pyridin sowie Mischungen der genannten Lösungsmittel oder deren Gemische mit Wasser Verwendung finden.
Die Alkylierungen werden zweckmäßig in Gegenwart eines basischen Mittels durchgeführt. Hierfür eignen sich beispielsweise Alkali- oder Erdalkalihydroxide, -carbonate, -hydride, -alkoholate und organische Basen wie Trialkylamine, quartäre Ammonium- oder Phosphoniumhydroxide und vernetzte Harze mit fixierten, gegebenenfalls substituierten Ammonium- oder Phosphoniumgruppen. Die Xanthinderivate können aber auch unmittelbar in Form ihrer gesondert hergestellten Salze, wie etwa der Alkali-, Erdalkali- oder gegebenenfalls substituierten Ammonium oder Phosphoniumsalze, eingesetzt werden. Weiterhin lassen sich die substituierten Xanthinderivate unter Mithilfe sogenannter Phasentransferkatalysatoren leicht alkylieren.
Bei den voranstehend beschriebenen Verfahrensweisen wird im allgemeinen bei einer Reaktionstemperatur von 0°C bis zum Siedepunkt des jeweils verwendeten Reaktionsmediums gearbeitet, vorzugsweise von 20°C bis 130°C, gegebenfalls bei erhöhtem oder vermindertem Druck, aber gewöhnlich bei Atmosphärendruck, wobei die Reaktionszeit von weniger als einer Stunde bis zu mehreren Stunden betragen kann.
Die Eliminierung von Abgangsgruppen unter Bildung der erfindungsgemäßen Xanthine der Formel I oder deren Vorstufen erfolgt unter Standardbedingungen, die vor allem im Rahmen der Schutzgruppentechnik bei Alkaloid- und Peptidsynthesen entwickelt wurden und somit als weitgehend bekannt vorausgesetzt werden können.
Danach wird die gegebenenfalls im Phenylring substituierte Benzylgruppe vorzugsweise reduktiv abgespalten. Neben der chemischen Reduktion mit Natrium in flüssigen Ammoniak kommt hierfür bevorzugt die Eliminierung durch katalytische Hydrierung in Gegenwart eines Edelmetall-Katalysators in Betracht,
wobei sich häufig der Ersatz von molekularen Wasserstoff durch Ameisensäure oder deren Salze, hier innsbesondere Ammoniumformiat, bewährt. Als Reaktionsmedium dient hierbei gewöhnlich ein niederer Alkohol, unter Zusatz von Ameisensäure oder Ammoniak; Lösungsmittel, wie Dimethylformamid oder Eisessig; aber auch deren Gemische mit Wasser können Verwendung finden. Geeignete Hydrierungskatalysatoren sind vornehmlich Palladium und Palladiumhydroxid auf Aktivkohle oder Bariumsulfat, während andere Edelmetalle wie Platin, Rhodium und Ruthenium aufgrund konkurrierender Kernhydrierung häufig Anlaß zu Nebenreaktionen geben und deshalb nur bedingt einsetzbar sind. Die Hydrogenolyse wird zweckmäßig bei Temperaturen von 20° bis 100°C und unter Atmosphärendruck oder bevorzugt leichtem Überdruck bis zu 10 bar durchgeführt, wobei in der Regel Reaktionszeiten von einigen Minuten bis mehreren Stunden benötigt werden.
Die substituierten Xanthine, die eine Alkoxymethylgruppe tragen, stellen O, N-Acetale dar und lassen sich demzufolge unter den üblichen Bedingungen der sauren Hydrolyse leicht demaskieren. Die Reaktion wird vorteilhaft unter Erwärmen in verdünnten Mineralsäuren, wie Salz- oder Schwefelsäure, gegebenenfalls unter Zusatz von Eisessig, Dioxan, Tetrahydrofuran oder einem niederen Alkohol als Lösungsvermittler, durchgeführt. Mitunter kommen auch Perchlorsäure oder organische Säuren, wie Trifluoressig-, Ameisen- und Essigsäure in Frage. Bei säurelabilen Derivaten hat sich die Verwendung von Pyridiniumtosylat (PPTS) bewährt. Die Reaktionstemperatur sollte bei Spaltung in mineralsaurer Lösung zur Vermeidung von Eliminationen 60°C nicht überschreiten. Die Spaltung der Ethergruppe kann prinzipiell auch mit Hilfe von Lewis-Säuren wie Zinkbromid und Titantetrabromid, in wasserfreien Milieu, vorzugsweise in Dichlormethan oder Chloroform, durchgeführt werden.
Die Halogenierung zu den erfindungsgemäßen 8-Halogenxanthinen erfolgt vorzugsweise dadurch, daß das entsprechende Xanthin mit elementaren Halogen in einem geeigneten Lösungsmittel, beispielsweise Methylenchlorid, Chloroform, Tetrachlorkohlenstoff vorzugsweise in Eisessig, umgesetzt wird. Die als Vorstufen verwendeten Alkylierungsmittel der Formel II sind größtenteils bekannt (B. Helferich et al., Ann. 655 (1962), 59) oder lassen sich in Analogie zu den literaturbekannten Methoden leicht herstellen.
Die gemischten Phosphonsäureester der Formel II, wobei A und B nicht identisch sind, können hergestellt werden, indem man die Methyl- oder Ethylester der entsprechenden Phosphonsäuren der Formel II, mit Säurehalogeniden anorganischer Säuren wie Thionylchlorid oder Phosphorpentachlorid in geeigneten, bevorzugt halogenierten, Lösungsmitteln zu den entsprechenden Monosäurehalogeniden umsetzt und diese, nach destillativer Aufreinigung, mit einem Alkohol in Gegenwart basischer Mittel in die entsprechenden gemischten Ester der Formel III, wobei A und B nicht identisch sind, überführt.

Die Herstellung physiologisch verträglicher Salze aus zur Salzbildung befähigten Verbindungen der Formel einschließlich deren stereoisomeren Formen, erfolgt in an sich bekannter Weise. So bilden Phosphonsäuren und Phosphonsäurehalbester mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder basischen Aminosäuren wie Lysin, Ornithin oder Arginin stabile Alkali-, Erdalkali-, oder gegebenenfalls substituierte Ammoniumsalze.

Die Erfindung betrifft ferner ein Arzneimittel, enthaltend eine Verbindung der Formel I, wobei die Verbindungen [4-(1,3-Dimethylxanthin-7-yl)-butyl]phosphonsäurediethylester, und 4-(1,3-Dimethylxanthin-7-yl)-butyl-phosphonsäure eingeschlossen sind.

Die Erfindung betrifft ferner die Verwendung der Verbindung der Formel I zur Herstellung von Arzneimitteln zur Prophylaxe und Therapie von Muskelatrophie, Muskeldystrophie, Kachexie, Sepsis, septischer Schock, Endotoxin Schock, SIRS, ARDS, zerebrale Malaria, chronische Lungenentzündung, Lungensarkoidose, Reperfusionsschäden, Narbenbildung, Darmentzündungen, Kolitis Ulcerosa, Kachexie, infolge von Infektionen, AIDS, Krebs, Trauma und Erkrankungen mit erhöhtem Proteinverlust, peripheren Durchblutungsstörungen, Erkrankungen mit gestörter Leukozyten-Adhäsion, Erkrankungen, die mit einer erhöhten oder unregulierten TNF-Produktion einhergehen wie rheumatoide Arthritis, rheumatoide Spondylitis, Osteoarthritis und andere arthritische Erkrankungen.

Die Erfindung betrifft auch ein Verfahren zur Herstellung eines Arzneimittels, das dadurch gekennzeichnet ist, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Die erfindungsgemäßen Arzneimittel, die als Wirkstoffe die Verbindungen der allgemeinen Formel I, gegebenenfalls in stereoisomerenreiner Form und/oder als physiologisch verträgliche Salze, entweder für sich allein, beispielsweise in Mikrokapseln, in Mischungen untereinander oder vorzugsweise in Kombination mit geeigneten pharmazeutischen Trägerstoffen, Verdünnungsmitteln und/oder anderen Hilfstoffen enthalten, können intravenös, intramuskulär, parenteral, rektal oder oral verabreicht werden.
Geeignete feste oder flüssige galenische Zubereitungsformen sind beispielsweise Granulate, Pulver, Dragees, Tabletten, (Mikro)kapseln, Suppositorien, Sirupe, Säfte, Suspensionen, Emulsionen, Tropfen oder injezierbare Lösungen sowie Präparate mit protrahierter Wirkstoff-Freigabe, bei deren Herstellung üblicherweise Hilfsmittel, wie Trägerstoffe, Spreng-, Binde-, Überzugs-, Quellungs-, Gleit-, oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, wie Lebertran, Sonnenblumen-, Erdnuß-, oder Sesamöl, Polyethylenglykole und Lösungsmittel, wie etwa steriles Wasser, physiologische Kochsalzlösung und ein- oder mehrwertige Alkohole, z.B. Glycerin genannt. Bei den stark sauer reagierenden Phosphon- und Phosphinsäuren gemäß Formel I wird der Wirkstoff so formuliert, daß er in Salzform mit physiologisch verträglichen pH-Wert vorliegt.
Vorzugsweise werden die pharmazeutischen Präparate in Dosierungseinheiten hergestellt und verabreicht, wobei jede Einheit als aktiven Bestandteil eine bestimmte Dosis mindestens einer Verbindung gemäß Formel I, gegebenenfalls in stereoisomerenreiner und/oder Salzform enthält. Bei festen Dosierungseinheiten, wie Tabletten, Kapseln, Dragees oder Suppositorien, kann diese Dosis bis zu etwa 1000 mg, bevorzugt jedoch etwa 100 bis 600 mg, und bei Injektionslösungen in Ampullenform bis zu etwa 300 mg, vorzugsweise aber etwa 20 bis 200 mg betragen.

Für die Behandlung eines erwachsenen, etwa 70 kg schweren Patienten sind - je nach Wirksamkeit der Verbindungen gemäß Formel I, Tagesdosen von 50 bis 3000 mg Wirkstoff, vorzugsweise etwa 150 bis 1000 mg, bei oraler Verabreichung und etwa 50 bis 1000 mg, bevorzugt etwa 100 bis 500 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Verbindungen der Formel I, ihre gegebenenfalls stereoisomeren Formen und/oder ihre physiologisch verträglichen Salze bei der Herstellung der vorgenannten galenischen Zubereitungsformen auch zusammen mit anderen geeigneten Wirkstoffen, beispielsweise Antithrombotika, Androgenen, Anabolika, Insulinen, Calciumkanal-Blockern, Plasmaexpandern und anderen Vasotherapeutika, formuliert werden.

### Beispiel 1

### [4-(1,3-Dimethylxanthin-7-yl)-butyl]-phosphonsäurediethylester

### a) 4-Chlorbutanphosphonsäurediethylester

### a₁) aus 1-Brom-4-chlorbutan:

Eine Mischung aus 342 g (2,0 mol) 1-Brom-4-chlorbutan und 166,2 g (1,0 mol) Triethylphosphit wurde bei 130°C Ölbadtemperatur unter Rühren und Einleiten von Stickstoff etwa 5 Stunden erhitzt bis etwa 109 g (1,0 mol) Bromethan in eine stark mit Eis gekühlte Vorlage überdestillierte. Das Reaktionsgemisch wurde zuerst am Kugelrohr und anschließend fraktioniert destilliert.
Ausbeute: 137 g (59,9 % der Theorie), Siedepunkt (0,1 mbar) 145 - 152°C C₈H₁₈ClO₃P (Molekulargewicht (MG) = 228,7)

### a₂) aus Diethylphosphit:

Zu einer Lösung von 510 g (4,0 mol) 1,4-Dichlorbutan in 400 ml siedenden Petrolether wurde im Laufe von 3 Stunden unter Rühren eine Lösung von Natriumdiethylphosphit zu getropft, die aus der berechneten Menge Natriumhydrid und 110,5 g Diethylphosphit (0,8 mol) in absolutem Diethylether bereitet worden war. Nach weiteren Erhitzen unter Rückfluß und abdestillieren der leicht flüchtigen Anteile wurde vom gebildeten Natriumchlorid abfiltriert und unter verminderten Druck destilliert.
Ausbeute: 103 g (56,3 % der Theorie), Siedepunkt (0,1 mbar) 145 - 152°C, C₈H₁₈ClO₃P (MG = 228,7)

### b) [4-(1,3-Dimethylxanthin-7-yl)-butyl]-phosphonsäurediethylester

10,9 g (0,05 mol) 1,3-Dimethylxanthin wurden in 150 ml DMF suspendiert, mit 13,7 g (0,06 mol) 4-Chlorbutanphosphonsäurediethylester und 7,0 g (0,05 mol) aktivierten Kaliumcarbonat versetzt und etwa 4 Stunden auf 70° C erhitzt. Anschließend wurde vom Feststoff abfiltriert, das Filtrat unter verminderten Druck eingeengt und der verbleibende ölige Rückstand in Ethylacetat aufgenommen, nochmals filtriert und aus Diisopropylether kristallisiert.
Ausbeute: 13,9 g ( 74% der Theorie), Schmelzpunkt: 122-124 °C, C₁₅H₂₅N₄O₅P (MG = 372,4), ¹H-NMR (CDCl₃) δ = 1,31 (t, 6H); 1,45-2,20 (m, 6H); 3,55 (s, 3H); 3,98 (s, 3H); 3,80-4,15 (m, 6H); 7,50 (s, 1H) ppm

### Beispiel 2

### [4-(3-Methylxanthin-7-yl)-propyl]-phosphonsäurediethylester

### a) 3-Brompropanphosphonsäurediethylester

Eine Mischung aus 605 g (3,0 mol) 1,3-Dibrompropan und 250 g (1,5 mol) Triethylphosphit wurde bei 130°C Ölbadtemperatur, unter Rühren und Einleiten von Stickstoff, etwa 5 Stunden erhitzt, bis etwa 154 g (1,5 mol) Bromethan in eine stark mit Eis gekühlte Vorlage überdestillierte. Das Reaktionsgemisch wurde zuerst am Kugelrohr und anschließend fraktioniert destilliert.
Ausbeute: 178 g (46 % der Theorie), Siedepunkt (1 mbar): 121-125°C C₇H₁₆ClO₃P (MG = 259,1)

### b) [4-(3-Methylxanthin-7-yl)-propyl]-phosphonsäurediethylester

16,6 g (0,1 mol) 3-Methylxanthin wurden in 250 ml DMF suspendiert, mit 31 g (0,12 mol) 3-Brompropanphosphonsäurediethylester und 16,5 g (0,12 mol) aktivierten Kaliumcarbonat versetzt und etwa 6 Stunden auf 90° C erhitzt. Anschließend wurde vom Feststoff abfiltriert, das Filtrat unter verminderten Druck eingeengt und der verbleibende ölige Rückstand über eine Kieselgelsäule chromatographiert (Eluent: Ethylacetat/Methanol 10:1).
Ausbeute: 4,5 g (13 % der Theorie), Schmelzpunkt: 211° C, C₁₃H₂₁N₄O₅P (MG = 344,3), ¹H-NMR (CDCl₃) δ = 1,30 (t, 6H); 1,58-1,80 (m, 2H); 2,19-2,32 (m, 2H); 3,55 (s, 3H); 4,00-4,18 (m, 4H); 4,42 (t, 2H); 7,81 (s, 1H); 9,59 (s, 1H, NH) ppm

### Beispiel 3

### [4-(3-Methylxanthin-7-yl)-butyl]-phosphonsäurediethylester

### a) 7-Ethoxymethyl-3-methylxanthin

52,5 g (0,28 mol) p-Toluolsulfonsäurechlorid wurden in 50 ml Dimethylformamid vorgelegt und unter Rühren und Eiskühlung 22,6 g (0,29 mol) Natriumacetat eingetragen. Es wurde etwa eine Stunde nach gerührt, mit 39,1 g Formaldehyddiethylacetal versetzt und wiederum eine Stunde nach gerührt. Anschließend wurden 41,5 g 3-Methylxanthin eingetragen und die Reaktionsmischung für 2 Stunden auf 90° C erhitzt. Nach Abkühlen auf 10° C wurde das ausgefallene Produkt abgenutscht, mit wenig Dimethylformamid nach gewaschen und mit entsalzten Wasser chloridfrei gewaschen. Das erhaltene Rohprodukt wurde aus Dimethylformamid umkristallisiert.
Ausbeute: 42,5 g ( 68,9% der Theorie), Schmelzpunkt: 262° C, C₉H₁₂N₄O₃ (MG = 224,2)

### b) 1-Benzyl-7-ethoxymethyl-3-methylxanthin

Zu einer Suspension von 224 g (1 mol) 7-Ethoxymethyl-3-methylxanthin und 165 g Kaliumkarbonat in 1000 ml Dimethylformamid wurden 205 g (1,2 mol) Benzylbromid gegeben und anschließend über Nacht bei 90°C gerührt. Die Reaktionslösung wurde heiß filtriert, das Filtrat unter verminderten Druck eingeengt und am Kugelrohr bei 1 mbar und 60°C bis zur Gewichtskonstanz getrocknet.

### c) 1-Benzyl-3-methylxanthin Hydrochlorid

Das nicht aufgereinigte 1-Benzyl-7-ethoxymethyl-3-methylxanthin wurde mit 1000 ml 5 N Salzsäure versetzt und 6 Stunden bei 60°C gerührt. Der ausgefallene Feststoff wurde abgesaugt, mit Ethanol gewaschen und unter verminderten Druck getrocknet (164 g). Aus der Mutterlauge konnten, nach Einengen auf etwa 50%, weitere 35 g des gewünschten Produktes isoliert werden.
Ausbeute: 199 g (68 % der Theorie), Schmelzpunkt: 227° C, C₁₃H₁₂ClN₄O₂ (MG = 291,4)

### d) [4-(1-Benzyl-3-methylxanthin-7-yl)-butyl]-phosphonsäurediethylester

20 g (0,078 mol) 1-Benzyl-3-methylxanthin Hydrochlorid, 21,4 g (0,094 mol) 4-Chlorbutanphosphonsäurediethylester und 26 g (0,2 mol) Kaliumcarbonat wurden in 400 ml Dimethylformamid suspendiert und 6 Stunden bei 70° C gerührt. Die Lösung wurde heiß filtriert und das Dimethylformamid unter verminderten Druck abgedampft. Der verbleibende ölige Rückstand wurde in Dichlormethan aufgenommen und mit Wasser mehrmals gewaschen. Nach Abdampfen des Lösungsmittels konnte der hellgelbe, ölige Rückstand ohne Reinigung weiter verwendet werden.
Ausbeute: 26 g (75 % der Theorie), Öl, C₂₁H₂₉N₄O₅P (MG = 448,5)

### e) [4-(3-Methylxanthin-7-yl)-butyl]-phosphonsäurediethylester

13 g (0,03 mol) [4-(1-Benzyl-3-methylxanthin-7-yl)-butyl]-phosphonsäurediethylester wurden mit 2 g Palladium auf Aktivkohle (10%) in 150 ml Ethanol suspendiert und nach Zugabe von 3 ml konzentrierter Ammoniaklösung bei 1,5 bar innerhalb 48 Stunden unter Schütteln hydriert. Der Katalysator wurde abfiltriert, das Lösungsmittel unter verminderten Druck abgedampft und der verbleibende Rückstand aus Diisopropylether kristallisiert.
Ausbeute: 8,9 g (83 % der Theorie), Schmelzpunkt: 160-168° C, C₁₄H₂₃N₄O₅P (MG = 358,3), ¹H-NMR (CDCl₃) δ = 1,29 (t, 6H); 1,52-2,05 (m, 6H); 3,54 (s, 3H); 3,95-4,15 (m, 4H); 4,28 (t, 2H); 7,60 (s, 1H); 9,22 (s, 1H, b) ppm

### Beispiel 4

### [4-(1-Ethyl-3-methylxanthin-7-yl)-butyl]-phosphonsäurediethylester

### a) 7-Ethoxymethyl-1-ethyl-3-methylxanthin

30 g (0,134 mol) 7-Ethoxymethyl-3-methylxanthin wurden mit 25 g (0,161 mol) Ethyljodid und 22,2 g (0,161 mol) Kaliumcarbonat in 500 ml absolutem DMF suspendiert und 5 Stunden bei 70° C gerührt. Die Lösung wurde filtriert und der verbleibende Rückstand in Diisopropylether kristallisiert. Es wurden 28,7 g (85% der Theorie) der Titelsubstanz erhalten und ohne Reinigung in Beispiel 4 b) eingesetzt.

### b) 1-Ethyl-3-methylxanthin

28,7 g (0,11 mol) 7-Ethoxymethyl-1-ethyl-3-methyixanthin wurden in einer Lösung von 200 ml 5 N Salzsäure und 200 ml Ethanol 60 Stunden bei 60° C gerührt. Die Reaktionsmischung wurde bis zur Trockene eingeengt und der verbleibende Rückstand in Ethylacetat/Diisopropylether kristallisiert.
Ausbeute: 20,2 g (91 % der Theorie), Schmelzpunkt: 212° C C₈H₁₀N₄O₂ (MG = 194,2)

### c) [4-(1-Ethyl-3-methylxanthin-7-yl)-butyl]-phosphonsäurediethylester

9,7 g (0,05 mol) 1-Ethyl-3-methylxanthin wurden mit 12,7 g (0,06 mol) Chlorbutanphosphonsäurediethylester und 8,3 g Kaliumkarbonat 6 Stunden bei 70°C gerührt. Die Lösung wurde filtriert, das Lösungsmittel abgedampft, der verbleibende Rückstand chromatographiert (Eluent: Ethylacetat/Methanol 10:1) und aus Ethylacetat/Diisopropylether kristallisiert.
Ausbeute: 7,2 g (38 % der Theorie), Schmelzpunkt: 108° C, C₁₆H₂₇N₄O₅P (MG = 386,4), ¹H-NMR (CDCl₃) δ = 1,23 (t, 3H); 1,28 (t, 6H); 1,55-2,05 (m, 6H); 3,55 (s, 3H); 3,95-4,15 (m, 6H); 4,28 (t, 2H); 7,52 (s, 1H) ppm

### Beispiel 5

### [4-(1-(5-Hydroxy-5-methylhexyl-3-methylxanthin-7-yl)-butyl]-phosphonsäurediethylester

10 g (0,036 mol) 5-Hydroxy-5-methylhexyl-3-methylxanthin wurden mit 5 g Kaliumkarbonat und 9,9 g (0,043 mol) Chlorbutanphosphonsäurediethylester 16 Stunden bei 90°C gerührt. Die Lösung wurde filtriert, eingeengt und der verbleibende Rückstand chromatographiert (Eluent: Dichlormethan/Methanol 10:1).
Ausbeute: 8,6 g (51 % der Theorie), Öl, C₂₁H₃₇N₄O₅P (MG =472,5), ¹H-NMR (CDCl₃) δ = 1,17 (s, 6H); 1,28 (t, 6H); 1,31-2,05 (m, 12H); 3,55 (s, 3H); 3,95-4,15 (m, 6H); 4,26 (t, 2H); 7,52 (s, 1H) ppm

### Beispiel 6

### [4-(1-Ethoxymethyl-3-methylxanthin-7-yl)-butyl]-phosphonsäure-diethylester

### a) 7-Benzyl-3-methylxanthin

Zu einer Suspension von 99,6 g (0,6 mol) 3-Methylxanthin in 1,5 l Dimethylformamid wurden unter Rühren portionsweise 16,8 g (0,7 mol) Natriumhydrid eingetragen. Die Mischung wurde nach Abschluß der Wasserstoffentwicklung auf 100° C erhitzt, tropfenweise mit 76 g (0,6 mol) Benzylchlorid versetzt und noch 6 Stunden auf 120° C erhitzt. Die Reaktionsmischung wurde abgekühlt und vorsichtig in 3 I Wasser gegossen. Es wurde abgenutscht, mit Wasser gewaschen, der Filterrückstand in Ethanol aufgenommen und 1 Stunde bei Raumtemperatur gerührt. Das Kristallisat wurde filtriert, mit Ethanol gewaschen und unter verminderten Druck getrocknet.
Ausbeute: 90,7 g (59 % der Theorie), Schmelzpunkt: 263° C C₁₃H₁₂N₄O₂ (MG = 256,3)

### b) 7-Benzyl-1-ethoxymethyl-3-methylxanthin

20 g (0,078 mol) 7-Benzyl-3-methylxanthin wurden mit 8,8 g (0,094 mol) Ethoxymethyl-chlorid und 13 g Kaliumcarbonat in 500 ml absoluten Dimethylformamid für 6 Stunden bei 80° C gerührt, filtriert, eingeengt und der verbleibende Rückstand chromatographiert (Eluent: Dichlormethan/Methanol 20:1).
Ausbeute: 17,6 g (70 % der Theorie), Öl, C₁₆H₁₈N₄O₃ (MG = 314,3)

### c) 1-Ethoxymethyl-3-methylxanthin

17,5 g (0,056 mol) 7-Benzyl-1-ethoxymethyl-3-methylxanthin wurden in 500 ml Ethanol über 1,8 g Palladium (10%) auf Aktivkohle bei Raumtemperatur für 8 Stunden hydriert. Der Katalysator wurde abfiltriert und das Filtrat bis zur Trockene eingeengt und aus Dimethylformamid/Diisopropylether kristallisiert. Ausbeute: 7,9 g (63,4 % der Theorie), Schmelzpunkt: 164-168° C, C₉H₁₂N₄O₃ (MG = 224,2)

### d) [4-(1-Ethoxymethyl-3-methylxanthin-7-yl)-butyl]-phosphonsäurediethylester

4 g (0,018 mol) 1-Ethoxymethylxanthin wurden mit 3 g Kaliumkarbonat und 4,9 g (0,0214 mol) Chlorbutanphosphonsäurediethylester 6 Stunden bei 70°C gerührt. Die Lösung wurde filtriert, eingeengt und der verbleibende Rückstand chromatographiert (Eluent: Dichlormethan/Methanol 20:1).
Ausbeute: 5,2 g (69 % der Theorie), Öl, C₁₇H₂₉N₄O₅P (MG = 416,4), ¹H-NMR (CDCl₃) δ = 1,20 (t, 3H); 1,28 (t, 6H); 1,53-2,05 (m, 6H); 3,55 (s, 3H); 3,66 (q, 2H); 3,95-4,15 (m, 4H); 4,28 (t, 2H); 5,46 (s, 2H); 7,54 (s, 1H) ppm

### Beispiel 7

### 4-{7-[4-(Diethoxy-phosphoryl)-butyl]-3-methylxanthin-1-yl}-butylphosphonsäurediethylester

8 g (0,022 mol) 7-(4-Diethylphosphonobutyl)-3-methylxanthin (gemäß Beispiel 24) wurden in 100 ml DMF suspendiert, mit 6,13 g (0.027 mol) 4-Chlorbutanphosphonsäurediethylester und 3,7 g (0,028 mol) aktivierten Kaliumcarbonat versetzt und 4 Stunden auf 70° C erhitzt. Anschließend wurde das Produkt filtriert, das Filtrat unter verminderten Druck eingeengt und der verbleibende ölige Rückstand über eine Kieselgelsäule chromatographiert (Eluent: Ethylacetat/Methanol 10:1).
Ausbeute: 9,1 g ( 74 % der Theorie), Öl, C₂₂H₄₀N₄O₈P₂ (MG = 550,5), ¹H-NMR (CDCl₃) δ = 1,27 (t, 12H); 1,50-2,05 (m, 12H); 3,55 (s, 3H); 3,88-4,17 (m, 10H); 4,28 (t, 2H); 7,64 (s, 1H) ppm

### Beispiel 8

### [3-(1,3-Dimethylxanthin-7-yl)-propyl]-phosphonsäurediethylester

Die Titelsubstanz wurde analog zu Beispiel 1 aus 1,3-Dimethylxanthin und 3-Brompropanphosphonsäurediethylester hergestellt.
Ausbeute: 18,4 g (77 % der Theorie), Schmelzpunkt: 93° C, C₁₄H₂₃N₄O₅P (MG = 358,34), ¹H-NMR (CDCl₃) δ = 1,32 (t, 6H); 1,55-1,80 (m, 2H); 2,10-2,32 (m, 2H); 3,40 (s, 3H); 3,60 (s, 3H); 4,00-4,19 (m, 4H); 4,45 (t, 2H); 7,80 (s, 1H) ppm

### Beispiel 9

### [3-(1,3-Dipropylxanthin-7-yl)-propyl]-phosphonsäurediethylester

Die Titelsubstanz wurde analog zu Beispiel 1 aus 1,3-Dipropylxanthin und 3-Brompropanphosphonsäurediethylester hergestellt und über Kieselgel chromatographiert (Eluent: Ethylacetat/Methanol 20:1).
Ausbeute: 6 g (49% der Theorie), Öl, C₁₈H₃₁N₄O₅P (MG = 414,5), ¹H-NMR (CDCl₃) δ = 0,94 (t, 3H); 0,97 (t, 3H); 1,30 (t, 6H); 1,55-1,76 (m, 6H); 2,08-2,30 (m, 2H); 3,91-4,28 (m, 8H); 4,40 (t, 2H); 7,61 (s, 1H) ppm

### Beispiel 10

### [5-(1,3-Dibutylxanthin-7-yl)-pentyl]-phosphonsäurediethylester

Die Titelsubstanz wurde analog zu Beispiel 9 aus 1,3-Dibutylxanthin und 5-Brompentanphosphonsäurediethylester hergestellt.
Ausbeute: 2,7 g (30,2 % der Theorie), Öl, C₂₂H₃₉N₄O₅P (MG = 470,6), ¹H-NMR (CDCl₃) δ = 0,95 (t, 3H); 0,96 (t, 3H); 1,32 (t, 6H); 1,33-1,98 (m, 16H); 3,95-4,18 (m, 8H); 4,28 (t, 2H); 7,52 (s, 1H) ppm

### Beispiel 11

### [4-(1,3-Dibutylxanthin-7-yl)-butyl]-phosphonsäurediethylester

Die Titelsubstanz wurde analog zu Beispiel 9 aus 1,3-Dibutylxanthin und 4-Chlorbutanphosphonsäurediethylester hergestellt.
Ausbeute: 4,5 g (52 % der Theorie), Öl, C₂₁H₃₇N₄O₅P (MG = 456,5), ¹H-NMR (CDCl₃) δ = 0,96 (t, 3H); 0,97 (t, 3H); 1,32 (t, 6H); 1,33-2,19 (m, 14H); 3,95-4,18 (m, 8H); 4,30 (t, 2H); 7,54 (s, 1H) ppm

### Beispiel 12

### [3-(1,3-Dipropylxanthin-7-yl)-propyl]-phosphonsäurediethylester

Die Titelsubstanz wurde analog zu Beispiel 9 aus 1,3.Dibutylxanthin und 3-Brompropanphosphonsäurediethylester hergestellt.
Ausbeute: 4 g (48 % der Theorie), Öl, C₂₀H₃₅N₄O₅P (MG = 442,5), ¹H-NMR (CDCl₃) δ = 0,96 (t, 3H); 0,99 (t, 3H); 1,34 (t, 6H); 1,34-1,82 (m, 10H); 2,10-2,35 (m, 2H); 3,95-4,20 (m, 8H); 4,41 (t, 2H); 7,63 (s, 1H) ppm

### Beispiel 13

### [4-(3-Methyl-7-propylxanthin-7-yl)-butyl]-phosphonsäurediethylester

Die Titelsubstanz wurde analog zu Beispiel 4 aus 3-Methyl-1-propylxanthin und 4-Chlorbutanphosphonsäurediethylester hergestellt und über Kieselgel chromatographiert (Eluent: Dichlormethan/Methanol 10:1).
Ausbeute: 4,5 g (47 % der Theorie), Schmelzpunkt: 69° C, C₁₇H₂₉N₄O₅P (MG =400,4), ¹H-NMR (CDCl₃) δ = 0,96 (t, 3H); 1,30 (t, 6H); 1,55-2,10 (m, 8H); 3,57 (s, 3H); 3,90-4,25 (m, 6H); 4,29 (t, 2H); 7,53 (s, 1H) ppm

### Beispiel 14

### [4-(1-Methoxyethyl-3-methylxanthin-7-yl)-butyl]-phosphonsäure-diethylester

Die Titelsubstanz wurde analog zu Beispiel 4 aus 1-Methoxyethyl-3-methylxanthin und 4-Chlorbutanphosphonsäurediethylester hergestellt und aus Diisopropylether kristallisiert.
Ausbeute: 5,6 g (37 % der Theorie), Schmelzpunkt: 59° C, C₁₇H₂₉N₄O₅P, (MG = 416,4), ¹H-NMR (CDCl₃) d= 1,30 (t, 6H); 1,60-2,08 (m, 6H); 3,36 (s, 3H); 3,58 (s, 3H); 3,65 (t, 2H); 4,00-4,18 (m, 4H); 4,22-4,33 (m, 4H); 7,53 (s, 1H) ppm

### Beispiel 15

### [4-(1-(3-Methylbutyl)-3-methylxanthin-7-yl)-butyl]-phosphonsäurediethylester

Die Titelsubstanz wurde analog zu Beispiel 1 aus [4-(3-Methylxanthin-7-yl)-butyl]-phosphonsäurediethylester und 2-Methylbutylchlorid hergestellt und über Kieselgel chromatographiert (Eluent: Dichlormethan/Methanol 10:1).
Ausbeute: 4,2 g (54 % der Theorie), Öl, C₁₉H₃₃N₄O₅P (MG = 428,5), ¹H-NMR (CDCl₃) d= 0,96 (d, 6H); 1,30 (t, 6H); 1,50-2,07 (m, 9H); 3,56 (s, 3H); 3,98-4,13 (m, 6H); 4,30 (t, 2H); 7,52 (s 1H) ppm

### Beispiel 16

### [5-(7-Ethoxymethyl-3-methylxanthin-1-yl)-pentyl]-phosphonsäurediethylester

### a) 1-(5-Brompentyl)-7-ethoxymethyl-3-methylxanthin

22,4 g (0,1 mol) 7-Ethoxymethylxanthin wurden mit 45,9 g (0,2 mol) 1,5-Dibrompentan und 27,6 g aktiviertem Kaliumcarbonat 1,5 Stunden bei 70°C gerührt. Die Lösung wurde filtriert, eingeengt und der verbleibende ölige Rückstand chromatographiert (Eluent: Ethylacetat).
Ausbeute: 21,4 g (57 % der Theorie), Öl, C₁₄H₂₁BrN₄O₃ (MG = 373,26)

### 2b) [5-(7-Ethoxymethyl-3-methylxanthin-1-yl)-pentyl]-phosphonsäurediethylester

4,8 g (0,035 mol) Phosphorigsäurediethylester wurden in 100 ml trockenen Dimethylformamid auf 0°C abgekühlt und langsam mit 0,8 g (0,035 mol) Natriumhydrid versetzt. Die Lösung wurde zur Vervollständigung der Deprotonierung noch 0,5 Stunden bei 20°C gerührt und anschließend mit 10 g (0,0268 mol) 1-(5-Brompentyl)-7-ethoxymethyl-3-methylxanthin, gelöst in wenig Dimethylformamid, versetzt. Nach 4 Stunden bei 20°C wurde die Reaktionsmischung mit wenig gesättigter Ammoniumchlorid-Lösung gequencht, eingeengt und über Kieselgel chromatographiert (Eluent: Dichlormethan/Methanol 15:1).
Ausbeute: 5,1 g (44 % der Theorie), Öl, C₁₈H₃₁N₄O₆P (MG = 430,4), ₁H-NMR (CDCl₃) d= 1,20 (t, 3H); 1,30 (t, 6H); 1,36-1,82 (m, 8H); 3,59 (s, 3H); 3,62 (q, 2H); 3,95-4,18 (m, 6H); 5,70 (s, 2H); 7,74 (s, 1H) ppm

### Beispiel 17

### [5-(3-Methyl-7-propylxanthin-1-yl)-pentyl]-phosphonsäurediethylester

Die Titelsubstanz wurde analog zu Beispiel 16 aus 1-(5-Brompentyl)-3-methyl-7-propylxanthin und Phosphorigsäuretdiethylester hergestellt.
Ausbeute: 10 g (29 % der Theorie), Öl, C₁₈H₃₁N₄O₅P (MG =414,5), ¹H-NMR (CDCl₃) δ = 0,94 (t, 3H); 1,29 (t, 6H); 1,40-1,95 (m, 10H); 3,56 (s, 3H); 3,95-4,15 (m, 6H); 4,23 (t, 2H); 7,52 (s, H) ppm

### Beispiel 18

### [5-(7-Methoxyethyl-3-methylxanthin-1-yl)-pentyl]-phosphonsäurediethylester

### a) 5-p-Toluolsulfonyloxypentylphosphonsäurediethylester

30 g (0,134 mol) 5-Hydroxypentylphosphonsäurediethylester (siehe Beispiel 21) wurden in 200 ml Pyridin gelöst, auf 0°C abgekühlt und unter Rühren mit 26,1 g (0,134 mol) 4-Toluolsulfonsäurechlorid versetzt. Die Reaktionsmischung wurde unter verminderten Druck eingeengt, in Ethylacetat aufgenommen, mehrmals mit 20%iger Zitronensäure-Lösung und Wasser gewaschen und über Natriumsulfat getrocknet. Nach Abdampfen des Lösungsmittels verblieb ein hellgelbes Öl, daß ohne weitere Reinigung eingesetzt wurde. Ausbeute: 37,5 g (74 % der Theorie)

### b) [5-(7-Methoxyethyl-3-methylxanthin-1-yl)-pentyl]-phosphonsäurediethylester

9,3 g (0,0245 mol) des Sulfonsäureesters gemäß a) wurden mit 5,5 g (0,0245 mol) 7-Methoxyethylxanthin und 6,7 g (0,049 mol) aktivierten Kaliumcarbonat in 150 ml Dimethylformamid 8 Stunden bei 80° C gerührt. Anschließend wurde die Reaktionslösung filtriert, eingeengt und über Kieselgel (Eluent: Ethylacetat/Methanol 20:1) chromatographiert. Nach Einengen der entsprechenden Lösung verblieb ein hellgelbes Öl.
Ausbeute: 6,2 g (59 % der Theorie), C₁₈H₃₁O₆P (MG = 430,4), ¹H-NMR (CDCl₃) δ = 1,30 (t, 6H); 1,40 - 1,85 (m, 8H); 3,32 (s, 3H); 3,57 (s, 3H); 3,69 (t, 2H); 4,05 (m, 6H); 4,45 (t, 2H); 7,62 (s, 1H) ppm

### Beispiel 19

### 5-(3,7-Dimethylxanthin-1-yl)-pentyl-phosphonsäurediethylester

Die Titelsubstanz wurde analog zu Beispiel 18 aus 0,0245 mol 5-p-Toluolsulfonyloxypentylphosphonsäurediethylester und 0,02 mol 1,7-Dimethylxanthin hergestellt und über Kieselgel chromatographiert (Eluent: Ethylacetat/Methanol 10:1). Ausbeute: 3,1 g (30 % der Theorie), Schmelzpunkt: 85° C, C₁₆H₂₇N₄O₅P (MG= 386,4), ¹H-NMR (CDCl₃) δ = 1,30 (t, 6H); 1,35-1,88 (m, 8H); 3,55 (s, 3H); 3,97 (s, 3H); 3,95-4,15 (m, 6H); 7,50 (s 1H) ppm

### Beispiel 20

### 5-(7-Ethyl-3-methylxanthin-1-yl)-pentyl-phosphonsäurediethylester

Die Titelsubstanz wurde analog zu Beispiel 19 aus 0,0245 mol 5-p-Toluolsulfonyloxypentylphosphonsäurediethylester und 0,024 mol 7-Ethyl-3-methylxanthin hergestellt.
Ausbeute: 6,9 g (70 % der Theorie), Schmelzpunkt: 61-63° C, C₁₇H₂₉N₄O₅P (MG = 400,4), ¹H-NMR (CDCl₃) δ = 1,30 (t, 6H); 1,51 (t, 3H); 1,51-1,82 (m,8H); 3,57 (s 3H); 3,95-4,18 (m, 6H); 4,33 (q, 2H); 7,55 (s, 1H) ppm

### Beispiel 21

### 5-(7-Ethoxymethyl-3-methylxanthin-1-yl)-1,1-dimethyl-pentyl-phosphonsäurediethylester

### a) Essigsäure-5-(diethylphosphono)-pentylester

100,34 g (0,48 mol) Essigsäure-5-brompentylester wurden mit 79,8 g (0,48 mol) Triethylphosphit 8 Stunden auf 150° C unter Rühren und Einleiten von Stickstoff erhitzt. Anschließend wurde unter verminderten Druck fraktioniert destilliert.
Ausbeute: 92 g (72 % der Theorie), Siedepunkt (0,4 mbar) 132 - 135°C, C₁₁H₂₃O₅P (MG = 266,3)

### b) 5-Hydroxypentylphosphonsäurediethylester

92 g (0,345 mol) Essigsäure-5-(diethylphosphono)pentylester wurden mit 74 g (0,7 mol) fein gepulvertem Natriumcarbonat in 500 ml Methanol unter Rühren 10 Stunden auf 50° C erhitzt. Die Lösung wurde filtriert, eingeengt und am Kugelrohr destilliert.
Ausbeute: 66,9 g (86,5 % der Theorie), Siedepunkt (0,1 mbar) 110 - 130°C, C₉H₂₁O₄P (MG = 224,2), ¹H-NMR (CDCl₃) δ = 1,31 (t, 6H); 1,38 - 1,80 (m, 8H); 1,90 (s, 1H); 3,63 (t, 2H); 4,08 (m, 4H) ppm

### c) 5-(Tetrahydropyran-2-yloxy)-pentyl-phosphonsäurediethylester

Eine Lösung von 66,9 g (0,298 mol) 5-Hydroxypentylphosphonsäurediethylester, 32,6 g (0,38 mol) 3,4-Dihydropyran und 1 g (0,004 mol) Pyridiniumtoluol-4-sulfonat in 800 ml Dichlormethan wurde 16 Stunden unter Rückfluß erhitzt. Die Lösung wurde mit Wasser und gesättigter Natriumhydrogencarbonat-Lösung gewaschen, über Natriumsulfat getrocknet und am Kugelrohr destilliert.
Ausbeute: 89,5 g (97,4 % der Theorie), Siedepunkt (0,1 mbar) 110 - 130°C, C₁₄H₂₉O₅P (MG = 308,3), ¹H-NMR (CDCl₃) δ = 1,31 (t, 6H); 1,39 - 1,82 (m, 14H); 3,42 (m, 2H); 3,75 (m, 2H); 4,08 (m, 4H); 4,65 (m, 1H) ppm

### d) 1-Methyl-5-(tetrahydropyran-2-yloxy)-pentyl-phosphonsäurediethylester

30 g (0,097 mol) 5-(Tetrahydropyran-2-yloxy)-pentyl-phosphonsäurediethylester wurden in 200 ml absolutem Tetrahydrofuran unter Argon-Atmosphäre vorgelegt, auf -70° C abgekühlt und mit 42,8 ml (0,107 mol) einer 2,5 molaren Lösung von Butyllithium in Hexan versetzt. Nach Zugabe wurde die Lösung noch 30 Minuten gerührt und dann 15,6 g (0,11 mol) Methyliodid langsam zu getropft. Es wurde noch 2 Stunden bei -70° C und 4 Stunden bei Raumtemperatur gerührt, vorsichtig mit einer gesättigten Natriumchlorid-Lösung versetzt und in Ethylacetat aufgenommen. Die organische Phase wurde mit Wasser und einer gesättigten Natriumchlorid-Lösung gewaschen, über Natriumsulfat getrocknet und am Kugelrohr destilliert.
Ausbeute: 23,4 g (75 % der Theorie), Siedepunkt (0,1 mbar) 120°C, C₁₅H₃₁O₅P (MG = 322,4), ¹H-NMR (CDCl₃) δ = 1,10 (d, 1,5H); 1,19 (d, 1,5H); 1,31 (t, 6H); 1,30 - 1,89 (m, 13H); 3,42 (m, 2H); 3,78 (m, 2H); 4,08 (m, 4H), 4,55 (m, 1H) ppm

### e) 1,1-Dimethyl-5-(tetrahydropyran-2-yloxy)-5-pentyl-phosphonsäurediethylester

23,3 g (0,0724 mol) 1-Methyl-5-(tetrahydropyran-2-yloxy)-pentylphosphonsäure-diethylester wurden in 200 ml absolutem Tetrahydrofuran unter Argon-Atmosphäre vorgelegt, auf -70° C abgekühlt und mit 31,9 ml (0,08 mol) einer 2,5 molaren Lösung von Butyllithium in Hexan versetzt. Nach beendeter Zugabe wurde die Lösung noch 30 Minuten gerührt und dann 12,3 g (0,087 mol) Methyliodid langsam zu getropft. Es wurde noch 2 Stunden bei -70° C und 4 Stunden bei Raumtemperatur gerührt, vorsichtig mit einer gesättigten Natriumchlorid-Lösung versetzt und in Ethylacetat aufgenommen. Die organische Phase wurde mit Wasser und einer gesättigten Natriumchlorid-Lösung gewaschen, über Natriumsulfat getrocknet und am Kugelrohr destilliert.
Ausbeute: 19,9 g (75 % der Theorie), Siedepunkt (0,1 mbar) 115 - 125°C, C₁₆H₃₃O₅P (MG = 336,4), ¹H-NMR (CDCl₃) δ = 1,09 (s, 3H); 1,17 (s, 3H); 1,30 (t, 6H); 1,38 - 1,89 (m, 12H); 3,44 (m, 2H); 3,74 (m, 2H); 4,09 (m, 4H), 4,56 (m, 1H) ppm

### f) 5-Hydroxy-1,1-dimethyl-pentylphosphonsäurediethylester

19,9 g (0,052 mol) 1,1-Dimethyl-5-(tetrahydropyran-2-yloxy)-5-pentylphosphonsäurediethylester wurden mit 1,5 g (0,006 mol) Pyridiniumtoluol-4-sulfonat in 200 ml wäßrigen Ethanol 8 Stunden bei 55° C gerührt. Das Lösungsmittel wurde abgedampft, der Rückstand in Ethylacetat aufgenommen, über eine Kieselgelsäule filtriert und nach Abdampfen der Lösungsmittel am Kugelrohr destilliert.
Ausbeute: 12,8 g (86,7 % der Theorie), Siedepunkt (0,1 mbar) 120 - 130°C, C₁₁H₂₅O₄P (MG = 252,3)

### g) 5-p-Toluolsulfonoxy-1,1-dimethyl-pentyl-phosphonsäurediethylester

12,8 g (0,051 mol) 5-Hydroxy-1,1-dimethyl-pentylphosphonsäurediethylester wurden in 100 ml Pyridin gelöst, auf 0° C abgekühlt und langsam mit 9,7 g (0,051 mol) 4-Toluolsulfonsäurechlorid versetzt. Die Reaktionsmischung wurde unter verminderten Druck eingeengt, in Ethylacetat aufgenommen, mehrmals mit 20%iger Zitronensäure-Lösung und Wasser gewaschen und über Natriumsulfat getrocknet. Nach Abdampfen des Lösungsmittels verblieb ein hellgelbes Öl (17,2 g), daß ohne weitere Reinigung eingesetzt wurde.
Ausbeute: 6,74 g (32,5 % der Theorie)

### h) 5-(7-Ethoxymethyl-3-methylxanthin-1-yl)-1,1-dimethyl-pentyl-phosphonsäurediethylester

6,75 g (0,017 mol) des Sulfonsäureesters gemäß g) wurden mit 3,73 g (0,017 mol) 7-Ethoxymethylxanthin und 4,6 g (0,033 mol) aktivierten Kaliumcarbonat in 100 ml Dimethylformamid 8 Stunden bei 80° C gerührt. Anschließend wurde die Reaktionslösung filtriert, eingeengt und über Kieselgel (Eluent: Ethylacetat/Methanol 20:1) chromatographiert. Nach Einengen der entsprechenden Fraktionen verblieb ein hellgelbes Öl.
Ausbeute: 4 g ( 44 % der Theorie), C₂₀H₃₅N₄O₆P (MG = 458,5), ¹H-NMR (CDCl₃) δ = 1,14 (d, 6H); 1,23 (t, 3H); 1,30 (t, 6H); 1,35-1,73 (m, 6H); 3,59 (s, 3H); 3,63 (q, 2H); 4,07 (m, 4H); 5,70 (s, 2H); 7,74 (s, 1H) ppm

### Beispiel 22

### [5-(7-Methoxyethyl-3-methylxanthin-1-yl)-1,1-dimethyl-pentyl]-phosphonsäurediethylester

Die Titelsubstanz wurde analog zu Beispiel 21 aus 0,02 mol des Sulfonsäureesters und 0,02 mol 7-Methoxyethyl-3-methylxanthin hergestellt.
Ausbeute: 3,8 g ( 39% der Theorie), C₂₀H₃₅N₄O₆P (MG = 458,5), ¹H-NMR (CDCl₃) δ = 1,13 (d, 6H); 1,30 (t, 6H); 1,35 - 1,70 (m, 6H); 3,31 (s, 3H); 3,57 (s, 3H); 3,70 (t, 2H); 3,95-4,18 (m, 6H); 4,47 (t, 2H); 7,62 (s, 1H) ppm

### Beispiel 23

### [4-(7-Benzyl-3-methylxanthin-1-yl)-butyl]-phosphonsäure-diethylester

20,4 g (0,08 mol) 7-Benzyl-3-methylxanthin wurden in 200 ml Dimethylformamid (DMF) suspendiert, mit 22 g (0,96 mol) 4-Chlorbutanphosphonsäurediethylester und 13,8 g (0,1 mol) aktivierten Kaliumcarbonat versetzt und 4 Stunden auf 70° C erhitzt, filtriert, das Filtrat unter verminderten Druck eingeengt und der verbleibende ölige Rückstand in Ethylacetat aufgenommen, nochmals filtriert und aus Diisopropylether kristallisiert.
Ausbeute: 22,6 g (63 % der Theorie), Schmelzpunkt: 120 - 121°C, C₂₁H₂₉N₄O₅P (MG = 448,5), ¹H-NMR (CDCl₃) δ = 1,23 (t, 6H); 1,70 (m, 6H); 3,55 (s, 3H); 4,07 (m, 6H); 5,48 (s, 2H); 7,33 (m, 5H); 7,54 (s, 1H) ppm

### Beispiel 24

### [4-(3-Methylxanthin-1-yl)-butyl]-phosphonsäurediethylester

15,7 g (0,035 mol) [4-(7-Benzyl-3-methylxanthin-1-yl)-butyl]-phosphonsäurediethylester wurden in 150 ml Ethanol über 1 g Palladium (10 %) auf Aktivkohle bei Raumtemperatur innerhalb von 8 Stunden hydriert. Der Katalysator wurde abfiltriert, das Filtrat wurde unter verminderten Druck eingeengt und der Rückstand wurde aus Diisopropylether kristallisiert.
Ausbeute: 11,7 g (93 % der Theorie), Schmelzpunkt: 140 - 141° C, C₁₄H₂₃N₄O₅P (MG = 358,3), ¹H-NMR (CDCl₃) δ = 1,34 (t, 6H); 1,60-2,15 (m, 6H); 3,64 (s, 3H); 4,10 (m, 6H); 7,85 (s, 1H); 11,2 (s, 1H) ppm

### Beispiel 25

### [4-(3-Methylxanthin-1-yl)-butyl]-phosphonsäure Diammoniumsalz

5,2 g (0,0125 mol) [4-(3-Methylxanthin-1-yl)-butyl]-phosphonsäurediethylester wurden 4 Stunden mit 30 ml 20%iger Salzsäure unter Rücksfluß erhitzt. Die Salzsäure wurde abgedampft und der ölige Rückstand über Kaliumhydroxid im Exsikkator getrocknet. Anschließend versetzte man die gebildete Phosphonsäure mit einer methanolischen Ammoniaklösung, dampfte das Lösungsmittel ab und kristallisierte den Rückstand aus einem Methanol/Diisopropylether Gemisch.
Ausbeute: 3,6 g ( 85,6% der Theorie), Schmelzpunkt: > 220° C, C₁₀H₂₁N₆O₅P (MG = 336,3), ¹H-NMR (D₂O) δ = 1,45-1,70 (m, 6H); 3,43 (s, 3H); 3,85 (t, 2H); 7,92 (s, 1H) ppm

### Beispiel 26

### [4-(7-Ethoxymethyl-3-methylxanthin-1-yl)-butyl]-phosphonsäure-diethylester

44,8 g (0,2 mol) 7-Ethoxymethyl-3-methylxanthin wurden in 500 ml DMF suspendiert, mit 55 g (0,24 mol) 4-Chlorbutanphosphonsäurediethylester und 50 g (0,32 mol) aktivierten Kaliumcarbonat versetzt und 4 Stunden auf 70° C erhitzt, filtriert, das Filtrat unter verminderten Druck eingeengt und der verbleibende ölige Rückstand in Ethylacetat aufgenommen, nochmals filtriert und aus Diisopropylether kristallisiert.
Ausbeute: 68,3 g ( 82 % der Theorie), Schmelzpunkt: 109 - 110° C, C₁₇H₂₉N₄O₆P (MG = 416,4), ¹H-NMR (CDCl₃) δ = 1,20 (t, 3H); 1,31 (t, 6H); 1,60-1,90 (m, 6H); 3,59 (s, 3H); 3,63 (q, 2H); 3,95-4,18 (m, 6H); 5,70 (s, 2H); 7,74 (s, 1H)

### Beispiel 27

### [4-(7-Ethoxymethyl-3-methylxanthin-1-yl)-butyl]-phosphonsäuremono-ethylester Ammoniumsalz

### a) 4-Chlorbutanphosphonsäure-(1)-ethylester-chlorid

14 g (0,062 mol) 4-Chlorbutanphosphonsäurediethylester in 150 ml absolutem Tetrachlorkohlenstoff wurden unter Rühren und Kühlung auf 20° C im Laufe von 4 Stunden portionsweise mit 12,8 g (0,062 mol) Phosphorpentachlorid versetzt. Nach weiterem Rühren bei Raumtemperatur lieferte die fraktionierte Destillation 8,45 g (0,039 mol) des entsprechenden Säurechlorids.
Ausbeute: 8,45 g (60% der Theorie), Siedepunkt (0,2 mbar): 102 - 108°C, C₆H₁₃ClO₂P (MG = 219,1)

### b) 4-Chlorbutanphosphonsäure-(1)-benzyl-ethylester

8,4 g (0,038 mol) 4-Chlorbutanphosphonsäure-(1)-ethylester-chlorid wurden in 100 ml Dichlormethan gelöst, mit 4,01 g (0,04 mol) Triethylamin versetzt und auf 0° C abgekühlt. Anschließend wurden 5,4 g (0,05 mol) Benzylalkohol zugetropft und die Reaktionsmischung 1 Stunde bei 0° C und 4 Stunden bei Raumtemperatur gerührt. Die Lösung wurde mit Wasser, 20%iger Zitronensäure und gesättigter Natriumhydrogencarbonat gewaschen, über Natriumsulfat getrocknet und nach Abdampfen des Dichlormethans am Kugelrohr destilliert.
Ausbeute: 8,3 g ( 72% der Theorie), Siedepunkt (0,1 mbar) > 90° C, C₁₃H₂₀ClO₃P (MG = 290,7)

### c) [4-(7-Ethoxymethyl-3-methylxanthin-1-yl)-butyl]-phosphonsäurebenzylethylester

6 g (0,027 mol) 7-Ethoxymethyl-3-methylxanthin wurden in 70 ml DMF suspendiert, mit 7,85 g (0,027 mol) 4-Chlorbutanphosphonsäure-(1)-benzylethylester und 5 g (0,032 mol) aktivierten Kaliumcarbonat versetzt und 4 Stunden auf 70° C erhitzt, filtriert, das Filtrat unter verminderten Druck eingeengt und der verbleibende ölige Rückstand über eine Kieselgelsäule chromatographiert (Eluent: Dichlormethan/Methanol 15:1).
Ausbeute: 6,5 g (50,3% der Theorie), Öl, C₂₂H₃₁N₄O₆P (MG = 478,5), ¹H-NMR (CDCl₃) d= 1,12 (t, 3H); 1,26 (t, 3H); 1,50-1,90 (m, 6H); 3,59 (s,3H); 3,62 (q, 2H); 3,95-4,18 (m, 6H); 5,02 (s, 1H); 5,06 (s, 1H); 5,69 (s, 2H); 7,15-7,43 (m, 5H); 7,87 (s, 1H) ppm

### d) [4-(7-Ethoxymethyl-3-methylxanthin-1-yl)-butyl]-phosphonsäuremonoethylester Ammoniumsalz

6,3 g (0,013 mol) 4-(7-Ethoxymethyl-3-methylxanthin-1-yl)butylphosphonsäurebenzylethylester wurden in 100 ml Ethanol über 0,5 g Palladium (10 %) auf Aktivkohle bei Raumtemperatur innerhalb von 4 Stunden hydriert. Der Katalysator wurde abfiltriert, das Filtrat unter verminderten Druck eingeengt, die erhaltene Lösung mit methanolischem Ammoniak versetzt und anschließend wurde der, nach Abdampfen des Lösungsmittels verbleibenden Rückstand, aus Diisopropylether kristallisiert.
Ausbeute: 4,6 g (87 % der Theorie), Schmelzbereich: 84 - 96° C, C₁₅H₂₈N₅O₆P (MG = 405,4), ¹H-NMR (DMSO-d₆) δ = 1,09 (t, 3H); 1,10 (t, 3H); 1,20-1,60 (m, 6H); 3,43 (s, 3H); 3,52 (q, 2H); 3,59-3,78 (m, 2H); 3,85 (t, 2H); 5,62 (s, 2H); 7,62 (s, 4H, NH₄); 8,28 (s, 1H) ppm

### Beispiel 28

### [4-(7-Ethoxymethyl-3-methylxanthin-1-yl)-butyllphosphonsäure Diammoniumsalz

Zu einer Lösung von 4,16 g (0,01 mol) [4-(7-Ethoxymethyl-3-methylxanthin-1-yl)-butyl]-phosphonsäurediethylester in 5 ml Dichlormethan wurden bei 0° C, unter Argonatmosphäre, 3,4 ml (0,026 mol) Trimethylsilylbromid langsam zu getropft. Die Lösung wurde 1 Stunde bei 0° C und 2 Stunden bei Raumtemperatur gerührt. Anschließend wurde das Dichlormethan abgedampft und der verbleibende ölige Rückstand, zur Entfernung von überschüssigen Trimethylsilylbromid und entstandenen Ethylbromid, am Kugelrohr im Ölpumpenvakuum getrocknet. Der Rückstand wurde in wenig Methanol aufgenommen und mit 1 ml konzentrierter Ammoniaklösung versetzt und 2 Stunden bei Raumtemperatur gerührt. Nach Abdampfen der Lösungsmittel erfolgte die Kristallisation des Diammoniumsalzes aus einem Methanol/Diisopropylether Gemisch.
Ausbeute: 2,96 g (75 % der Theorie), Schmelzpunkt: 159° C, C₁₃H₂₁N₄O₆P (MG = 360,3), ¹H-NMR (D₂O) δ = 1,11 (t, 3H); 1,40-1,75 (m, 6H); 3,47 (s, 3H); 3,60 (q, 2H); 3,90 (t, 2H); 5,64 (s, 2H); 8,12 (s, 1H) ppm

### Beispiel 29

### [4-(3,7-Dimethylxanthin-1-yl)-butyl]-phosphonsäurediethylester

Die Titelsubstanz wurde analog zu Beispiel 23 aus 0,075 mol Theobromin und 0,09 mol 4-Chlorbutan-phosphonsäurediethylester hergestellt und aus Diisopropylether kristallisiert.
Ausbeute: 6,1 g (22 % der Theorie), Schmelzpunkt: 80° C, C₁₅H₂₅N₄O₅P (MG = 372,4), ¹H-NMR (CDCl₃) δ = 1,31 (t, 6H); 1,62-1,90 (m, 6H); 3,56 (s 3H); 3,98 (s, 3H); 3,98-4,18 (m, 6H); 7,50 (s, 1H) ppm

### Beispiel 30

### [4-(7-Ethyl-3-methylxanthin-1-yl)-butyl]-phosphonsäurediethylester

7,2 g (0,02 mol) [4-(3-Methylxanthin-1-yl)-butyl]-phosphonsäurediethylester wurden in 100 ml DMF suspendiert, mit 4,7 g (0,03 mol) Ethyliodid und 4,1 g (0,03 mol) aktivierten Kaliumcarbonat versetzt und 4 Stunden auf 60° C erhitzt. Der Feststoff wurde abfiltriert, das Filtrat unter verminderten Druck eingeengt und der verbleibende ölige Rückstand in Ethylacetat aufgenommen, nochmals filtriert und aus Diisopropylether kristallisiert.
Ausbeute: 4,9 g (63 % der Theorie), Schmelzpunkt: 62 - 63° C, C₁₆H₂₇N₄O₅P (MG= 386,4), ¹H-NMR (CDCl₃) d= 1,31 (t, 6H); 1,51 (t, 3H); 1,35-1,85 (m, 6H); 3,57 (s, 3H); 3,94-4,20 (m, 6H); 4,33 (q, 2H); 7,55 (s, 1H) ppm

### Beispiel 31

### [4-(7-Methoxyethyl-3-methylxanthin-1-yl)-butyll-phosphonsäure-diethylester

9 g (0,04 mol) 7-Methoxyethyl-3-methylxanthin wurden in 100 ml DMF suspendiert, mit 11 g (0,048 mol) 4-Chlorbutanphosphonsäurediethylester und 10 g (0,064 mol) aktivierten Kaliumcarbonat versetzt und 4 Stunden auf 70° C erhitzt. Der Feststoff wurde abfiltriert, das Filtrat unter verminderten Druck eingeengt und der verbleibende ölige Rückstand in Ethylacetat aufgenommen, nochmals filtriert und aus Diisopropylether kristallisiert.
Ausbeute: 12,1 g ( 72 % der Theorie), Schmelzpunkt: 126 - 127° C, C₁₇H₂₉N₄O₆P (MG = 416,4), ¹H-NMR (CDCl₃) δ = 1,31 (t, 6H); 1,45-1,70 (m, 6H); 3,32 (s, 3H); 3,56 (s, 3H); 3,69 (t, 2H); 3,95-4,15 (m, 6H); 4,47 (t, 2H); 7,63 (s, 1H)

### Beispiel 32

### [4-(3-Methyl-7-propyl-xanthin-1-yl)-butyl]-phosphonsäure-diethylester

Die Titelsubstanz wurde analog zu Beispiel 23 aus 0,072 mol 3-Methyl-7-propylxanthin und 0,072 mol 4-Chlorbutanphosphonsäurediethylester hergestellt, über Kieselgel chromatographiert (Eluent: Dichlormethan/Methanol 20:1) und aus Diisopropylether kristallisiert.
Ausbeute: 14,4 g (50 % der Theorie), Schmelzpunkt: 88-90° C, C₁₇H₂₉N₄O₅P (MG = 400,4), ¹H-NMR (CDCl₃) d= 0,94 (t, 3H); 1,30 (t, 6H); 1,56-1,98 (m, 8H); 3,56 (s, 3H); 3,95-4,15 (m, 6H); 4,22 (t, 2H); 7,52 (s, 1H) ppm

### Beispiel 33

### [3-(3,7-Dimethylxanthin-1-yl)-propyl]-phosphonsäurediethylester

Die Titelsubstanz wurde analog zu Beispiel 32 aus 0,067 mol Theobromin und 0,08 mol 3-Brompropan-phosphonsäurediethylester hergestellt.
Ausbeute: 15,6 g (65% der Theorie), Schmelzpunkt: 50-56° C, C₁₄H₂₃N₄O₅P (MG = 358,3), ¹H-NMR (CDCl₃) δ = 1,30 (t, 6H); 1,70-2,10 (m, 4H); 3,59 (s, 3H); 4,00 (s, 3H); 4,05-4,18 (m, 6H); 7,73 (s, 1H) ppm

### Beispiel 34

### [3-(7-Ethyl-3-methylxanthin-1-yl)-propyl]-phosphonsäurediethylester

Die Titelsubstanz wurde analog zu Beispiel 32 aus 0,041 mol 7-Ethyl-3-methylxanthin und 0,049 mol 3-Brompropanphosphonsäurediethylester hergestellt. Ausbeute: 12,3 g (80 % der Theorie), Schmelzpunkt: 108° C, C₁₅H₂₅N₄O₅P (MG = 372,4), ¹H-NMR (CDCl₃) δ = 1,31 (t, 6H); 1,53 (t, 3H); 1,70-2,10 (m, 4H); 3,59 (s, 3H); 4,12-4,18 (m, 6H); 4,36 (q, 2H); 7,62 (s, 1H) ppm

### Beispiel 35

[3-(7-Methoxyethyl-3-methylxanthin-1-yl)-propyl]-phosphonsäure-diethylester Die Titelsubstanz wurde analog zu Beispiel 32 aus 0,048 mol 7-Methoxymethyl-3-methylxanthin und 0,058 mol 3-Brompropanphosphonsäurediethylester hergestellt.
Ausbeute: 8,6 g (45 % der Theorie), Öl, C₁₆H₂₇N₄O₅P (MG = 402,4), ¹H-NMR (CDCl₃) δ = 1,29 (t, 6H); 1,70-2,05 (m, 4H); 3,30 (s, 3H); 3,56 (s, 3H); 3,68 (t, 2H); 3,95-4,15 (m, 6H); 4,45 (t, 2H); 7,61 (s, 1H) ppm

### Beispiel 36

[3-(7-Ethoxymethyl-3-methylxanthin-1-yl)-propyl]-phosphonsäure-diethylester Die Titelsubstanz wurde analog zu Beispiel 32 aus 0,048 mol 7-Ethoxymethyl-3-methylxanthin und 0,058 mol 3-Brompropanphosphonsäurediethylester hergestellt und über Kieselgel chromatographiert (Eluent: Ethylacetat/Methanol 10:1). Ausbeute: 8,5 g (44 % der Theorie), Öl, C₁₆H₂₇N₄O₅P (MG = 402,4), ¹H-NMR (CDCl₃) δ = 1,17 (t, 3H); 1,28 (t, 6H); 1,70-2,05 (m, 4H); 3,56 (s, 3H); 3,59 (q, 2H); 3,95-4,15 (m, 6H); 5,68 (s, 2H); 7,73 (s, 1H) ppm

### Beispiel 37

### [4-(3-Ethylxanthin-1-yl)-butyl]-phosphonsäurediethylester

### a) 7-Benzyl-3-ethylxanthin

18 g (0,1 mol) 3-Ethylxanthin wurden mit 19,2 g (0,11 mol) Benzylbromid und 15,2 g (0,11 mol) Kaliumcarbonat 2 Stunden bei 60° C gerührt. Die Reaktionslösung wurde in einen Erlenmeyerkolben überführt und mit 1000 ml Wasser versetzt. Der Niederschlag wurde abgenutscht, mehrmals mit warmen Wasser gewaschen und bei 50° C unter verminderten Druck getrocknet.
Ausbeute: 24,3 g (90 % der Theorie), C₁₄H₁₄N₄O₂ (MG = 270,3)

### b) [4-(7-Benzyl-3-ethylxanthin-1-yl)-butyl]-phosphonsäurediethylester

Die Titelsubstanz wurde analog zu Beispiel 23 aus 0,04 mol 7-Benzyl-3-ethylxanthin und 0,048 mol 4-Chlorbutanphosphonsäurediethylester hergestellt. Ausbeute: 15,8 g (85 % der Theorie), C₂₂H₃₁N₄O₅P (MG = 462,5)

### c) [4-(3-Ethylxanthin-1-yl)-butyl]-phosphonsäurediethylester

Die Titelsubstanz wurde analog zu Beispiel 24 aus 0,03 mol [4-(7-Benzyl-3-ethylxanthin-1-yl)-butyl]-phosphonsäurediethylester hergestellt.
Ausbeute: 10,5 g (93 % der Theorie), Schmelzpunkt: 134° C, C₁₅H₂₅N₄O₅P (MG =372,4), ¹H-NMR (CDCl₃) δ = 1,23-1,40 (m, 9H); 1,60-1,95 (m, 6H); 3,98-4,25 (m, 8H); 7,82 (s 1H); 13,10 (s, 1H, NH) ppm

### Beispiel 38

### [4-(3-Ethyl-7-propylxanthin-1-yl)-butyl]-phosphonsäurediethylester

Die Titelsubstanz wurde analog zu Beispiel 30 aus 8,2 mmol [4-(3-Ethylxanthin-1-yl)-butyl]-phosphonsäurediethylester und 9,8 mmol Brompropanhergestellt und über Kieselgel chromatographiert (Eluent: Dichlormethan/Methanol 20:1).
Ausbeute: 2,7 g (80 % der Theorie), Schmelzpunkt: 102° C, C₁₈H₃₁N₄O₅P (MG = 414,45), ¹H-NMR (CDCl₃) δ = 0,95 (t, 3H); 1,30 (t, 6H); 1,33 (t, 3H); 1,55-1,98 (m, 8H); 3,95-4,28 (m, 10 H); 7,52 (s, 1H) ppm

### Beispiel 39

### [3-(3-Cyclopropyl-7-propylxanthin-1-yl)-butyl]-phosphonsäure-diethylester

### a) Cyclopropylharnstoff

400,5 g (6,87 mol) Cyclopropylamin wurden unter Eiskühlung mit 1400 ml 5 N Salzsäure und anschließend mit 568 g (6,87 mol) Kaliumcyanat versetzt. Die Lösung wurde noch 4 Stunden bei 70°C gerührt, im Wasserstrahlvakuum bis zur Trockene eingedampft und in 2000 ml Ethanol aufgenommen. Das ausgefallene Kaliumchlorid wurde abfiltriert, das Filtrat eingeengt und mit Petrolether versetzt. Der kristalline Niederschlag wurde abgenutscht, mit Petrolether gewaschen und an der Luft getrocknet.
Ausbeute: 659,7 g (95 % der Theorie, eventuell mit Kaliumchlorid verunreinigt), Schmelzpunkt: 100-120° C, C₄H₈N₂O (MG = 100)

### b) 1-Cyanacetyl-3-cyclopropylharnstoff

Zu einer Lösung von 329 g (3,875 mol) Cyanessigsäure in 476 g (4,57 mol) Acetanhydrid wurden bei einer Temperatur von 80°C 450 g (3,875 mol) Cyclopropylharnstoff langsam eingetragen. Die Lösung wurde auf 90°C erwärmt und 2 Stunden bei dieser Temperatur gerührt. Anschließend wurde auf 20° C abgedampft-kühlt, der Niederschlag abgesaugt, mit Wasser gewaschen und bis zur Gewichtskonstanz bei 80° C getrocknet.
Ausbeute: 503 g (78 % der Theorie), Schmelzpunkt: 179° C,
C₇H₉N₃O₂ (MG = 167,1)

### c) 1-Cyclopropyl-6-aminouracil

Die Cyclisierung von 1-Cyanacetyl-3-cyclopropylharnstoff (3 mol) erfolgte analog den bekannten Verfahren.
Ausbeute: 317 g (63 % der Theorie), Schmelzpunkt: 278° C, C₇H₉N₃O₂ (MG = 167,1)

### d) 3-Cyclopropylxanthin

Die Herstellung von 3-Cyclopropylxanthin aus 1-Cyclopropyl-6-aminouracil (1,88 mol) erfolgte analog zu den bekannten Verfahren.
Ausbeute: 174,6 g (48 % der Theorie), Schmelzpunkt: 294° C (Zersetzung), C₈H₈N₄O₂ (MG = 192,2)

### e) 3-Cyclopropyl-7-propylxanthin

9,6 g (0,05 mol) 3-Cyclopropylxanthin wurden in 150 ml Dimethylformamid suspendiert und langsam mit 1,45 g (0,06 mol) Natriumhydrid deprotoniert. Am Ende der Wasserstoffentwicklung wurde die Lösung auf 60° C erhitzt und tropfenweise mit 6,77 g (0,055 mol) Brompropan versetzt. Nach 3 Stunden Rühren bei 70° C wurde die Lösung filtriert, das Dimethylformamid unter verminderten Druck abgedampft und der Rückstand aus Diisopropylether kristallisiert.
Ausbeute: 7,5 g (64 % der Theorie), Schmelzpunkt: 163° C, C₁₁H₁₄N₄O₂ (MG = 234,3), ¹H-NMR (CDCl₃) δ = 0,94 (t, 3H); 1,00-1,25 (m, 4H); 1,80-2,00 (m, 2H); 2,89-3,02 (m, 1H); 4,22 (t, 2H); 7,56 (s, 1H); 8,45 (s, 1H, NH) ppm f) [3-(3-Cyclopropyl-7-propylxanthin-1-yl)-butyl]-phosphonsäurediethylester

Die Alkylierung von 3-Cyclopropyl-7-propylxanthin (0,015 mol) erfolgte gemäß Beispiel 4. Die Lösung wurde filtriert, eingeengt und der verbleibende Rückstand chromatographiert (Eluent: Dichlormethan/Methanol 15:1).
Ausbeute: 3,8 g (62 % der Theorie), Öl, C₁₈H₂₉N₄O₅P (MG = 412,4), ¹H-NMR (CDCl₃) δ = 0,93 (t, 3H); 0,95-1,20 (m, 4H); 1,29 (t, 6H); 1,70-2,05 (m, 6H); 2,92-3,05 (m, 1H); 3,95-4,13 (m, 6H); 4,21 (t, 2H); 7,53 (s, 1H); (H) ppm

### Beispiel 40

### [3-(3,7-Dibutylxanthin-1-yl)-propyl]-phosphonsäurediethylester

Die Titelsubstanz wurde analog zu Beispiel 23 aus 0,02 mol 3,7-Dibutylxanthin und 0,022 mol 3-Brompropanphosphonsäurediethylester hergestellt und über Kieselgel chromatographiert (Eluent: Dichlormethan/Methanol 20:1).
Ausbeute: 4,5 g (54 % der Theorie), Öl, C₂₀H₃₅N₄O₅P (MG = 442,5), ¹H-NMR (CDCl₃) δ = 0,93 (t, 6H); 1,20-1,45 (m, 10H); 1,63-2,05 (m, 8H); 3,95-4,16 (m, 8H); 4,23 (t, 2H); 7,50 (s, 1H) ppm

### Beispiel 41

### {4-[7-(3-Methylbutyl)-3-methylxanthin-1-yl)-butyl]-phosphonsäure-diethylester

### a) 7-(3-Methylbutyl)-3-methylxanthin

100 g (0,602 mol) 3-Methylxanthin, gelöst in 1200 ml DMF, wurden langsam mit 16 g (0,66 mol) Natriumhydrid versetzt. Die Lösung wurde auf 60° C erhitzt und am Ende der Wasserstoffentwicklung mit 109 g (0,66 mol) 1-Brom-3-methylbutan versetzt. Die Reaktionsmischung wurde 12 Stunden bei 100° C gerührt und in 2000 ml Wasser eingetragen. Der Niederschlag wurde abgedampft-nutscht, mit Wasser gewaschen und bis zur Gewichtskonstanz getrocknet.
Ausbeute: 113,5 g (80 % der Theorie), Schmelzpunkt: 204° C, C₁₁H₁₆N₄O₂ (MG = 236,3)

### b) {4-[7-(3-Methylbutyl)-3-methylxanthin-1-yl)-butyl]-phosphonsäurediethylester

Die Titelsubstanz wurde analog zu Beispiel 23 aus 0,0254 mol 7-(3-Methylbutyl)-3-methylxanthin und 0,03 mol 4-Chlorbutanphosphonsäurediethylester hergestellt und über Kieselgel chromatographiert (Eluent: Dichlormethan/ Methanol 20:1) und aus Diisopropylether kristallisiert.
Ausbeute: 2,2 g (20 % der Theorie), Schmelzpunkt: 118° C, C₁₉H₃₃N₄O₅P (MG = 428,5), ¹H-NMR (CDCl₃) δ = 0,96 (d, 6H); 1,31 (t, 6H); 1,54-1,90 (m, 9H); 3,56 (s, 3H); 3,95-4,18 (m, 6H); 4,30 (t, 2H); 7,52 (s, 1H) ppm

### Beispiel 42

### [3-(3-Methylxanthin-1-yl)-propyl]-phosphonsäurediethylester

### a) [7-Benzyl-3-(3-methylxanthin-1-yl)-propyl]-phosphonsäurediethylester

Die Titelsubstanz wurde analog zu Beispiel 23 aus 7-Benzyl-3-methylxanthin, (0,04 mol) und 3-Brompropanphosphonsäurediethylester (0,046 mol) hergestellt.
Ausbeute: 12 g (70 % der Theorie), Öl, C₂₀H₂₇N₄O₅P (MG = 434,5)

### b) [3-(3-Methylxanthin-1-yl)-propyl]-phosphonsäurediethylester

Die Titelsubstanz wurde analog zu Beispiel 24 aus 0,026 mol [7-Benzyl-3-(3-methylxanthin-1-yl)-propyl]-phosphonsäurediethylester hergestellt und aus Diisopropylether kristallisiert.
Ausbeute: 8,6 g (89 % der Theorie), Schmelzpunkt: 125° C, C₁₃H₂₁N₄O₅P (MG = 344,3), ¹H-NMR (CDCl₃) δ = 1,31 (t, 6H); 1,78-2,14 (m, 4H); 3,62 (s, 3H); 4,00-4,19 (m, 6H); 7,83 (s, 1H); 13,02 (s, 1H, NH) ppm

### Beispiel 43

### [5-(7-Benzyl-3-methylxanthin-1-yl)-pentyl]-phosphonsäurediethylester

Die Titelsubstanz wurde analog zu Beispiel 23 aus 7-Benzyl-3-methylxanthin (0,059 mol) und 5-Brompentanphosphonsäurediethylester (0,07 mol) hergestellt.
Ausbeute: 21,8 g (80 % der Theorie), Öl, C₂₂H₃₁N₄O₅P (MG = 462,5), ¹H-NMR (CDCl₃) δ = 1,30 (t, 6H); 1,40-1.82 (m, 8H); 3,56 (s, 3H); 3,95-4,15 (m, 6H); 5,48 (s, 2H); 7,32-7,36 (m, 5H); 7,54 (s, 1H) ppm

### Beispiel 44

### [3-(3-Methylxanthin-1-yl)-pentyl]-phosphonsäurediethylester

Die Titelsubstanz wurde analog zu Beispiel 24 aus 0,045 mol [7-Benzyl-3-(3-methylxanthin-1-yl)-pentyl]-phosphonsäurediethylester hergestellt und aus Ethylacetat/Diisopropylether kristallisiert.
Ausbeute: 11,7 g (70 % der Theorie), Schmelzpunkt: 109° C, C₁₅H₂₅N₄O₅P (MG = 372,4), ¹H-NMR (CDCl₃) δ = 1,31 (t, 6H); 1,38-1,88 (m, 8H); 3,63 (s, 3H); 4,00-4,18 (m, 6H); 7.82 (s, 1H); 12,98 (s, 1H, NH) ppm

### Beispiel 45

### [4-(3-Ethyl-7-methoxyethylxanthin-1-yl)-butyl]-phosphonsäure-diethylester

Die Titelsubstanz wurde analog zu Beispiel 30 aus [3-(3-Methylxanthin-1-yl)-pentyl]-phosphonsäurediethylester (0,008 mol, Beispiel 43) und Methoxyethylchlorid hergestellt. Ausbeute: 3,1 g (90 % der Theorie), Schmelzpunkt: 110° C, C₁₈H₃₁N₄O₆P (MG = 430,4), ¹H-NMR (CDCl₃) δ = 1,30 (t, 6H); 1,33 (t, 3H); 1,60-1,78 (m, 6H); 3,32 (s, 3H); 3,69 (t, 2H); 3,93-4,21 (m, 8H); 4,45 (t, 2H); 7,61 (s, 1H) ppm

### Beispiel 46

### [3-(7-Cyclopropylmethyl-3-methylxanthin-1-yl)-propyl]-phosphonsäurediethylester

Die Titelsubstanz wurde analog zu Beispiel 30 aus 0,015 mol [3-(3-Methylxanthin-1-yl)-propyl]-phosphonsäurediethylester (Beispiel 42) und 0,017 mol Chlormethylcyclopropan hergestellt und über Kieselgel chromatographiert (Eluent: Dichlormethan/Methanol 20:1).
Ausbeute: 3,5 g (59 % der Theorie), Schmelzpunkt: 86-87° C, C₁₇H₂₇N₄O₅P (MG = 398,4), ¹H-NMR (CDCl₃) δ = 0,38-0,45 (m, 2H); 0,60-0,71 (m, 2H); 1,30 (t, 6H); 1,25-1,40 (m, 1H); 1,70-2,05 (m, 4H); 3,57 (s, 3H); 4,00-4,19 (m, 8H); 7,64 (s, 1H) ppm

### Beispiel 47

### [3-(7-Methylethylxanthin-1-yl)-propyl]-phosphonsäurediethylester

Die Titelsubstanz wurde analog zu Beispiel 46 aus 0,02 mol 7-lsopropyl-3-methylxanthin und 0,022 mol 3-Brompropanphosphonsäurediethylester hergestellt.
Ausbeute: 5,4 g (70 % der Theorie), Schmelzpunkt: 134° C, C₁₆H₂₇N₄O₅P (MG = 386,4), ¹H-NMR (CDCl₃) δ = 1,31 (t, 6H); 1,57 (d, 6H); 1,65-2,05 (m, 4H); 3,57 (s, 3H); 4,02-4,18 (m, 6H); 5,02 (s, 1H); 7,65 (s, 1H) ppm

### Beispiel 48

### [5-(7-Butyl-3-methylxanthin-1-yl)-pentyl]-phosphonsäurediethylester

Die Titelsubstanz wurde analog zu Beispiel 46 aus 0,008 mol [5-(3-Methylxanthin-1-yl)-pentyl]-phosphonsäurediethylester und 0,01 mol 1-Brombutan hergestellt.
Ausbeute: 2,9 g (85 % der Theorie), Öl, C₁₉H₃₃N₄O₅P (MG 428,5), ¹H-NMR (CDCl₃) δ = 0,94 (t, 3H); 1,29 (t, 6H); 1,29-1,95 (m, 12H); 3,55 (s, 3H); 3,93-4,14 (m, 6H); 4,26 (t, 2H); 7,51 (s, 1H) ppm

### Beispiel 49

### [5-(7-Cyclopropylmethyl-3-methylxanthin-1-yl)-pentyl]-phosphonsäurediethylester

Die Titelsubstanz wurde analog zu Beispiel 46 aus 0,011 mol [5-(3-Methylxanthin-1-yl)-pentyl]-phosphonsäurediethylester und 0,013 mol Chlormethylcyclopropan hergestellt.
Ausbeute: 2,7 g (58 % der Theorie), Öl, C₁₉H₃₁N₄O₅P (MG = 426,5), ¹H-NMR (CDCl₃) δ = 0,35-0,45 (m, 2H); 0,60-0,70 (m, 2H); 1,28 (t, 6H); 1,31-1,78 (m, 9H); 3,55 (s, 3H); 3,92-4,18 (m, 8H); 7,62 (s, 1H) ppm

### Beispiel 50

### [4-(7-Cyclopropylmethyl-3-methylxanthin-1-yl)-butyl]-phosphonsäurediethylester

Die Titelsubstanz wurde analog zu Beispiel 46 aus 0,016 mol [5-(3-Methylxanthin-1-yl)-butyl]-phosphonsäurediethylester und 0,02 mol Chlormethylcyclopropan hergestellt.
Ausbeute: 4,8 g (73 % der Theorie), Schmelzpunkt: 104° C, C₁₈H₂₉N₄O₅P (MG = 412,4), ¹H-NMR (CDCl₃) δ = 0,38-0,48 (m, 2H); 0,62-0,72 (m, 2H); 1,30 (t, 6H); 1,30-1,42 (m, 1H); 1,60-1,88 (m, 6H); 3,57 (s, 3H); 3,98-4,20 (m, 8H); 7,63 (s, 1H) ppm

### Beispiel 51

### {4-[7-(2-Methylpropyl)-3-methylxanthin-1-yl]-butyl}-phosphonsäure-diethylester

Die Titelsubstanz wurde analog zu Beispiel 46 aus 0,016 mol [5-(3-Methylxanthin-1-yl)-butyl]-phosphonsäurediethylester und 0,02 mol 1-Brom-2-methylpropan hergestellt und aus Ethylacetat/Diisopropylether kristallisiert.
Ausbeute: 3,5 g (53 % der Theorie), Schmelzpunkt: 80° C, C₁₈H₃₁N₄O₅P (MG = 414,45), ¹H-NMR (CDCl₃) δ = 0,93 (d, 6H); 1,30 (t, 6H); 1,60-1,88 (m, 6H); 2,21 (s, 1H); 3,56 (s, 3H); 3,95-4,18 (m, 8H); 7,49 (s, 1H) ppm

### Beispiel 52

### [4-(7-Methylethylxanthin-1-yl)-butyl]-phosphonsäurediethylester

Die Titelsubstanz wurde analog zu Beispiel 46 aus 0,016 mol 5-(3-Methylxanthin-1-yl)-butyl]-phosphonsäurediethylester und 0,02 mol 2-Brompropan hergestellt. Ausbeute: 4,7 g (73 % der Theorie), Schmelzpunkt: 78-80° C, C₁₇H₂₉N₄O₅P (MG = 400,4), ¹H-NMR (CDCl₃) δ = 1,29 (t, 6H); 1,56 (d, 6H); 1,65-2,05 (m, 6H); 3,56 (s, 3H); 4,00-4,18 (m, 6H); 5,02 (se, 1H); 7,63 (s, 1H) ppm

### Beispiel 53

### [4-(3-Benzyl-7-ethoxymethylxanthin-1-yl)-butyl]-phosphonsäure-diethylester

### a) 3-Benzyl-7-ethoxymethylxanthin

Die Titelsubstanz wurde analog zu 7-Ethoxymethyl-3-methylxanthin (Beispiel 3) hergestellt. Ausbeute: 12,3 g (82 % der Theorie), C₉H₁₆N₄O₃ (MG = 300,3) b) [4-(3-Benzyl-7-ethoxymethylxanthin-1-yl)-butyl]-phosphonsäurediethylester Die Titelsubstanz wurde analog zu Beispiel 23 aus 0,037 mol 3-Benzyl-7-ethoxymethylxanthin und 0,045 mol 4-Chlorbutanphosphonsäurediethylester hergestellt und über Kieselgel chromatographiert (Eluent: Dichlormethan/ Methanol 20:1). Ausbeute: 8,8 g (48 % der Theorie), Öl, C₂₃H₃₃N₄O₆P (MG = 492,5), ¹H-NMR (CDCl₃) δ = 1,10-1,35 (m, 9H); 1,55-1,94 (m, 6H); 3,61 (q, 2H); 3,93-4,18 (m, 6H); 5,26 (s, 2H); 5,67 (s, 2H); 7,22-7,38 (m, 3H); 7,43-7,53 (m, 2H); 7,74 (s, 1H) ppm

### Beispiel 54

### [4-(7-Ethoxymethyl-3-ethylxanthin-1-yl)-butyl]-phosphonsäure-diethylester

### a) 7-Ethoxymethyl-3-ethylxanthin

Die Titelsubstanz wurde analog zu Beispiel 53 a) hergestellt.
Ausbeute: 4,5 g (70 % der Theorie), C₁₀H₁₄N₄O₃ (MG = 236,2)

### b) [4-(7-Ethoxymethyl-3-ethylxanthin-1-yl)-butyl]-phosphonsäurediethylester

Die Titelsubstanz wurde analog zu 53 aus 0,015 mol 7-Ethoxymethyl-3-ethylxanthin und 0,017 mol 4-Chlorbutanphosphonsäurediethylester hergestellt. Ausbeute: 5,03 g (78% der Theorie), Schmelzpunkt: 80°C, C₁₈H₃₁N₄O₆P (MG = 430,4), ¹H-NMR (CDCl₃) δ = 1,20 (t, 3H); 1,30 (t, 6H); 1,33 (t, 3H); 1,58-1,90 (m, 6H); 3,62 (q, 2H); 3,95-4,22 (m, 8H); 5,68 (s, 2H); 7,73 (s, 1H) ppm

### Beispiel 55

### [3-(3-Methyl-7-propylxanthin-1-yl)-propyl]-phosphonsäure-diethylester

Die Titelsubstanz wurde analog zu Beispiel 23 aus 0,048 mol 3-Methyl-7-propylxanthin und 0,058 mol 3-Brompropanphosphonsäurediethylester hergestellt.
Ausbeute: 14,7 g (79 % der Theorie), Schmelzpunkt: 77-79° C, C₁₆H₂₇N₄O₅P (MG = 386,4), ¹H-NMR (CDCl₃) δ = 0,93 (t, 3H); 1,29 (t, 6H); 1,70-2,05 (m, 6H); 3,56 (s, 3H); 4,00-4,18 (m, 6H); 4,23 (t, 2H); 7,52 (s, 1H) ppm

### Beispiel 56

### 5-[7-(3-Methylbutyl)-3-methylxanthin-1-yl)-propyl]-phosphonsäure-diethylester

Die Titelsubstanz wurde analog zu Beispiel 46 aus 0,025 mol 7-(3-Methylbutyl)-3-methylxanthin (siehe Beispiel 41) und 0,028 mol 5-Brompentanphosphonsäurediethylester hergestellt.
Ausbeute: 4,8 g (43 % der Theorie), Öl, C₂₀H₃₅N₄O₅P (MG = 442,5), ¹H-NMR (CDCl₃) δ = 0,96 (d, 6H); 1,30(t, 6H); 1,36-1,84 (m, 11H); 3,56 (s, 3H); 3,93-4,12 (m, 6H); 4,30 (t, 2H); 7,52 (s, 1H) ppm

### Beispiel 57

### [5-(3-Ethyl-7-propylxanthin-1-yl)-pentyl]-phosphonsäurediethylester

Die Titelsubstanz wurde analog zu Beispiel 46 aus 0,0225 mol 3-Ethyl-7-propylxanthin und 0,027 mol 5-Brompentanphosphonsäurediethylester hergestellt.
Ausbeute: 3 g (31% der Theorie), Öl, C₁₉H₃₃N₄O₅P (MG = 428,5), ¹H-NMR (CDCl₃) δ = 0,93 (t, 3H); 1,28 (t, 6H); 1,32 (t, 3H); 1,40-1,93 (m, 10H); 3,90-4,23 (m, 10H); 7,50 (s, 1H) ppm

### Beispiel 58

### [4-(8-Brom-3-methylxanthin-1-yl)-propyl]-phosphonsäurediethylester

Zu einer Lösung von 20 g (0,058 mol) [3-(3-Methylxanthin-1-yl)-propyl]-phosphonsäurediethylester (Beispiel 42) und 6,24 g (0,076 mol) Natriumacetat in 150 ml Essigsäure wurden bei 20°C 12,15 g (0,076 mol) Brom zu getropft und noch 5 Stunden bei 50° C gerührt. Die Essigsäure wurde abgedampft, der Rückstand in Dichlormethan aufgenommen, mit Natriumthiosulfatlösung und Wasser gewaschen, und über Natriumsulfat getrocknet. Es wurde unter verminderten Druck abgedampft, getrocknet und aus Wasser kristallisiert.
Ausbeute: 19,3 g (79 % der Theorie), Schmelzpunkt: 190-192° C, C₁₃H₂₀BrN₄O₅P (MG = 423,2), ¹H-NMR (CDCl₃) δ = 1,31 (t, 6H); 1,78-2,13 (m, 4H); 3,58 (s, 3H); 4,02-4,18 (m, 6H); 13,60 (s, 1H, NH) ppm

### Beispiel 59

### [1-(3,7-Dimethylxanthin-1-yl)-methyl]-dimethylphosphinoxid

Eine Lösung von 20,2 g (0,1 mol) Theophyllin Natriumsalz in 400 ml DMF wurde mit 12,7 g Chlormethyldimethylphosphinoxid 3 Stunden auf 130°C erhitzt. Die Lösung wurde filtriert, das Lösungsmittel abgedampft und der Rückstand in Methanol aufgenommen. Der Niederschlag wurde abfiltriert und unter verminderten Druck bis zur Gewichtskonstanz getrocknet.
Ausbeute: 17,2 g (64 % der Theorie), Schmelzpunkt: 250-252° C, C₁₀H₁₅N₄O₃P (MG = 270,2)

### Beispiel 60

### [1-(1,3-Dimethylxanthin-7-yl)-methyl]-dimethylphosphinoxid

Die Titelsubstanz wurde analog zu Beispiel 59 aus 20,2 g (0,1 mol) Theobromin Natriumsalz und Chlormethyldimethylphosphinoxid hergestellt und aus Ethylacetat kristallisiert.
Ausbeute: 23 g (87% der Theorie), Schmelzpunkt: 206-210° C, C₁₀H₁₅N₄O₃P (MG = 270,2)

### Beispiel 61

### [1-(7-Dimethylphosphinyl-3-methylxanthin-1-yl)-methyl]-dimethyl-phosphinoxid

Die Titelsubstanz wurde analog zu Beispiel 59 aus 22 g (0,1 mol) 3-Methylxanthin Dinatriumsalz und Chlormethyldimethylphosphinoxid hergestellt und aus Methanol kristallisiert.
Ausbeute: 30 g (86 % der Theorie), Schmelzpunkt: 250-254° C, C₁₂H₂₀N₄O₄P (MG = 346,3)

### Beispiel 62

### [1-(1-Hexyl-3-methylxanthin-7-yl)-methyl]-dimethylphosphinoxid

Die Titelsubstanz wurde analog zu Beispiel 59 aus 26 g (0,1 mol) 1-Hexyl-3-methylxanthin Natriumsalz und Chlormethyldimethylphosphinoxid hergestellt und aus Hexan kristallisiert.
Ausbeute: 19 g (56 % der Theorie), Schmelzpunkt: 132-135° C, C₁₅H₂₅N₄O₃P (MG = 340,4)

### Beispiel 63

### [1-(8-Brom-3,7-dimethylxanthin-1-yl)-methyl]-dimethylphosphinoxid

Die Titelsubstanz wurde analog zu Beispiel 59 aus 28 g (0,1 mol) 8-Brom-3,7-dimethylxanthin Natriumsalz und Chlormethyldimethylphosphinoxid hergestellt und über Kieselgel chromatographiert.
Ausbeute: 9,6 g (34 % der Theorie), Schmelzpunkt: 268-272° C, C₁₀H₁₄BrN₄O₃P (MG = 349,1)

### Beispiel 64

### [4-(1,3-Dimethylxanthin-7-yl)-butyl]-dimethylphosphinoxid

Die Titelsubstanz wurde analog zu Beispiel 63 aus 10,1 g (0,05 mol) 1,3-Dimethylxanthin Natriumsalz und 4-Chlorbutyldimethylphosphinoxid hergestellt. Ausbeute: 5,5 g (35 % der Theorie), Schmelzpunkt: 195-203° C, C₁₃H₂₁N₄O₃P (MG = 312,31)

### Beispiel 65

### [1-(3,7-Dimethylxanthin-1-yl)-methyl]-dimethylphosphinoxid

Die Titelsubstanz wurde analog zu Beispiel 63 aus 10,1 g (0,05 mol) 3,7-Dimethylxanthin Natriumsalz und 4-Chlorbutyldimethylphosphinoxid hergestellt.
Ausbeute: 2,5 g (16 % der Theorie), Schmelzpunkt: 148-150° C, C₁₃H₂₁N₄O₃P (MG = 312,3)

### Beispiel 66

### [3-(3,7-Dimethylxanthin-1-yl)-propyl]-dimethylphosphinoxid

Die Titelsubstanz wurde analog zu Beispiel 59 aus 10,1 g (0,05 mol) 3,7-Dimethylxanthin Natriumsalz und 3-Chlorpropyldimethylphosphinoxid hergestellt und aus Ethylacetat kristallisiert.
Ausbeute: 4 g (27 % der Theorie), Schmelzpunkt: 167-168° C, C₁₂H₁₉N₄O₃P (MG = 298,3)

### Beispiel 67

### [1-(1,3-Dibutylxanthin-7-yl)-methyl]-dimethylphosphinoxid

5 g (0,019 mol) 1,3-Dibutlyxanthin wurden mit 2,88 g (0,0228 mol) Chlormethyldimethylphosphinoxid und 3,2 g (0,0228 mol) Kaliumcarbonat in 80 ml DMF 6 Stunden lang bei 80° C gerührt. Die Lösung wurde auf Raumtemperatur abgekühlt, filtriert und das Filtrat unter verminderten Druck eingeengt. Der verbleibende Rückstand wurde in Petrolether/Ethylacetat kristallisiert.
Ausbeute: 4 g (59 % der Theorie), Schmelzpunkt: 148° C, C₁₆H₂₇N₄O₃P (MG 354,4), ¹H-NMR (CDCl₃) δ = 0,95 (t, 3H); 0,97 (t, 3H); 1,30-1,73 (m, 14H); 4,00 (t, 2H); 4,12 (t, 2H); 4,82 (d, 2H); 7,85 (s, 1H) ppm

### Beispiel 68

### [1-(1,3-Dipropylxanthin-7-yl)-methyl]-dimethylphosphinoxid

Die Titelsubstanz wurde analog zu Beispiel 67 aus 5 g (0,0186 mol) 1,3-Dipropylxanthin und 2,8 g (0,0224 mol) Chlormethyldimethylphosphinoxid hergestellt. Ausbeute: 4,9 g (82 % der Theorie), Schmelzpunkt: 172° C, C₁₄H₂₃N₄O₃P (MG = 326,3), ¹H-NMR (CDCl₃) δ = 0,94 (t, 3H); 0,97 (t, 3H); 1,57 (d, 6H); 1,57-1,88 (m, 4H); 3,91-4,12 (m, 4H); 4,81 (d, 2H); 7,84 (d, 1H) ppm

### Beispiel 69

### [1-(7-Benzyl-3-phenylxanthin-1-yl)-methyl]-dimethylphosphinoxid

### a) 7-Benzyl-3-phenylxanthin

Zu einer Suspension von 30 g (0,1315 mol) 3-Phenylxanthin in 600 ml DMF wurden portionsweise 3,2 g (0,1315 mol) Natriumhydrid gegeben, 30 Minuten bei 25° C gerührt und anschließend auf 80°C erhitzt. Dann wurden 27 g (0,1578 mol) Benzylbromid zugegeben und die Lösung 6 Stunden bei 80° C belassen. Nach Abkühlen auf 50° C wurde die Reaktionsmischung in 2000 ml Eiswasser gegossen, der erhaltene Niederschlag abgesaugt, gut mit Wasser gewaschen und unter verminderten Druck getrocknet. Der trockene Rückstand wurde aus Dioxan/Methanol kristallisiert. Ausbeute: 27 g (63 % der Theorie), Schmelzpunkt: 246° C, C₁₈H₁₄N₄O₂ (MG = 318,3)

### b) [1-(7-Benzyl-3-phenylxanthin-1-yl)-methyl]-dimethylphosphinoxid

Die Titelsubstanz wurde analog zu Beispiel 67 aus 6,5 g (0,02 mol) 7-Benzyl-3-phenylxanthin und 3,1 g (0,0245 mol) Chlormethyldimethylphosphinoxid hergestellt. Ausbeute: 6,5 g (78% der Theorie), Schmelzpunkt: 232-234° C, C₂₁H₂₁N₄O₃P (MG = 408,4), ¹H-NMR (CDCl₃) δ = 1,62 (d, 6H); 4,53 (d, 2H); 5,51 (s, 2H); 7,37-7,58 (m, 11H) ppm

### Beispiel 70

### [1-(3-Phenylxanthin-1-yl)-methyl]-dimethylphosphinoxid

Die Hydrierung von 6,4 g (0,0157 mol) 1-(7-Benzyl-3-phenylxanthin-1-yl)-methyl]-dimethylphosphinoxid erfolgte analog zu Beispiel 24.
Ausbeute: 3,9 g (61% der Theorie), Schmelzpunkt: 312-314° C, C₁₄H₁₅N₄O₃P (MG = 318,3), ¹H-NMR (CDCl₃) δ = 1,49 (d, 6H); 4,32 (d, 2H); 7,40-7,59 (m, 5H); 8,00 (s, 1H); 13,75 (s, 1H, NH) ppm

### Beispiel 71

### [1-(7-Cyclopropylmethyl-3-phenylxanthin-1-yl)-methyl]-dimethylphosphinoxid

3,9 g (0,0123 mol) [1-(3-Phenylxanthin-1-yl)-methyl]-dimethylphosphinoxid (Beispiel 70) wurden mit 1,33 g (0,0147 mol) Chlormethylcyclopropan und 2 g Kaliumkarbonat 6 Stunden lang bei 70° C gerührt. Die Lösung wurde filtriert, eingeengt und der verbleibende Rückstand auf Kieselgel chromatographiert (Eluent: Ethylacetat/ Methanol 10:1). Ausbeute: 2,5 g (55% der Theorie), Schmelzpunkt: 226° C, C₁₈H₂₁N₄O₃P (MG 372,4), ¹H-NMR (CDCl₃) δ = 0,40-0,49 (m, 2H); 0,63-0,72 (m, 2H); 1,27-1,45 (m, 1H); 1,63 (d, 6H); 4,19 (d, 2H); 4,53 (d, 2H); 7,40-7,60 (m, 5H); 7,61 (s, 1H) ppm

### Beispiel 72

### [1-(3,7-Dibutylxanthin-1-yl)-methyl]-dimethylphosphinoxid

Die Titelsubstanz wurde analog zu Beispiel 67 aus 5 g (0,0189 mol) 3,7-Dibutylxanthin und 2,9 g (0.0227 mol) Chlormethyldimethylphosphinoxid hergestellt.
Ausbeute: 4,3 g (64% der Theorie), Schmelzpunkt: 124° C, C₁₆H₂₇N₄O₃P (MG = 354,4), ¹H-NMR (CDCl₃) δ = 0,95 (t, 6H); 1,22-1.48 (m, 4H); 1,60 (d, 6H); 1,63-1,92 (m, 4H); 4,10 (t, 2H); 4,28 (t, 2H); 4,49 (d, 2H); 7,54 (s, 1H)ppm

### Beispiel 73

### [1-(3-Cyclopropyl-7-propylxanthin-1-yl}-methyl]-dimethylphosphinoxid

Die Titelsubstanz wurde analog zu Beispiel 67 aus 3,2 g (0,0137 mol) 3-Cyclopropyl-7-propylxanthin (siehe Beispiel 39) und 2,07 g (0,0164 mol) Chlormethyldimethylphosphinoxid hergestellt. Ausbeute: 3,6 g (81% der Theorie), Schmelzpunkt: 177-179° C, C₁₄H₂₁N₄O₃P (MG = 324,3), ¹H-NMR (CDCl₃) δ = 0,93 (t, 3H); 0,95-1,25 (m, 4H); 1,60 (d, 6H); 1,82-1,95 (m, 2H); 2,95-3,06 (m, 1H); 4,23 (t, 2H); 4,46 (d, 2H); 7,56 (s, 1H) ppm

### Beispiel 74

### [1-(7-Ethyl-3-methylxanthin-1-yl)-methyl]-dimethylphosphinoxid

Die Titelsubstanz wurde analog zu Beispiel 67 aus 5 g (0,0258 mol) 7-Ethyl-3-methylxanthin und 3.91 g (0,031 mol) Chlormethyldimethylphosphinoxid hergestellt. Ausbeute: 6 g (82% der Theorie), Schmelzpunkt: 164° C, C₁₁H₁₇N₄O₃P (MG = 284,3), ¹H-NMR (CDCl₃) δ = 1,50 (t, 3H); 1,61 (d, 6H); 3,58 (s, 3H); 4,33 (q, 2H); 4,80 (d, 2H); 7,58 (s, 1H) ppm
Pharmakologischen Prüfungen

### 1. Wirksamkeit im Muskelfunktionstest der Ratte

Männliche Wistar Ratten (Körpergewicht ca. 300 g) wurden unter Standard-Bedingungen gehalten mit Futter und Wasser ad libitum. Die rechte hintere Extremität wurde ruhiggestellt (immobilisiert), indem den Tieren unter Kurzzeit-Narkose eine Art "Schuh" angepaßt wurde. Aus einer flüssigen Plastikmasse, die auch zur Herstellung von Zahnprothesen verwendet wird, wurden zwei ergonomisch angepaßte Hälften des "Schuhs" gegossen, diese den Tieren unter kurzer Narkose angelegt und mit Hilfe eines Drahtes zusammengefügt. Das Material ist leicht aber hart, so daß die Tiere das Material nicht anknabbern können, und der "Schuh" kann jederzeit geöffnet werden, um den Zustand der ruhiggestellten Extremität begutachten zu können. Die beiden Hälften wurden darüber hinaus mit Fensterleder ausgepolstert, um Kompressionen und mögliche Verletzungen durch Reibung zu vermeiden. Das System hat auch den Vorteil, daß der Winkel der Ruhigstellung bei allen Tieren standardisiert gleich ist; dies wäre bei einem Gipsverband nicht möglich. Der gewählte Winkel erlaubt es den Tieren, trotz "Schuh" eine normale Sitzposition einzunehmen und auch das Körpergewicht mit dieser Extremität zu tragen. Nur eine komplette Streckung oder Beugung des Beines ist nicht möglich.
Während der 3 Wochen Immobilisation zeigten die Tiere ein normales Verhalten, eine normale Körpergewichtsentwicklung und Ambulation im Käfig.
Trotzdem trat eine signifikante Verringerung der Skelettmuskel-Masse (Atrophie) der immobilisierten Extremität auf und zwar bis zu - 35 % im Vergleich zu der kontralateralen nicht - immobilisierten hinteren Extremität. Da der Soleus-Muskel am stärksten betroffen war beziehen sich die in Tabelle 1 angeführten Änderungen im Muskelgewicht nach Präparatgabe auf diesen Muskel. Ein Unterschied von ≤ 25 % zwischen der rechten immobilisierten und linken nicht immobilisierten Hinterextremität wurde als Wirkung eingestuft.
Nach 3 Wochen Ruhigstellung der rechten hinteren Extremität und gleichzeitiger oraler Gabe von Testpräparaten (1 x pro Tag mit Schlundsonde; in der Regel 25 mg/kg Körpergewicht, in Carboxymethylcellulose, wurden die Tiere gewogen und mit Pentobarbital (35 mg/kg i.p.) narkotisiert. Funktionelle Parameter wurden während einer direkten elektrischen Stimulation einer Muskelgruppe (Gastrocnemius, Plantaris und Soleus) gemessen. Diese Muskelgruppe, im folgenden nur "Muskel" genannt, wurde am Sehnenansatz des Knöchels freipräpariert und über die Sehne an einen Kraftaufnehmer angebunden.

Für die Erfassung der Kontraktionskraft wurden die Muskeln isometrisch stimuliert, d.h. eine Vorlast von 0,5 N wurde angelegt und der Muskel wurde über eine Serie von elektrischen Pulsen, die zu einer maximalen Kontraktion führten, jede 0,7 sec für 80 msec für die Dauer von 15 min, gereizt. Über die Zeit fällt die Kontraktionskurve aufgrund der Ermüdbarkeit des Muskels ab,
wobei der Abfall des atrophischen Muskels signifikant stärker ist als der Abfall des kontralateralen normalen Muskels. Die Fläche unter dieser Kontraktionskurve wurde ausgewertet und der Unterschied in Prozent zwischen dem rechten atrophischen und dem linken normalen Muskel verglichen (Kraft unter der Kontraktionskurve als Differenz in Prozent (Δ %)). Die Differenz beträgt in der Kontrollgruppe etwa 25 %.
Eine isotonische Muskelreizung wurde angewendet um die geleistete Arbeit des Muskels über die Zeit zu erfassen. Bei dieser Art der Messung wird die Verkürzung des Muskels während der Kontraktion zugrunde gelegt. Hierzu wurde dem Muskel eine Vorlast angelegt, die dem Körpergewicht entspricht. Eine Serie von Stimulationspulsen (80 msec) wurde jede 3 sec ausgelöst und der Muskel für 15 min gereizt. Die Daten (Kontraktionskraft und zurückgelegter Weg) wurde mit einem automatisierten System umgerechnet auf die geleistete Arbeit in der Einheit J. Die Differenz der geleisteten Arbeit zwischen dem immobilisierten atrophischen und dem normalen Muskel betrug bei den Kontrolltieren etwa -25 %.

Ein Präparat, das einen rechts/links Unterschied von -12 % (Δ %) bei der isotonischen und/oder isometrischen Funktionsmessung zeigte, wurde als wirksam eingestuft. Werte, die positiv werden, weisen darauf hin, daß die funktionellen Parametern des immobilisierten Muskels besser waren als die des kontralateralen normalen Muskels.

Die Ergebnisse sind in Tabelle I aufgeführt.

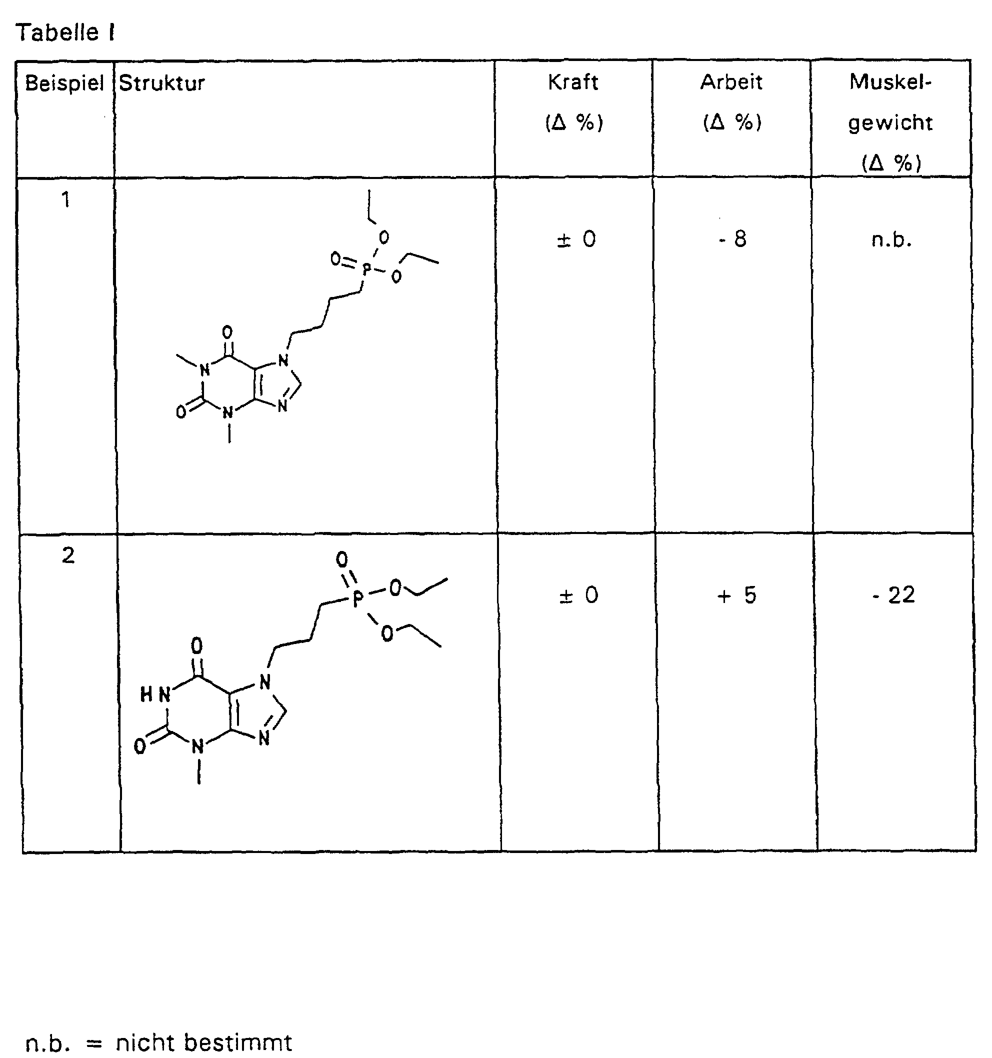

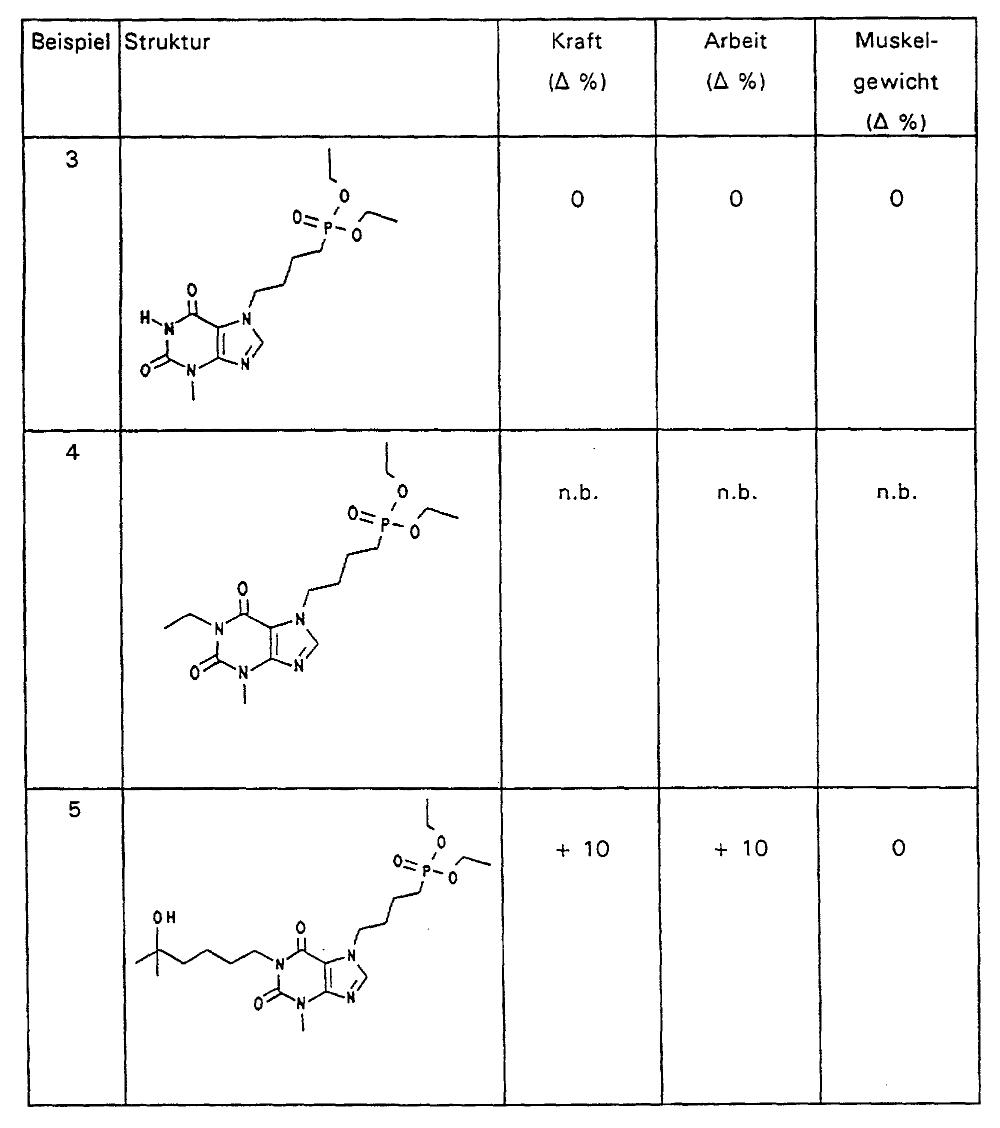

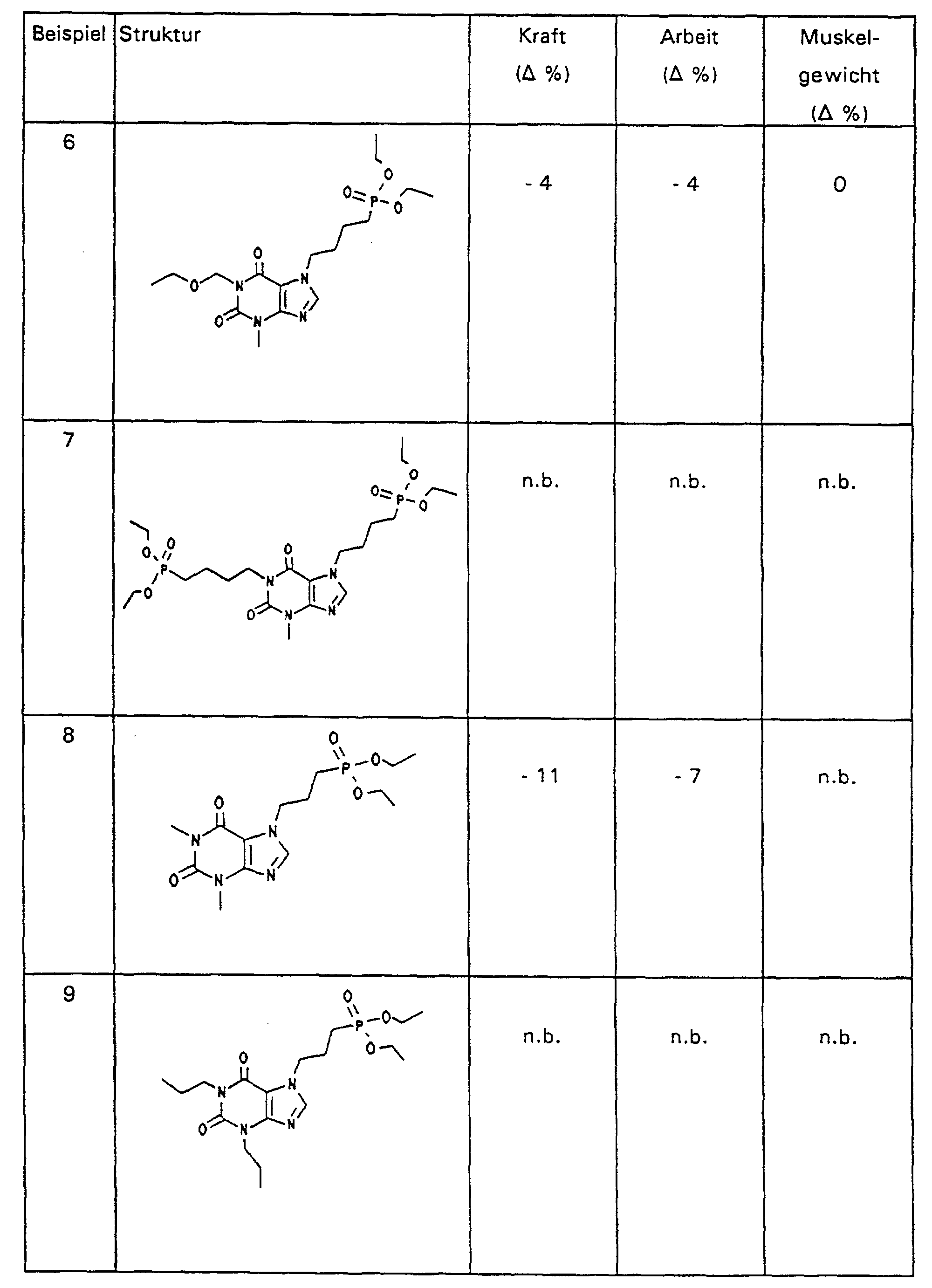

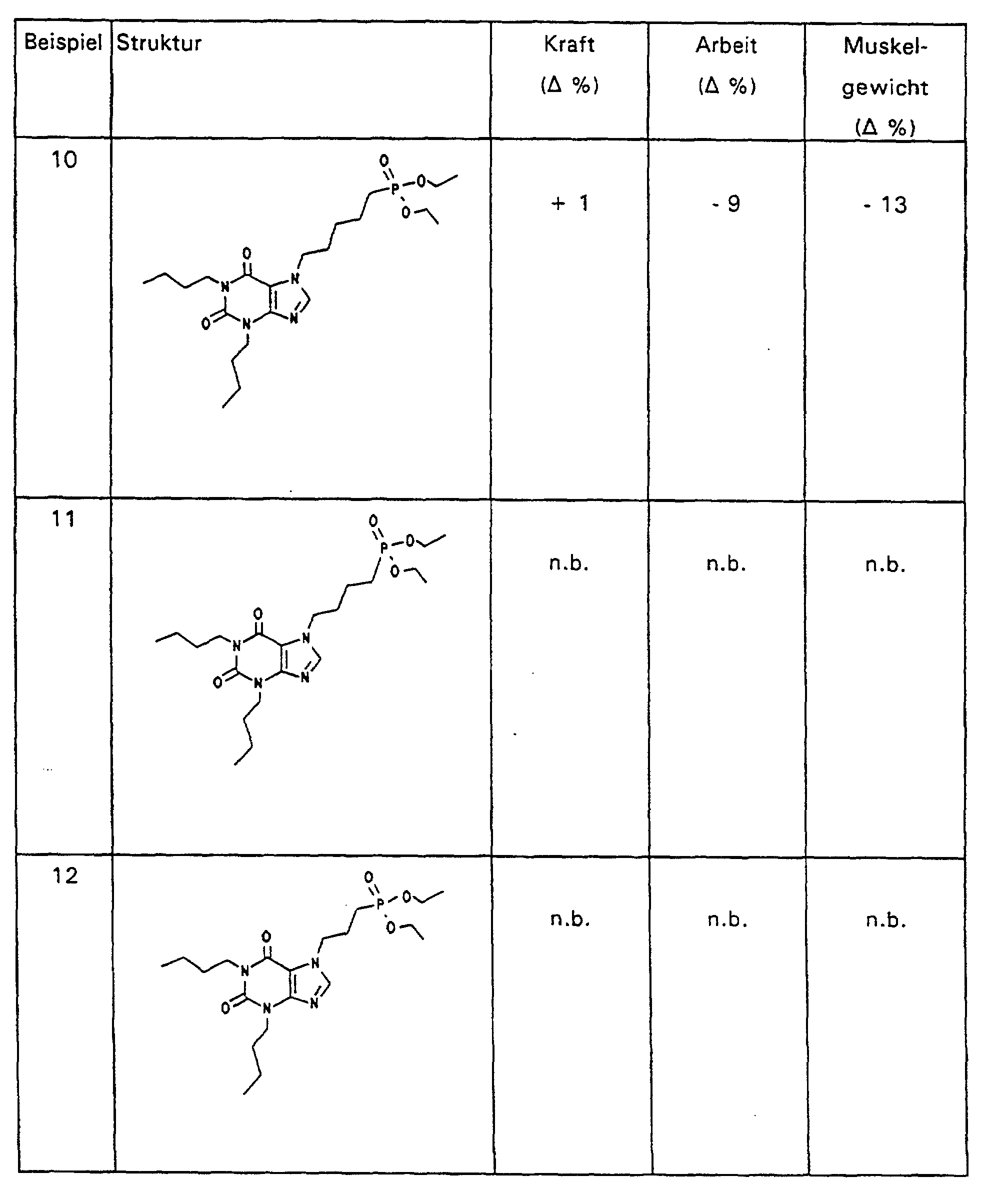

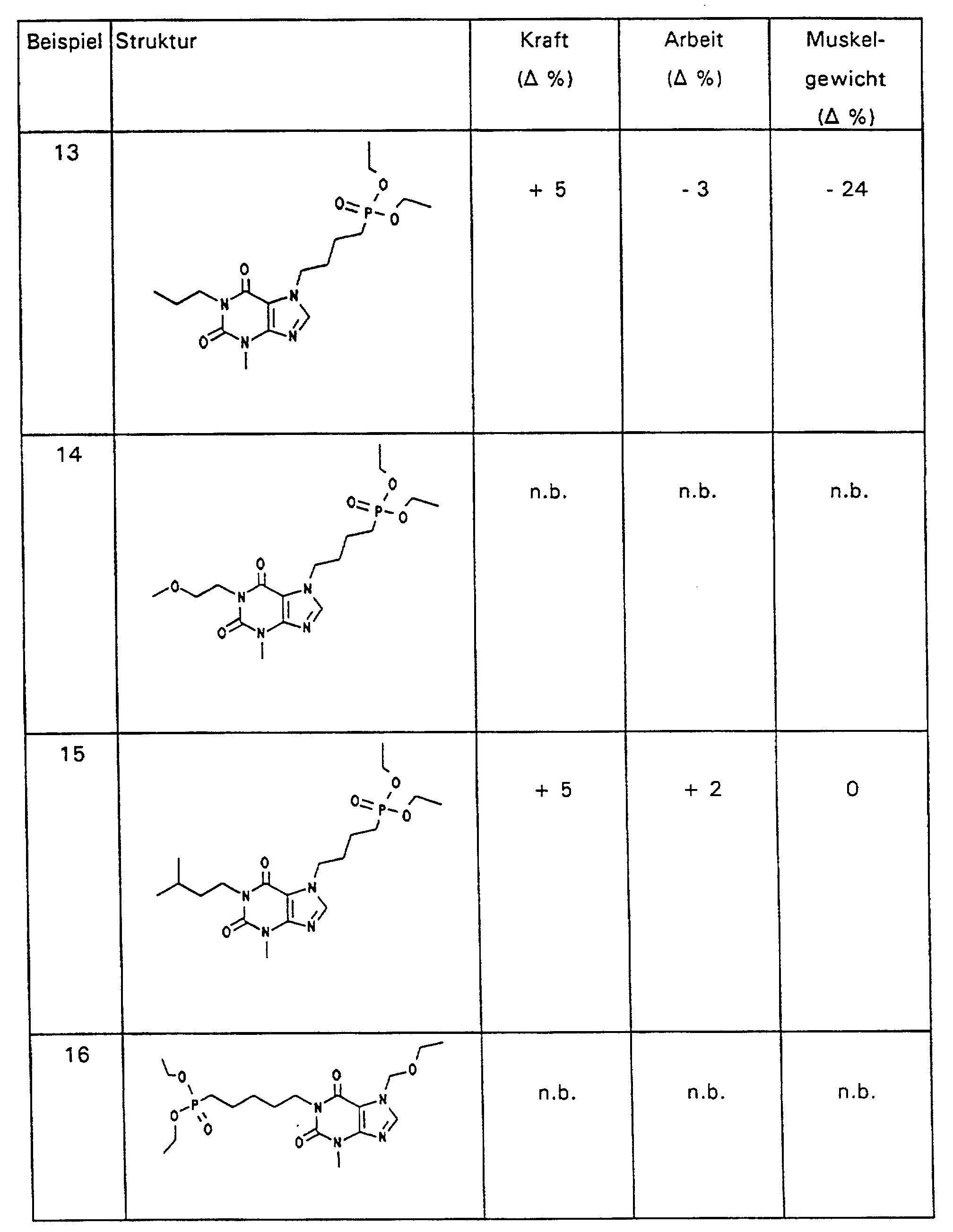

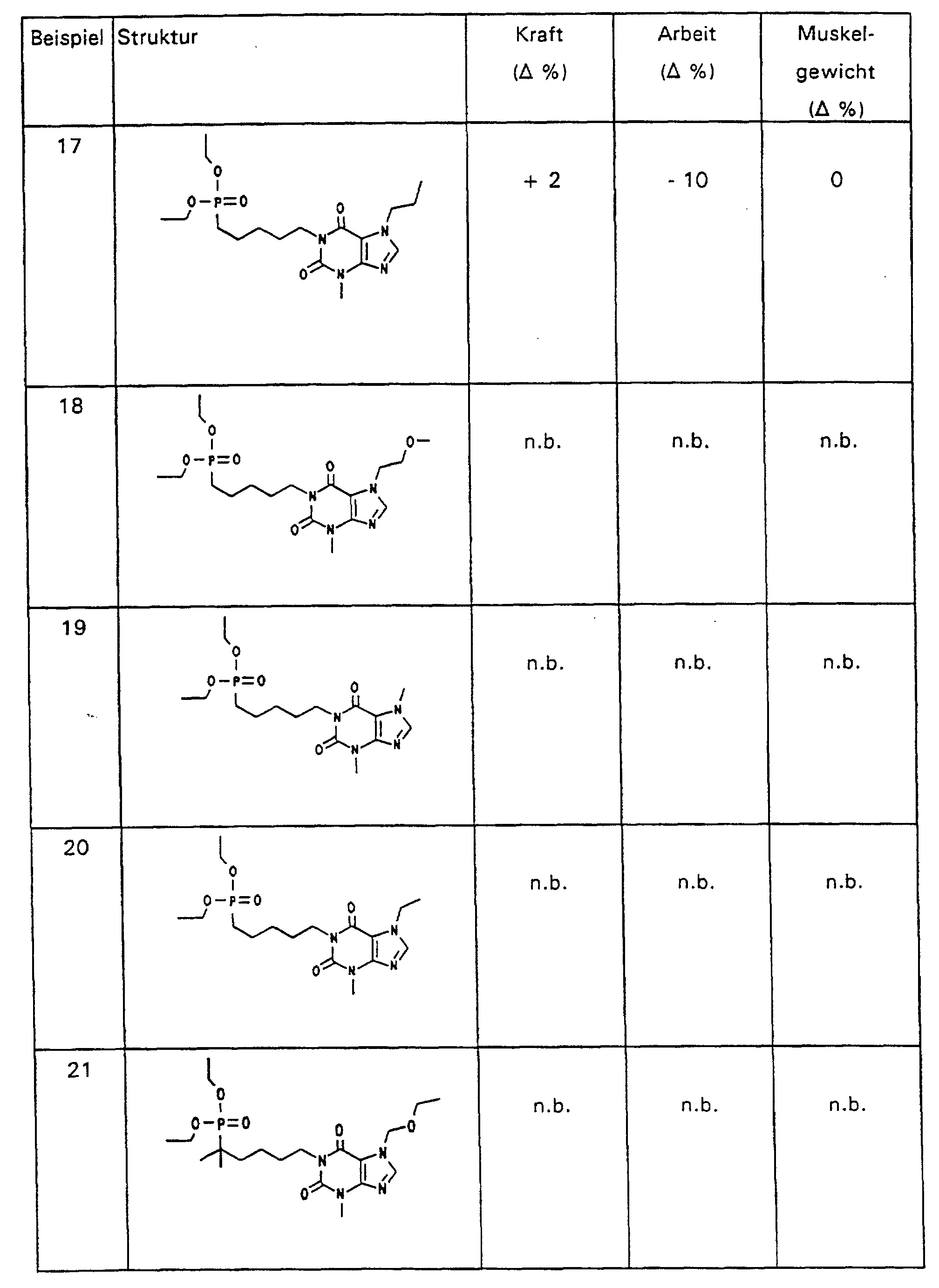

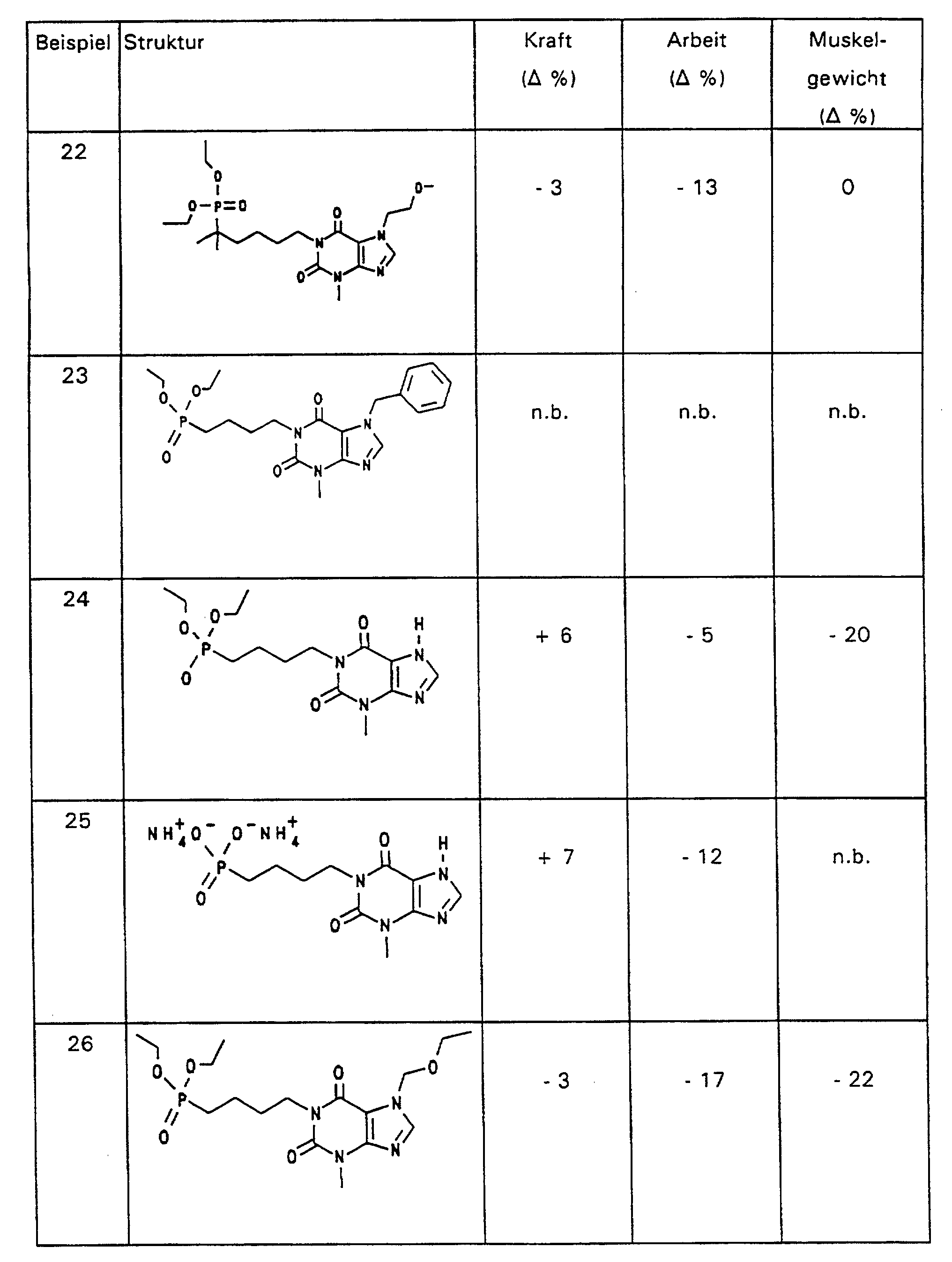

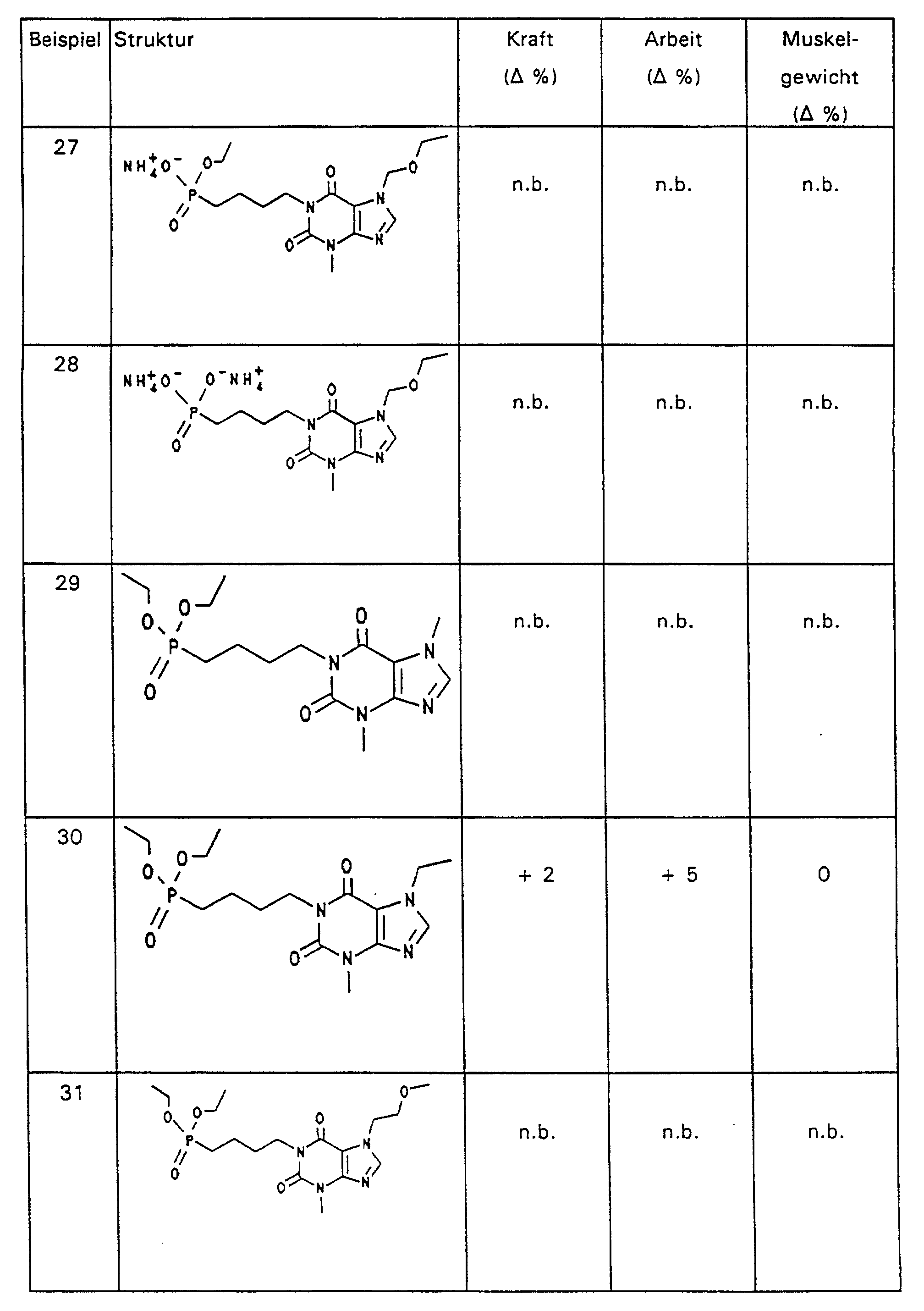

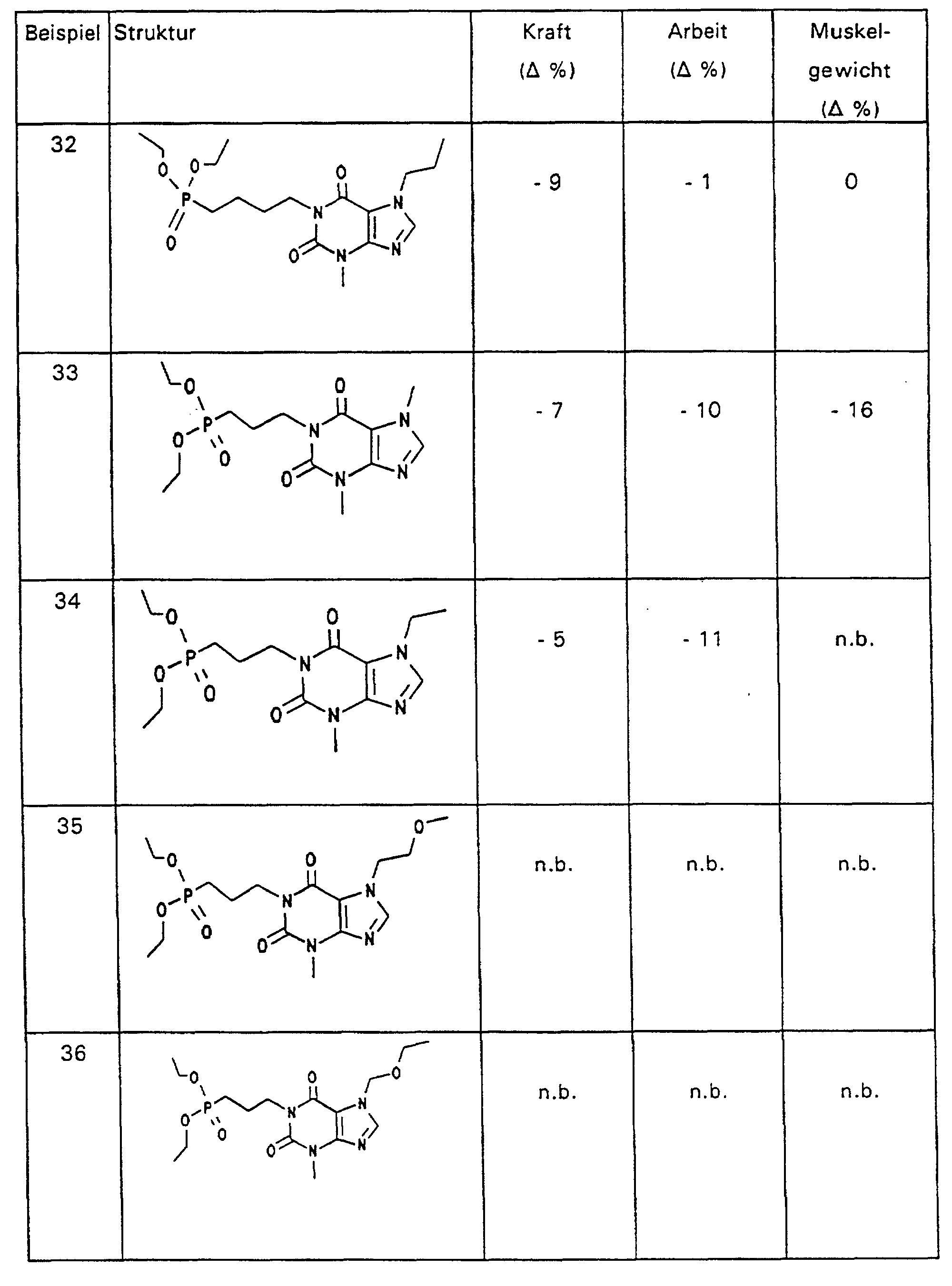

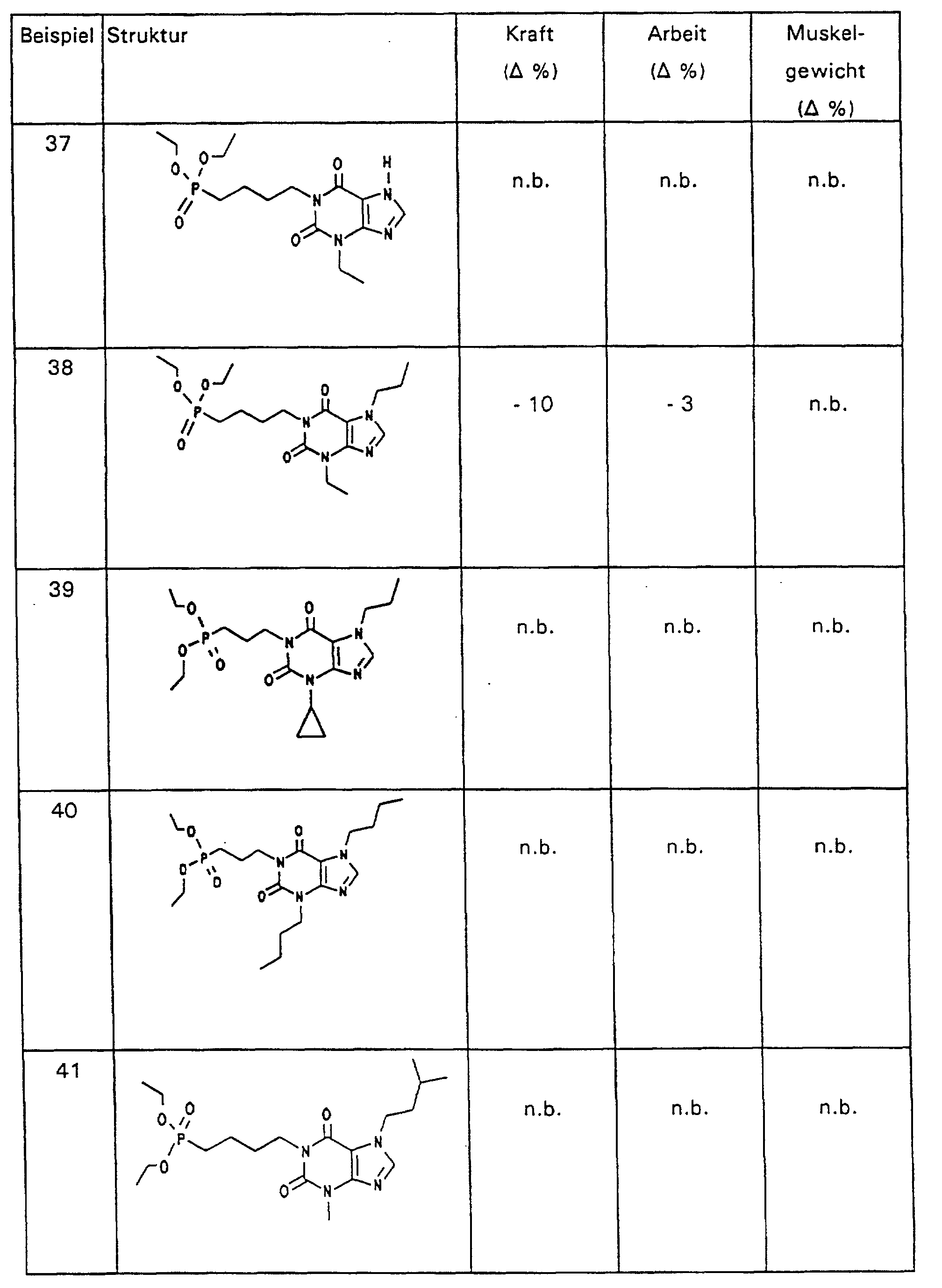

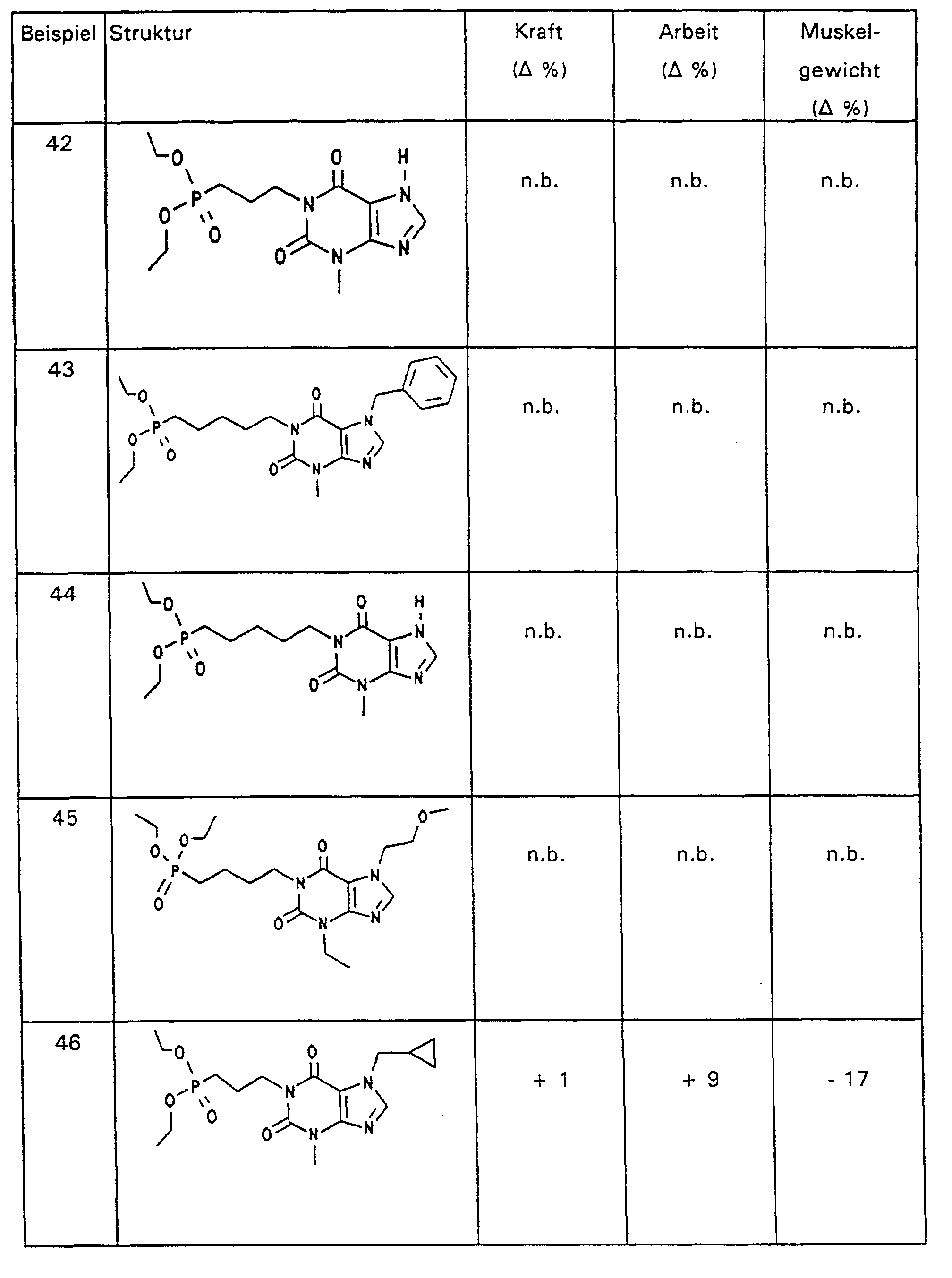

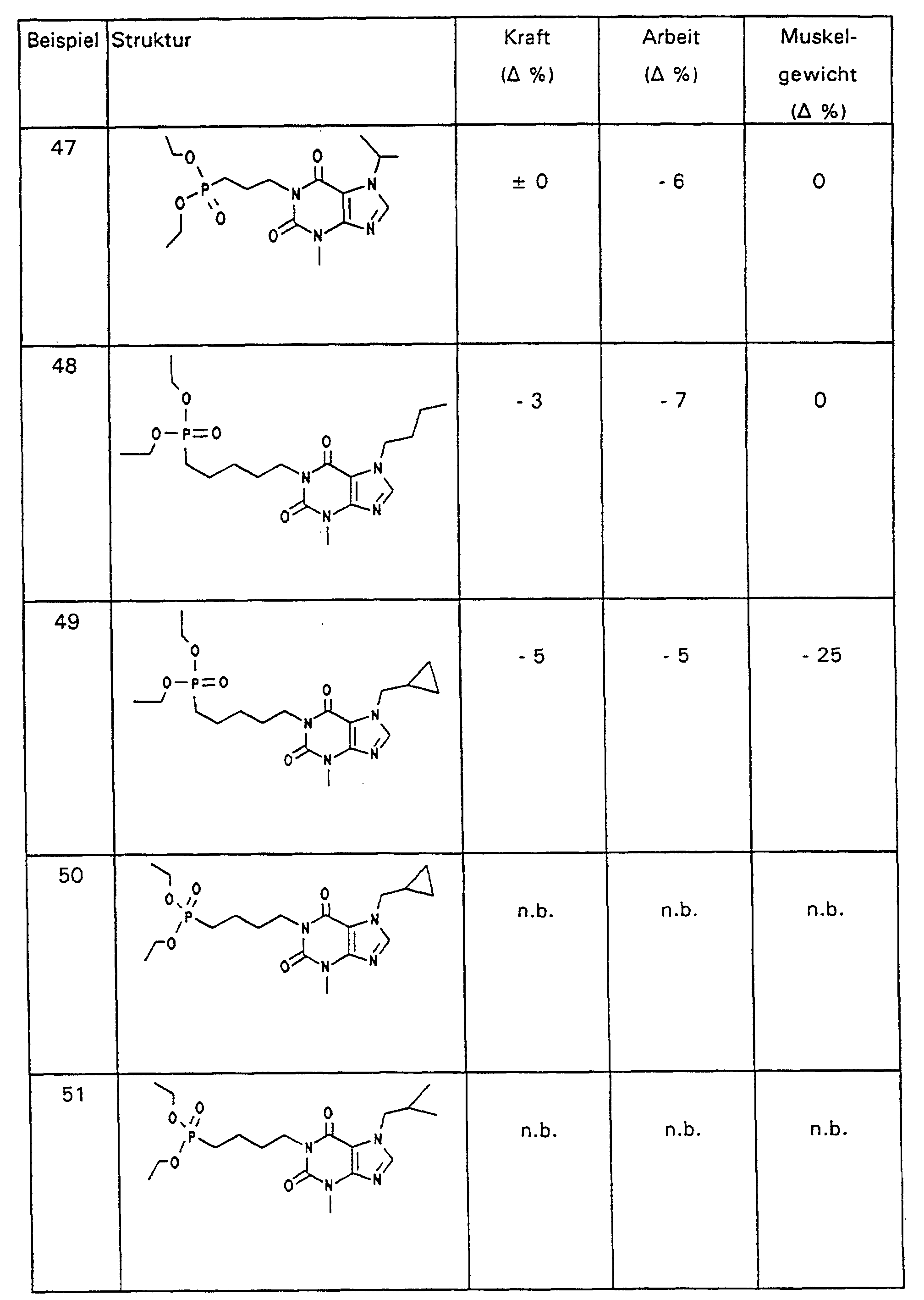

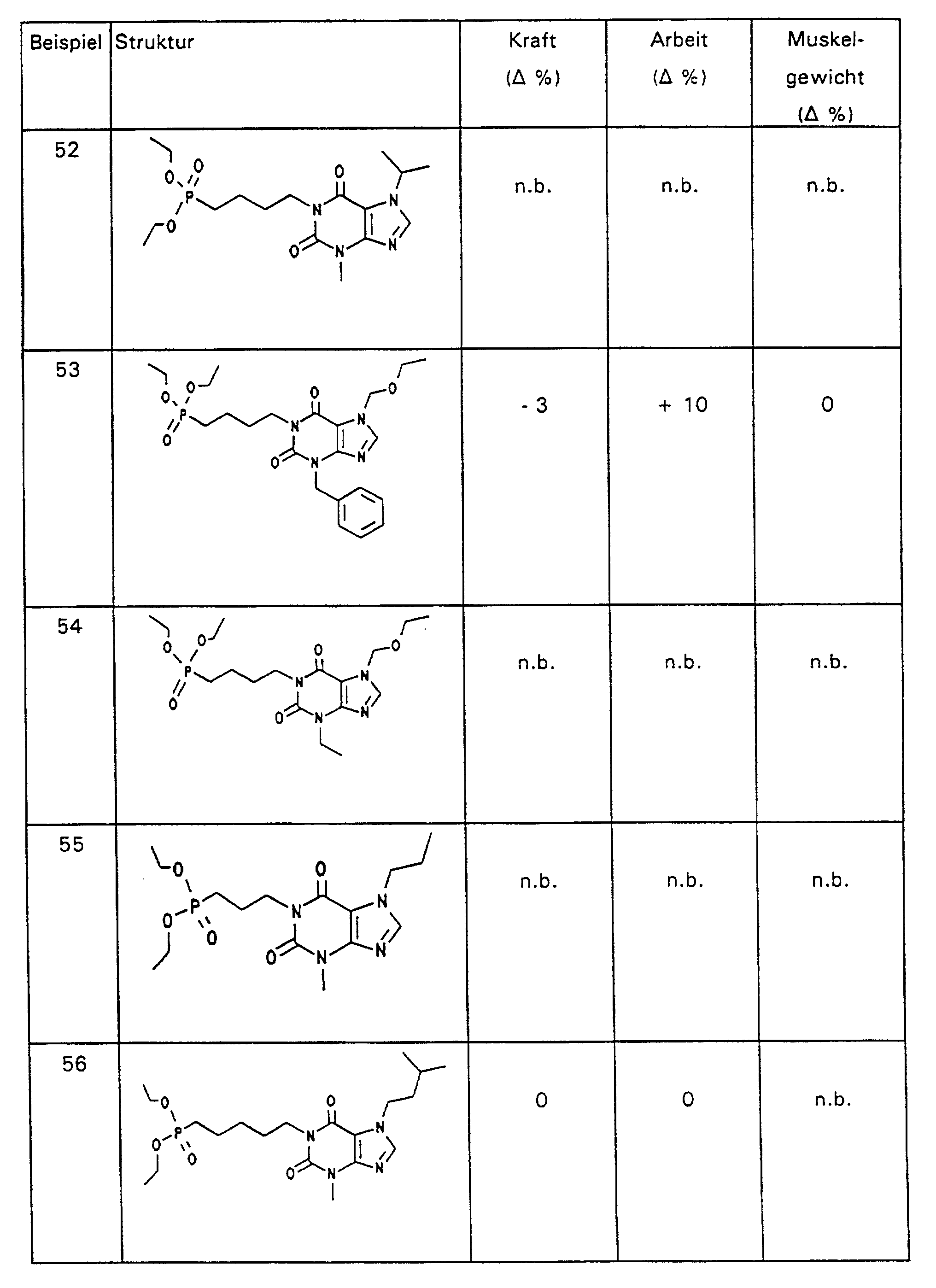

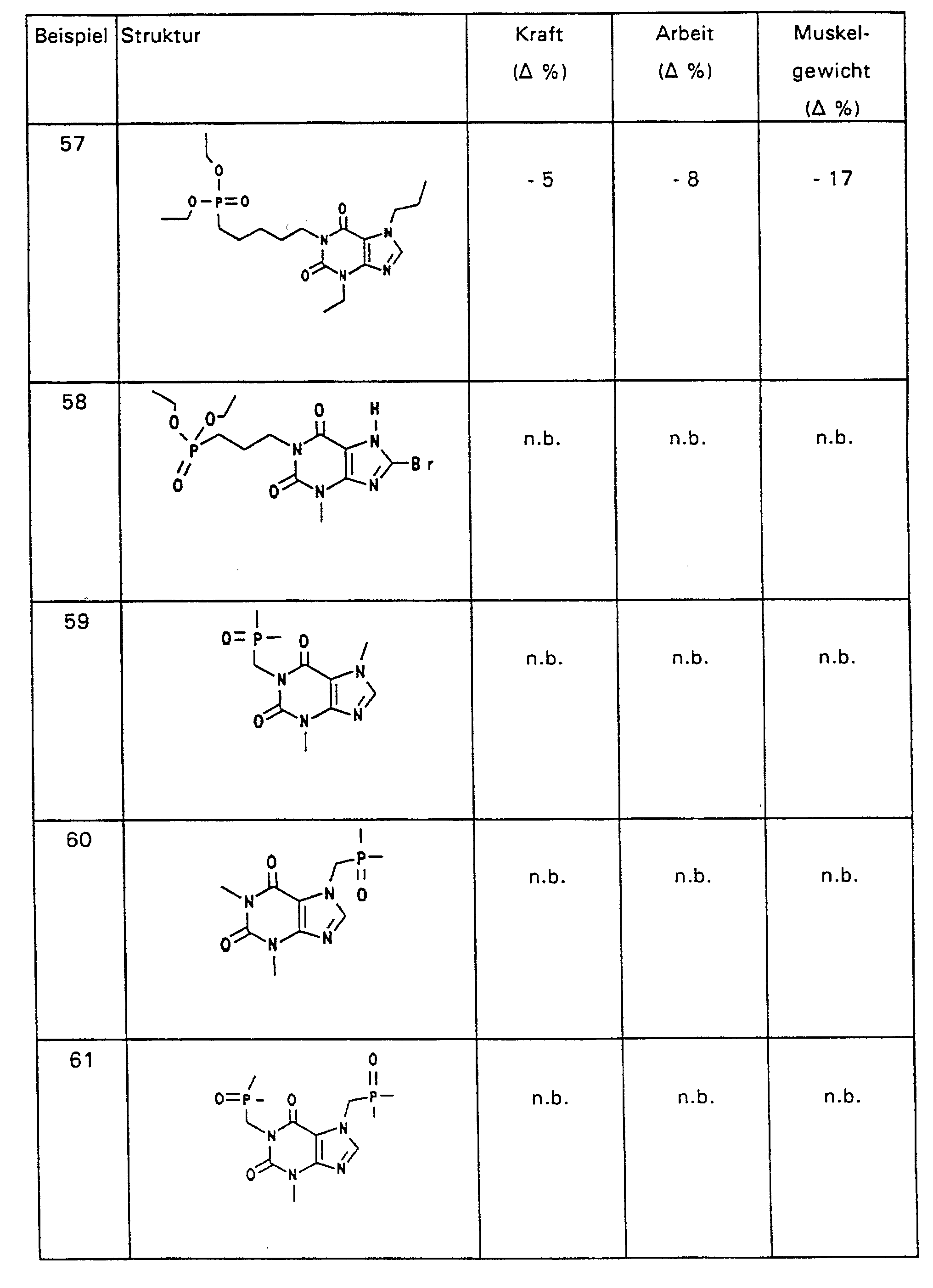

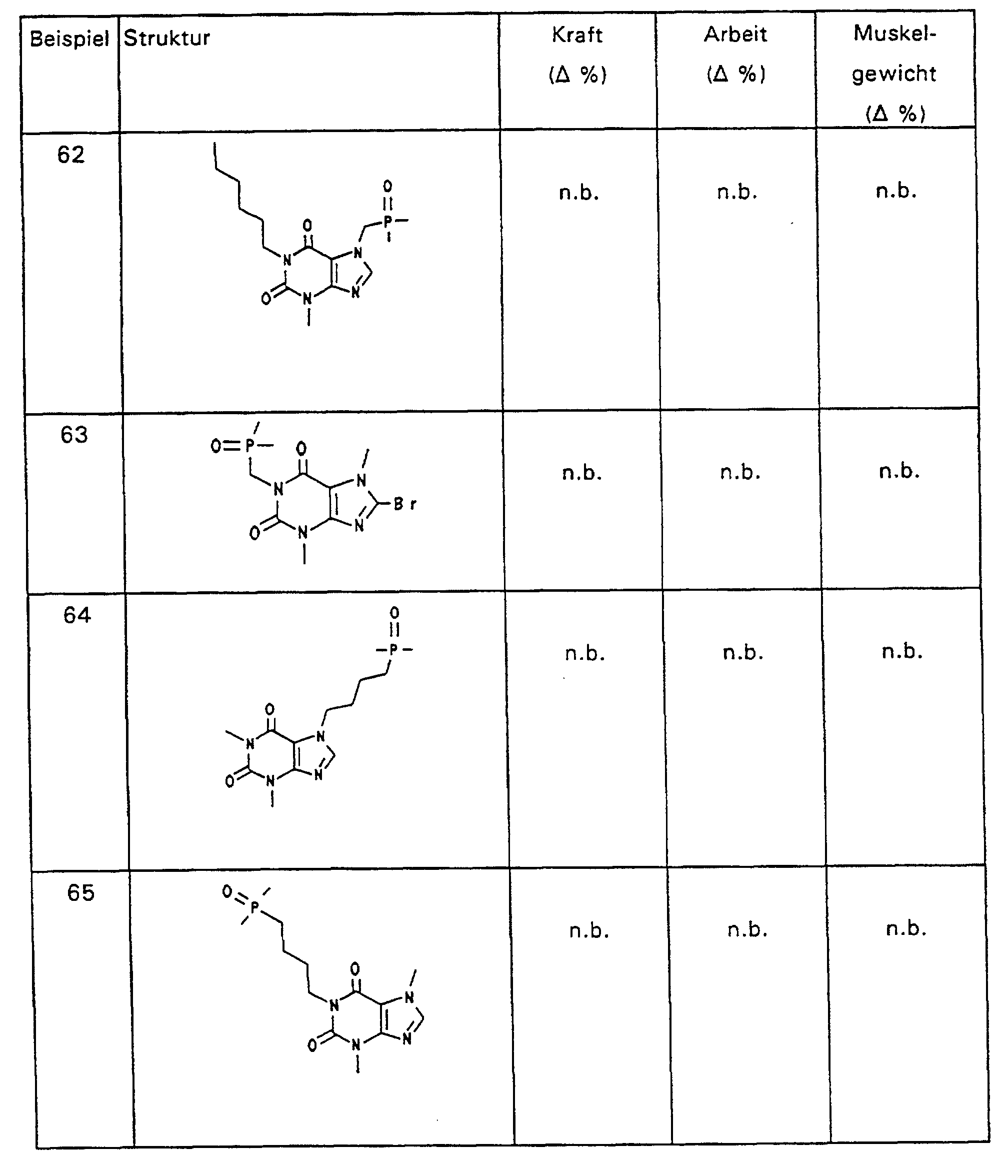

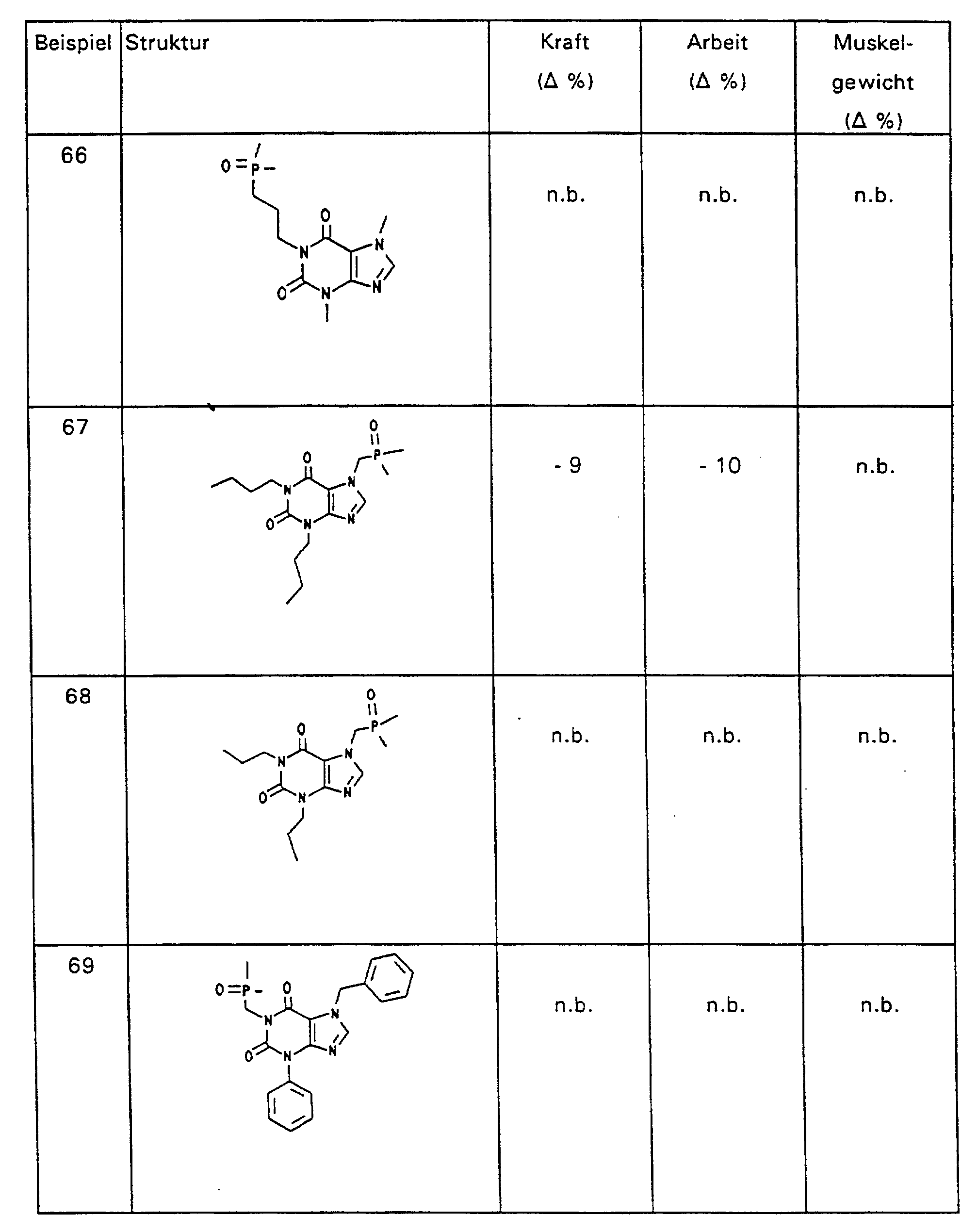

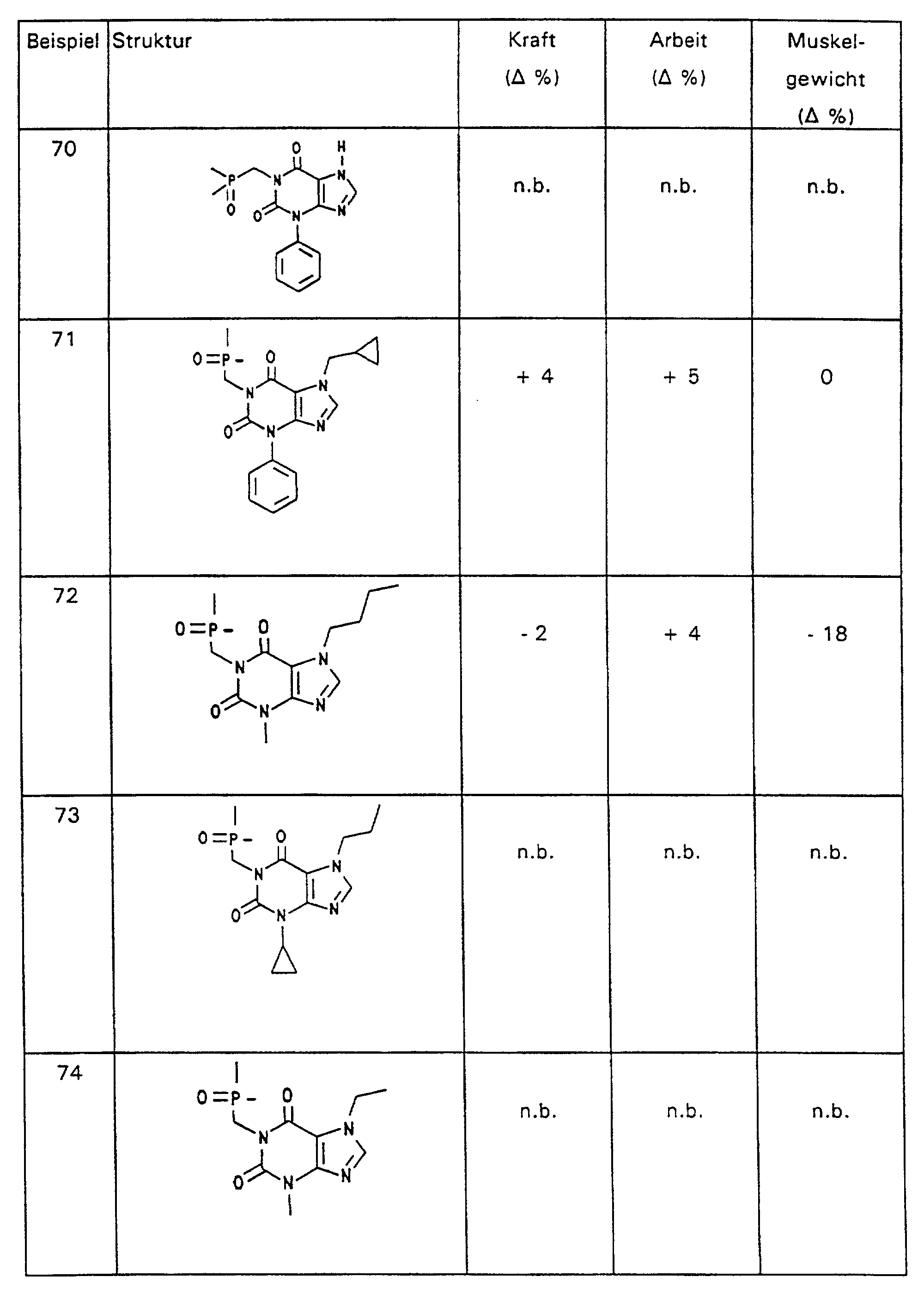

### 2. Prüfung auf Beeinflussung der Endotoxinletalität

Einzelinjektionen von Lipopolysaccharid (LPS), aus gram-negativen Bakterien, führen über eine Reihe von verschiedenen Symptomen zum septischen Schock, der meistens letal ausgeht. Endotoxin (LPS) aus Salmonella abortus equi in einer Dosis von 60 mg/kg i.p. führt zum Schock und ad exitum innerhalb von maximal 72 h p.a. (die Dosis von 60 mg/kg entspricht in etwa: 1,20 mg LPS/Tier).

Als Versuchstiere werden weibliche NMRI-Mäuse, aus der Tierzucht der Hoechst AG/Hattersheim mit einem Körpergewicht von 22 bis 24 g verwendet.

Die Testsubstanz wird intraperitoneal (i.p.), in einem Applikationsvolumen von 10 ml/kg gleichzeitig mit dem LPS verabreicht. Die Anzahl der überlebenden Tiere nach 72 Stunden LPS-Gabe wird im Vergleich zu einer Placebo-Kontrolle angegeben. Die Anzahl der getesteten Tiere pro Gruppe ist 5; Dosis 25 mg/kg.

Die Ergebnisse sind in Tabelle II aufgeführt.

**Tabelle II**

| Beispiel | Hemmung der Mortalität in % |
|---|---|
| 2 | 100 |
| 24 | 100 |
| 34 | 60 |
| 38 | 60 |
| 39 | 100 |
| 40 | 100 |

### 3. Wirksamkeit als Antagonist der Kontraktion der isolierten Trachea des Meerschweinchens

Die Verbindungen der Formel I werden als Antagonisten der Kontraktion isolierter Trachea des Meerschweinchens verwendet. Als Agonisten wurden PGF₂ₐ (Upjohn, Deutschland), Calciumionophor A23187 (Calbiochem, Bad Soden, Deutschland) und Substanz P (American Peptide Comp., USA) eingesetzt:
a) PGF2a
   PGF2a entfaltet seine kontraktile Wirkung über eigene Rezeptoren. Hemmung von Kontraktionen, welche durch dieses Cyclooxigenaseprodukt induziert werden, sind nur durch kompetitive Antagonisierung am Rezeptor oder des nachgeschalteten Signalweges möglich.
b) Calciumionophor A23187 (Calbiochem, Bad Soden) bewirkt durch erhöhte Aufnahme von Calcium-Ionen, in der Zelle eine Aktivierung aller calciumabhängigen Signalkaskaden. Insbesondere Lipoxigenasehemmer und generell antientzündlich wirkende Substanzen zeigen in diesem Modell Effekte.
c) Substanz P
   Substanz P als Transmitterstoff zwischen Immunsystem und Nervensystem zeigt eine kontraktile Wirkung gegenüber glatter Muskulatur. Die Antagonisierung ist einerseits durch spezifische Rezeptorantagonisten, andererseits auch durch Inhibition des nachgeschalteten Signalwegs möglich. Insbesondere Präparate, welche die intrazelluläre Konzentration von cyclischen Nukleotiden direkt oder indirekt erhöhen, zeigen hier spasmolytische Eigenschaften.

### Präparation der Organteile und Versuchsdurchführung:

Das Meerschweinchen wird durch finale Lachgasnarkose getötet. Die Trachea wird in ganzer Länge herauspräpariert und in 15 einzelne Knorpelringe geschnitten. Jeweils fünf Ringe werden zu einer Kette zusammengebunden und im Organbad unter 3 g Vorlast fixiert. Nach 45 Minuten Äquilibrierungszeit wird mit dem Agonisten eine Kontraktion erzeugt. Auf dem Plateau vom Maximum wird der Antagonist (Testpräparat) kumulativ addiert. Die Auswertung erfolgt in % Kraftänderung bezogen auf das Kontraktionsmaximum. Die Versuchsdurchführung erfolgt bei 37°C, als physiologische Nährlösung wird eine mit 95 Vol.-% O₂, 5 Vol.-% CO₂ durchperlte, modifizierte Krebs-Henseleit-Lösung benutzt.
Versuchstiere: Meerschweinchen Albino (Tierzucht Anstalt Hattersheim, Kastengrund, Deutschland), Gewicht: 200 bis 300 g, männliches oder weibliches Geschlecht

### Zusammensetzung des Organbads (Nährlösung):

| Modifizierte Krebs-Henseleit-Lösung | | | |
|---|---|---|---|
| für den Calciumionophor A23187 | | für den Agonisten SP für die Agonisten PgF₂ₐ | |
| NaCl | 6,9 g | NaCl | 7,9 g |
| KH₂PO₄ | 0,14 g | KH₂PO₄ | 0,18 g |
| NaHCO₃ | 2,1 g | NaHCO₃ | 1,37 g |
| Glucose | 2,0 g | Glucose | 1,53 g |
| KCl | 0,35 g | KCl | 0,25 g |
| CaCl₂ | 0,28 g | CaCl₂ | 0,27 g |
| MgSO₄ | 0,14 g | auf 1 l Aqua bidest. | |
| auf 1 l Bidestilliertes Wasser (Aqua bidest.) | | | |

Vehikel: Aqua bidest., Ethanol oder Isopropanol; Applikation: ins Organbad Anzahl der Applikation: Kumulativ, bei SP Einzeldosen

Ergebnisse: Es zeigt sich eine antagonistische Wirkung der erfindungsgemäßen Verbindungen in Bezug auf KCl, Prostaglandin PgF_{2α}, Calciumionophor (A 23187) und Substanz P. Die Ergebnisse sind in Tabelle 3 aufgeführt.

**Tabelle 3**

| Antikontraktile Wirkung (Trachea) | | | |
|---|---|---|---|
| Beispiel | PGF_{2α} | Ca-Ionophor | SP-Antagonismus |
| 2 | >10 | >10 | 10-30 |
| 4 | >10 | >10 | 10-30 |
| 9 | | | 10-30 |
| 15 | | | 30-60 |
| 17 | | | 30-60 |
| 20 | | | 10-30 |
| 23 | >10 | >10 | 10-30 |
| 24 | >10 | | 30-60 |
| 30 | >10 | 10 | 10-30 |
| 32 | | | 10-30 |
| 33 | | | 10-30 |
| 35 | | >10 | 30-60 |
| 38 | >10 | 10 | 10-30 |
| 47 | | | 10-30 |

Alle Zahlenwerte sind in der Einheit µg/ml angegeben und beziehen sich auf den Konzentrationsbereich an der getesteten, erfindungsgemäßen Verbindung der Formel I (siehe Beispielnummern in Tabelle 1), der nötig ist, um eine Dilatation zu bewirken, die dem ED₅₀-Bereich entspricht.

### 4. Wirksamkeit als Antagonist der Kontraktion isolierter Lungenstreifen des Meerschweinchens

Der durchgeführte Test basiert auf dem gleichen Prinzip wie der in der pharmakologischen Prüfung 3 angegebene Test; hier werden jedoch Lungenstreifen verwendet.

### Präparation der Organteile und Versuchsdurchführung:

Das Meerschweinchen wird in einer Lachgasnarkose getötet. Der gesamte Lungentrakt wird von der Trachea ausgehend herausgeschnitten. Die Lungenlappen werden zirkulär geschnitten, so daß 3 mm breite Streifen entstehen. Die Streifen der Oberlappen werden geteilt um insgesamt sechs etwa gleich große Streifen zu erhalten. Die Streifen werden unter eine Vorlast von 4 g in die Organbäder eingehängt. Als Nährlösung werden modifizierte Krebs-Henseleit-Lösungen verwandt. Bei Verwendung von "Platelet activating factor" (PAF) als Agonist entspricht die Zusammensetzung dieser Lösung der für den Agonisten SP in "Test 3"; für den Agonisten LTD₄ wird eine Krebs-Henseleit-Lösung wie im Falle des Agonisten KCl (Test 3) verwendet. Zur Durchperlung des Bades wird 95 Vol.-% O₂, 5 Vol.-% CO₂ benutzt, die Badtemperatur beträgt 37°C. Die Versuchsdurchführung erfolgt in einer therapeutisch-kumulativen Methodik, mit den Dosierungsabstufungen 1, 3, 6 und 10 µg/ml.

Versuchstiere, Organbad, Vehikel, Art der Applikation entsprechen dem in der pharmakologischen Prüfung 3 gesagten.

Ergebnis: Es zeigt sich eine antagonistische Wirkung der Verbindung der Formel I in Bezug auf "Leukotrien D₄" (LTD₄) und das Membranlipid "Platelet activating factor". Die Ergebnisse werden in Tabelle 4 gezeigt.

**Tabelle 4**

| Antikontraktile Wirkung (Lunge) | | |
|---|---|---|
| Beispiel | PAF | LTD₄ |
| 1 | 3-9 | |
| 2 | 3-6 | 3-6 |
| 4 | 1-3 | |
| 7 | 3-6 | |
| 8 | 5-10 | |
| 9 | | 3-6 |
| 13 | 6-10 | |
| 17 | 1-3 | 1-3 |
| 23 | 1-3 | 1-3 |
| 25 | 5-15 | |
| 28 | 3-9 | |
| 30 | 3-6 | 6-10 |
| 31 | 6-10 | |
| 32 | 6-10 | 6-10 |
| 33 | 3-6 | |
| 35 | 3-6 | |
| 36 | 5-15 | |
| 37 | 1-3 | |
| 38 | 1-3 | |
| 39 | | 3-6 |
| 43 | 1-3 | 1-6 |
| 45 | 1-3 | |
| 49 | | 3-6 |
| 53 | 1-3 | |
| 54 | 1-3 | |
| 55 | 1-3 | |

Alle Zahlenwerte sind in der Einheit µg/ml angegeben und beziehen sich auf den Konzentrationsbereich an getesteter Verbindung der Formel I (siehe Beispielnummern in Tabelle 1), der nötig ist, um eine Kontraktion zu bewirken, die dem IC₅₀-Wert entspricht.

### 5. Hemmung der Phosphodiesterase III-Aktivität

Der Test wird mit der Phosphodiesterase der Fa. Boehringer Mannheim (Mannheim, Deutschland), Bestellnr. 108 243 nach der Methode von T. SAEKI, I. SAITO, Biochem. Pharm. 46, Nr. 5, (1986), Seiten 833-839 durchgeführt.

Die Ergebnisse sind in Tabelle 5 aufgeführt.

| Beispiel | PDE III Inhibition [%] |
|---|---|
| 2 | 65 |
| 17 | 77 |
| 20 | 65 |
| 23 | 65 |
| 32 | 65 |
| 34 | 82 |
| 39 | 65 |
| 41 | 56 |
| 43 | 52 |
| 46 | 67 |
| 47 | 64 |
| 48 | 52 |
| 49 | 57 |
| 50 | 51 |
| 55 | 59 |
| 57 | 70 |

Alle Zahlenwerte sind in % Hemmung der Enzymaktivität bei 100 µM der entsprechenden Verbindung der Formel I angegeben.

## Patentansprüche

1. Verbindung der Formel I 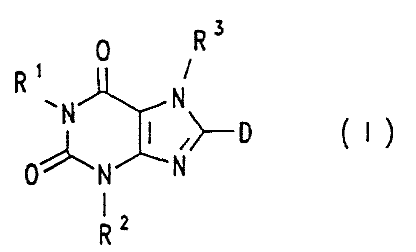 und/oder ein physiologisch verträgliches Salz der Verbindung der Formel I und/oder eine gegebenenfalls stereoisomere Form der Verbindung der Formel I, wobei
R¹ und R³ gleich oder verschieden sind und mindestens einer der Reste
R¹ und R³ für
a) einen Rest der Formel XI steht, 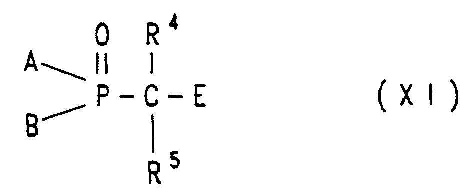 worin R⁴ und R⁵ gleich oder verschieden sind und unabhängig voneinander
1.1 Wasserstoffatom,
1.2 Hydroxyl oder
1.3 (C₁-C₆)-Alkyl darstellen,
A und B gleich oder verschieden sind und unabhängig voneinander
2.1 (C₁-C₄)-Alkyl,
2.2 (C₁-C₆)-Alkoxy,
2.3 Hydroxyl oder
2.4 Benzyloxy darstellen,
E eine kovalente Bindung oder ein geradkettiges oder verzweigtes Alkyl mit 1 bis 5 C-Atomen darstellt, und
b) falls nur einer der Reste R¹ oder R³ die unter a) genannte Bedeutung hat, der andere Rest R¹ oder R³ für
1) Wasserstoffatom,
2) (C₁-C₆)-Alkyl, geradkettig oder verzweigt,
3) (C₂-C₆)-Alkyl, geradkettig oder verzweigt, wobei die Kohlenstoffkette durch 1 oder 2 Sauerstoffatome unterbrochen ist und 2 aneinander gebundene Sauerstoffatome ausgeschlossen sind,
4) (C₁-C₈)-Alkyl, geradkettig oder verzweigt, substituiert durch
4.1 eine Oxogruppe oder
4.2 ein oder zwei Hydroxylreste,
5) (C₂-C₆)-Alkenyl, geradkettig oder verzweigt,
6) Benzyl,
7) Benzyl, ein- bis fünffach substituiert durch
7.1 (C₁-C₄)-Alkyl oder
7.2 (C₁-C₄)-Alkoxy,
8) (C₃-C₆)-Cycloalkyl oder
9) (C₁-C₄)-Alkyl-(C₃-C₆)-cycloalkyl, steht, und
R² für
1) Wasserstoffatom,
2) (C₁-C₆)-Alkyl, geradkettig oder verzweigt,
3) (C₂-C₆)-Alkyl, geradkettig oder verzweigt, wobei die Kohlenstoffkette durch 1 oder 2 Sauerstoffatome unterbrochen ist und 2 aneinander gebundene Sauerstoffatome ausgeschlossen sind,
4) Benzyl,
5) Benzyl, ein- bis fünffach substituiert durch
5.1 (C₁-C₄)-Alkyl oder
5.2 (C₁-C₄)-Alkoxy,
6) Phenyl,
7) (C₃-C₆)-Cycloalkyl oder
8) (C₁-C₄)-Alkyl-(C₃-C₆)-Cycloalkyl, steht, und
D für
1) Wasserstoffatom oder
2) Fluor-, Chlor-, Brom- oder Jodatom, steht,
wobei die Verbindungen [4-(1,3-Dimethylxanthin-7-yl)-butyl]-phosphonsäurediethylester, 5-(1,3-Dimethoxyxanthin-7-yl)-pentylphosphonsäure, 4-(1,3-Dimethylxanthin-7-yl)-butyl-phosphonsäure und 3-(1,3-Dimethylxanthin-7-yl)-propyl-phosphonsäure ausgeschlossen sind.

2. Verbindung der Formel I gemäß Anspruch 1, **dadurch gekennzeichnet, daß** A und B für
1) Hydroxyl,
2) C₂-Alkoxy oder
3) Methyl stehen.

3. Verbindung der Formel I gemäß Anspruch 1, **dadurch gekennzeichnet, daß** R² für
1) Wasserstoffatom,
2) (C₂-C₆)-Alkyl, geradkettig oder verzweigt, wobei die Kohlenstoffkette durch 1 Sauerstoffatom unterbrochen ist,
3) Benzyl,
4) Cyclopropyl oder
5) -CH₂-Cyclopropyl, steht.

4. Verbindung der Formel I gemäß einem oder mehreren der Ansprüche 1 bis 3, **dadurch gekennzeichnet, daß** einer der Reste R¹ oder R³ für einen Rest der Formel XI steht und der andere Rest R¹ oder R³ für
1) Wasserstoffatom,
2) (C₂-C₆)-Alkyl, geradkettig oder verzweigt, wobei die Kohlenstoffkette durch 1 oder 2 Sauerstoffatome unterbrochen ist und 2 aneinander gebundene Sauerstoffatome ausgeschlossen sind,
3) Benzyl oder
4) -CH₂-Cyclopropyl, steht.

5. Verfahren zur Herstellung der Verbindung der Formel I gemäß Anspruch 1, **dadurch gekennzeichnet, daß** man
A) eine Verbindung der Formel III, 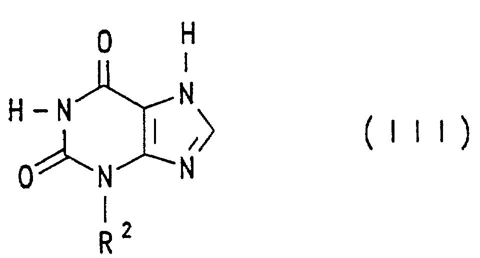 wobei R² die in Formel I gemäß Anspruch 1 genannte Bedeutung hat,
in Gegenwart basischer Mittel mit einem Alkylierungsmittel der Formel II, 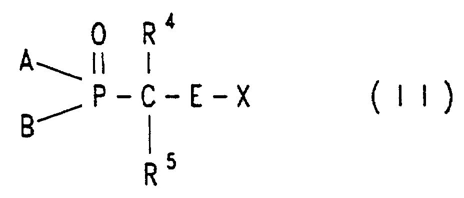 wobei X Chlor, Brom, Jod oder Sulfonsäureesterrest bedeutet und R⁴, R⁵, A, B und E die in Formel XI genannte Bedeutung haben, zu einer Verbindung der Formel I gemäß Anspruch 1 umsetzt, wobei R¹ Wasserstoffatom bedeutet und R³ ein Rest der Formel XI 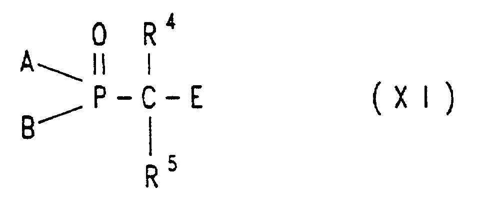 ist, und R² die in Formel I genannte Bedeutung hat, oder
B) eine gemäß A) hergestellte Verbindung der Formel I in Gegenwart basischer Mittel mit einem Alkylierungsmittel der Formel II, wobei X, R⁴, R⁵, A, B und E wie in Verfahren A) definiert sind, zu einer Verbindung der Formel I gemäß Anspruch 1 umsetzt, wobei R¹ und R³ gleich oder verschieden sind und für einen Rest der Formel XI stehen, oder
C) eine gemäß A) hergestellte Verbindung der Formel I in Gegenwart basischer Mittel mit einer Verbindung R⁶-X, wobei
R⁶ für
1) Wasserstoffatom,
2) (C₁-C₆)-Alkyl, geradkettig oder verzweigt,
3) (C₂-C₆)-Alkyl, geradkettig oder verzweigt, wobei die Kohlenstoffkette durch 1 oder 2 Sauerstoffatome unterbrochen ist und 2 aneinander gebundene Sauerstoffatome ausgeschlossen sind,
4) (C₁-C₆)-Alkyl, geradkettig oder verzweigt, substituiert durch
4.1 eine Oxogruppe oder
4.2 eine oder zwei Hydroxylgruppen,
5) (C₂-C₆)-Alkenyl, geradkettig oder verzweigt,
6) Benzyl,
7) Benzyl, ein- bis fünffach substituiert durch
7.1 (C₁-C₄)-Alkyl oder
7.2 (C₁-C₄)-Alkoxy,
8) (C₃-C₆)-Cycloalkyl oder
9) (C₁-C₄)-Alkyl-(C₃-C₆)-cycloalkyl, steht, und
X die unter A) genannte Bedeutung hat,
zu einer Verbindung der Formel I gemäß Anspruch 1
umsetzt, wobei
R¹ die Bedeutung von R⁶ hat, R² gemäß der Verbindung der Formel I definiert ist und R³ die Bedeutung der Formel II hat, oder
D) eine Verbindung der Formel IV, 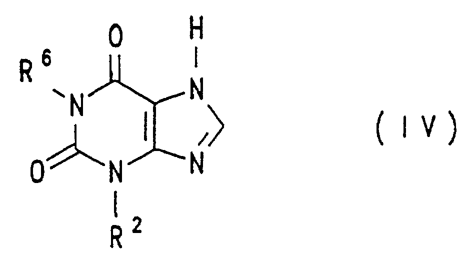 wobei R⁶ die unter C) genannte Bedeutung hat und R² die in Formel I genannte Bedeutung hat, in Gegenwart basischer Mittel mit einem Alkylierungsmittel der Formel II zu einer Verbindung der Formel I umsetzt, wobei
R¹ die Bedeutung von R⁶ hat, R² die in Formel I genannte Bedeutung hat und R³ die in Formel XI genannte Bedeutung hat, oder
E) eine Verbindung der Formel V 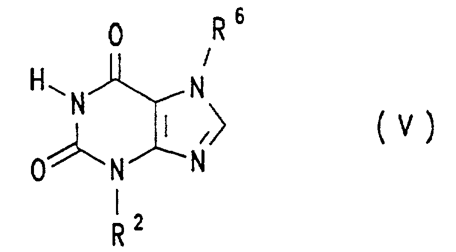 wobei R⁶ die unter C) genannte Bedeutung und R² die in Formel I genannte Bedeutung haben, in Gegenwart basischer Mittel mit einem Alkylierungsmittel der Formel II zu einer Verbindung der Formel I umsetzt, wobei R¹ die in Formel XI genannte Bedeutung hat, R² die in Formel I genannte Bedeutung hat und R³ die Bedeutung von R⁶ hat, oder
F) eine Verbindung der Formel I, wobei mindestens ein R¹, R² und R³ ein Benzyl-, Alkoxymethyl- oder Alkoxyalkoxymethylrest ist und mindestens ein R¹ und R³ ein Rest der Formel XI ist, unter reduzierenden oder hydrolytischen Bedingungen den Benzyl-, Alkoxymethyl- oder Alkoxyalkoxymethylrest der Formel I abspaltet, oder
G) eine Verbindung der Formel VI, 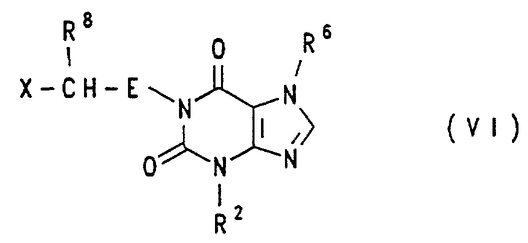 wobei
R⁶ die unter C) genannte Bedeutung hat,
R² die in Formel I genannte Bedeutung hat,
E die in Formel Xl genannte Bedeutung hat,
R⁸ Wasserstoffatom oder (C₁-C₄)-Alkyl ist und
X Chlor, Brom, Jod oder ein Sulfonsäureesterrest ist,
in Gegenwart einer starken Base wie Natriumhydrid, Butyllithium oder Lithiumdiisopropylamid in einem inerten Lösungsmittel mit einer Verbindung der Formel VIII, 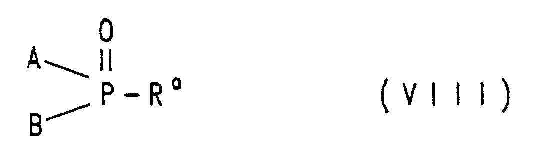 wobei A und B die in Formel I genannte Bedeutung haben und
R^{a} Wasserstoffatom oder (C₁-C₄)-Alkyl ist, zu einer Verbindung der Formel I umsetzt, wobei R¹ die in Formel XI genannte Bedeutung hat, R² die in Formel I genannte Bedeutung hat und R³ die Bedeutung von R⁶ hat, oder
H) eine Verbindung der Formel VI, wobei R², R⁶, R⁸, E und X die unter G) genannte Bedeutung haben mit einer Verbindung der Formel IX,
P(OR¹⁰)₃ (IX)
wobei P Phosphor, O Sauerstoff und R¹⁰ ein (C₁-C₄)-Alkyl sind, in eine Verbindung der Formel I umsetzt, wobei R¹ die in Formel Xl genannte Bedeutung hat, R² die in Formel I genannte Bedeutung hat und R³ die Bedeutung von R⁶ hat, oder
I) eine Verbindung der Formel I, wobei R¹, R², R³, E und D die in Formel I genannte Bedeutung haben und A und B für (C₁-C₄)-Alkyl oder (C₁-C₄)-Alkoxy stehen, mit Mitteln wie Mineralsäuren oder Silylhalogenide in die entsprechenden Phosphonsäurederivate der Formel I überführt, oder
K) eine Verbindung der Formel I, wobei R¹, R², R³, E und D die in Formel I genannte Bedeutung haben und A oder B für eine Benzyloxygruppe steht, selektiv in den entsprechenden Phosphonsäurehalbester überführt, oder
L) eine Verbindung der Formel I, wobei R¹, R², R³, E, A und B die in Formel I genannte Bedeutung haben und D für Wasserstoffatom steht halogeniert, wobei eine entsprechende Verbindung der Formel I entsteht in der D für Fluor, Chlor, Brom oder Jod steht, oder
M) die nach Verfahren A) bis L) hergestellte Verbindung der Formel I entweder in freier Form isoliert oder im Falle des Vorliegens von sauren oder basischen Gruppen gegebenenfalls in physiologisch verträgliche Salze umwandelt.

6. Arzneimittel, enthaltend eine Verbindung der Formel I, gemäß einem oder mehreren der Ansprüche 1 bis 4, wobei die Verbindungen [4-(1,3-Dimeth-ylxanthin-7-yl)-butyl]-phosphonsäurediethylester, und 4-(1,3-Dimethylxanthin-7-yl)-butyl-phosphonsäure eingeschlossen sind.

7. Verwendung von mindestens einer Verbindung der Formel I gemäß einem oder mehreren der Ansprüche 1 bis 4, wobei die Verbindungen [4-(1,3-Di-methylxanthin-7-yl)-butyll-phosphonsäurediethylester, und 4-(1,3-Dimethylxanthin-7-yl)-butyl-phosphonsäure eingeschlossen sind, zur Herstellung eines Arzneimittels zur Prophylaxe oder Therapie von Muskelatrophie, Muskeldystrophie, Kachexie, Sepsis, septischer Schock, Endotoxin Schock, Systemic Inflammation Response Syndrome, Adult Respiratory Distress Syndrome, zerebrale Malaria, chronische Lungenentzündung, Lungensarkoidose, Reperfusionsschäden, Narbenbildung, Darmentzündungen, Kolitis Ulcerosa, Kachexie, infolge von Infektionen, AIDS, Krebs, Trauma und anderen Erkrankungen mit erhöhtem Proteinverlust, peripheren Durchblutungsstörungen, Erkrankungen mit gestörter Leukozyten-Adhäsion, sowie Erkrankungen, die mit einer erhöhten oder unregulierten Tumor Nekrose Faktor - Produktion einhergehen wie rheumatoide Arthritis, rheumatoide Spondylitis, Osteoarthritis und andere arthritische Erkrankungen.

8. Verfahren zur Herstellung eines Arzneimittels gemäß Anspruch 6, **dadurch gekennzeichnet, daß** man mindestens eine Verbindung der Formel I gemäß Anspruch 1 und/oder mindestens ein physiologisch verträgliches Salz der Verbindung der Formel I und/oder eine gegebenenfalls stereoisomere Form der Verbindung der Formel I und/oder eine nach dem Verfahren nach Anspruch 5 erhaltene Verbindung, wobei die Verbindungen [4-(1,3-Dimethylxanthin-7-yl)-butyl]-phosphonsäurediethylester, und 4-(1,3-Dimethyl-xanthin-7-yl)-butylphosphonsäure eingeschlossen sind, mit physiologisch annehmbaren Hilfs- und Trägerstoffen und gegebenenfalls weiteren Zusatzstoffen und/oder anderen Wirkstoffen in eine geeignete Darreichungsform bringt.

## Claims

1. A compound of the formula I 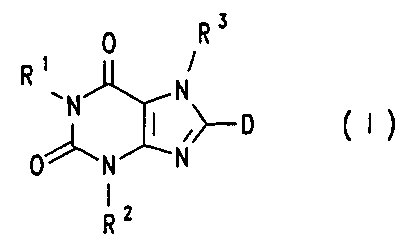 and/or
a physiologically tolerable salt of the compound of the formula I
and/or an optionally stereoisomeric form of the compound of the formula I, where
R¹ and R³ are identical or different and at least one of the radicals R¹ and R³ is
a) a radical of the formula XI 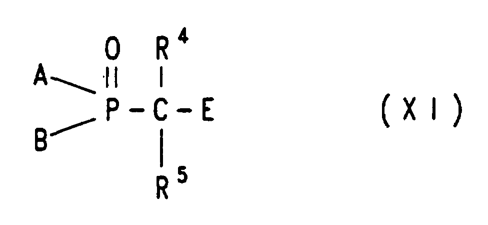 in which R⁴ and R⁵ are identical or different and independently of one another are
1.1 a hydrogen atom,
1.2 hydroxyl or
1.3 (C₁-C₆)-alkyl,
A and B are identical or different and independently of one another are
2.1 (C₁-C₄)-alkyl,
2.2 (C₁-C₆)-alkoxy,
2.3 hydroxyl or
2.4 benzyloxy,
E is a covalent bond or a straight-chain or branched alkyl having 1 to 5 carbon atoms, and
b) if only one of the radicals R¹ or R³ have the meaning mentioned under a), the other radical R¹ or R³ is
1) a hydrogen atom,
2) (C₁-C₆)-alkyl, straight-chain or branched,
3) (C₂-C₆)-alkyl, straight-chain or branched, where the carbon chain is interrupted by 1 or 2 oxygen atoms and 2 oxygen atoms bonded to one another are excluded,
4) (C₁-C₈)-alkyl, straight-chain or branched, substituted by
4.1 an oxo group or
4.2 one or two hydroxyl groups,
5) (C₂-C₆)-alkenyl, straight-chain or branched,
6) benzyl,
7) benzyl, mono- to pentasubstituted by
7.1 (C₁-C₄)-alkyl or
7.2 (C₁-C₄)-alkoxy,
8) (C₃-C₆)-cycloalkyl or
9) (C₁-C₄)-alkyl-(C₃-C₆)-cycloalkyl, and
R² is
1) a hydrogen atom,
2) (C₁-C₆)-alkyl, straight-chain or branched,
3) (C₂-C₆)-alkyl, straight-chain or branched, the carbon chain being interrupted by 1 or 2 oxygen atoms and 2 oxygen atoms bonded to one another being excluded,
4) benzyl,
5) benzyl, mono- to pentasubstituted by
5.1 (C₁-C₄)-alkyl or
5.2 (C₁-C₄)-alkoxy,
6) phenyl,
7) (C₃-C₆)-cycloalkyl or
8) (C₁-C₄)-alkyl-(C₃-C₆)-cycloalkyl, and
D is
1) a hydrogen atom or
2) a fluorine, chlorine, bromine or iodine atom,
the compounds diethyl [4-(1,3-dimethylxanthin-7-yl)butyl]phosphonate, 5-(1,3-dimethoxyxanthin-7-yl)pentylphosphonic acid, 4-(1,3-dimethylxanthin-7-yl)butylphosphonic acid and 3-(1,3-dimethylxanthin-7-yl)propyl-phosphonic acid being excluded.

2. A compound of the formula I as claimed in claim 1, wherein A and B are
1) hydroxyl,
2) C₂-alkoxy or
3) methyl.

3. Acompound of the formula I as claimed in claim 1, wherein R² is
1) a hydrogen atom,
2) (C₂-C₆)-alkyl, straight-chain or branched, the carbon chain being interrupted by 1 oxygen atom,
3) benzyl,
4) cyclopropyl or
5) -CH₂-cyclopropyl, steht.

4. A compound of the formula I as claimed in one or more of claims 1 to 3, wherein one of the radicals R¹ or R³ is a radical of the formula XI and the other radical R¹ or R³ is
1) a hydrogen atom,
2) (C₂-C₆)-alkyl, straight-chain or branched, the carbon chain being interrupted by 1 or 2 oxygen atoms and 2 oxygen atoms bonded to one another being excluded,
3) benzyl or
4) -CH₂-cyclopropyl.

5. A process for the preparation of the compound of the formula I as claimed in claim 1, which comprises
A) reacting a compound of the formula III 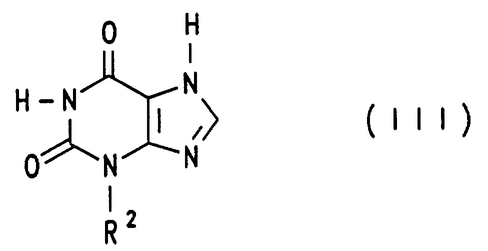 where R² has the meaning mentioned in formula I as claimed in claim 1,
in the presence of basic agents with an alkylating agent of the formula II 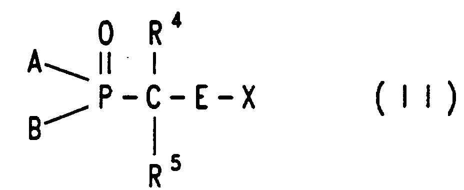 where X is chlorine, bromine, iodine or a sulfonic acid ester radical and R⁴, R⁵, A, B and E have the meaning mentioned in formula XI,
to give a compound of the formula I as claimed in claim 1,
where R¹ is a hydrogen atom and R³ is a radical of the formula XI 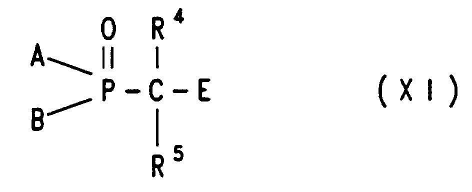 and R² has the meaning mentioned in formula I, or
B) reacting a compound of the formula I prepared according to A) in the presence of basic agents with an alkylating agent of the formula II, where X, R⁴, R⁵, A, B and E are defined as in process. A), to give a compound of the formula I as claimed in claim 1, where R¹ and R³ are identical or different and are a radical of the formula XI, or
C) reacting a compound of the formula I prepared according to A) in the presence of basic agents with a compound R⁶-X, where
R⁶ is
1) a hydrogen atom,
2) (C₁-C₆)-alkyl, straight-chain or branched,
3) (C₂-C₆)-alkyl, straight-chain or branched, where the carbon chain is interrupted by 1 or 2 oxygen atoms and 2 oxygen atoms bonded to one another are excluded,
4) (C₁-C₆)-alkyl, straight-chain or branched, substituted by
4.1 an oxo group or
4.2 one or two hydroxyl groups,
5) (C₂-C₆)-alkenyl, straight-chain or branched,
6) benzyl,
7) benzyl, mono- to pentasubstituted by
7.1 (C₁-C₄)-alkyl or
7.2 (C₁-C₄)-alkoxy,
8) (C₃-C₆)-cycloalkyl or
9) (C₁-C₄)-alkyl- (C₃-C₆)-cycloalkyl, and
X has the meaning mentioned under A),
to give a compound of the formula I as claimed in claim 1, where
R¹ has the meaning of R⁶, R² is defined according to the compound of the formula I and R³ has the meaning of the formula II, or
D) reacting a compound of the formula IV 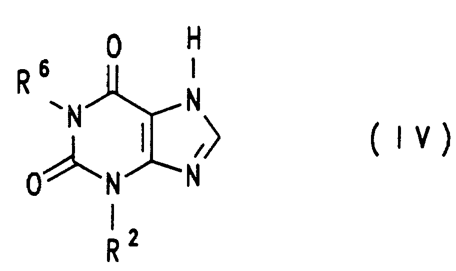 where R⁶ has the meaning mentioned under C) and R² has the meaning mentioned in formula I, in the presence of basic agents with an alkylating agent of the formula II to give a compound of the formula I, where
R¹ has the meaning of R⁶, and R² has the meaning mentioned in formula I and R³ has the meaning mentioned in formula XI, or
E) reacting a compound of the formula V 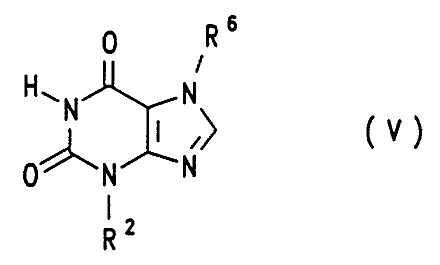 where R⁶ has the meaning mentioned under C) and R² has the meaning mentioned in formula I, in the presence of basic agents with an alkylating agent of the formula II to give a compound of the formula I, where R¹ has the meaning mentioned in formula XI, R² has the meaning mentioned in formula I and R³ has the meaning of R⁶, or
F) in a compound of the formula I, where at least one R¹, R² and R³ is a benzyl, alkoxymethyl or alkoxyalkoxymethyl radical and at least one R¹ and R³ is a radical of the formula XI, removing the benzyl, alkoxymethyl or alkoxyalkoxymethyl radical from the formula I under reducing or hydrolytic conditions, or
G) reacting a compound of the formula VI 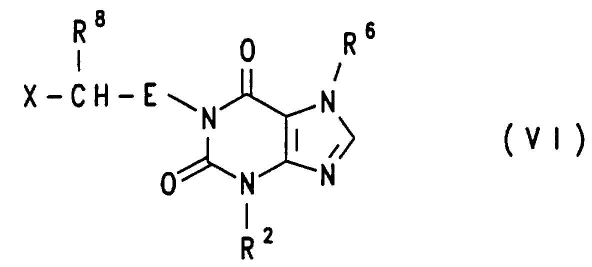 where
R⁶ has the meaning mentioned under C),
R² has the meaning mentioned in formula I,
E has the meaning mentioned in formula XI,
R⁸ is a hydrogen atom or (C₁-C₄)-alkyl and
X is chlorine, bromine, iodine or a sulfonic acid ester radical,
in the presence of a strong base such as sodium hydride, butyllithium or lithium diisopropylamide in an inert solvent with a compound of the formula VIII 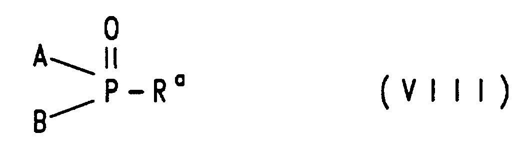 where A and B have the meaning mentioned in formula I and
R^{a} is a hydrogen atom or (C₁-C₄)-alkyl, to give a compound of the formula I, where R¹ has the meaning mentioned in formula XI, R² has the meaning mentioned in formula I and R³ has the meaning of R⁶, or
H) reacting a compound of the formula VI, where R², R⁶, R⁸, E and X have the meaning mentioned under G) with a compound of the formula IX
P(OR¹⁰)₃ (IX)
where P is phosphorus, O is oxygen and R¹⁰ is a (C₁-C₄)-alkyl, to give a compound of the formula I, where R¹ has the meaning mentioned in formula XI, R² has the meaning mentioned in formula I and R³ has the meaning of R⁶, or
I) converting a compound of the formula I where R¹, R², R³, E and D have the meaning mentioned in formula I and A and B are (C₁-C₄)-alkyl or (C₁-C₄)-alkoxy, with agents such as mineral acids or silyl halides into the corresponding phosphonic acid derivatives of the formula I, or
K) converting a compound of the formula I, where R¹, R², R³, E and D have the meaning mentioned in formula I and A or B is a benzyloxy group, selectively into the corresponding phosphonic acid hemiesters, or
L) halogenating a compound of the formula I, where R¹, R², R³, E, A and B have the meaning mentioned in formula I and D is a hydrogen atom, a corresponding compound of the formula I being formed in which D is fluorine, chlorine, bromine or iodine, or
M) isolating the compound of the formula I prepared according to processes A) to L) either in free form or, in the case of the presence of acidic or basic groups, optionally converting it into physiologically tolerable salts.

6. A pharmaceutical, comprising a compound of the formula I, as claimed in one or more of claims 1 to 4, the compounds diethyl [4-(1,3-dimethylxanthin-7-yl)butyl]phosphonate and 4-(1,3-dimethylxanthin-7-yl)-butylphosphonic acid being included.

7. The use of at least one compound of the formula I as claimed in one or more of claims 1 to 4, the compounds diethyl [4-(1,3-di-methylxanthin-7-yl)-butyl]phosphonate and 4-(1,3-dimethylxanthin-7-yl)butyl-phosphonic acid being included for the production of a pharmaceutical for the prophylaxis or therapy of muscular atrophy, muscular dystrophy, cachexia, sepsis, septic shock, endotoxic shock, systemic inflammation response syndrome, adult respiratory distress syndrome, cerebral malaria, chronic pneumonia, pulmonary sarcoidosis, reperfusion damage, scar formation, inflammation of the bowel and ulcerative colitis, as a result of infections, (AIDS), cancer, trauma and other disorders having increased protein loss, peripheral circulatory disorders, disorders having altered leucocyte adhesion, and also disorders which are associated with an increased or unregulated tumor necrosis factor production, such as rheumatoid arthritis, rheumatoid spondylitis, osteoarthritis and other arthritic disorders.

8. A process for the production of a pharmaceutical as claimed in claim 6, wherein at least one compound of the formula I as claimed in claim 1 and/or at least one physiologically tolerable salt of a compound of the formula I and/or an optionally stereoisomeric form of the compound of the formula I and/or a compound obtained by the process as claimed in claim 5, the compounds diethyl [4-(1,3-dimethylxanthin-7-yl)butyl]phosphonate and 4-(1,3-dimethyl-xanthin-7-yl)butylphosphonic acid being included, are brought into a suitable administration form with physiologically acceptable auxiliaries and excipients and, if appropriate, further additives and/or other active compounds.

## Revendications

1. Composé de formule I 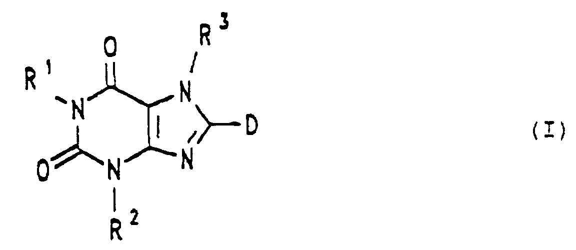 et/ou un sel toléré sur le plan physiologique du composé de formule I et/ou éventuellement une forme stéréoisomère du composé de formule I, où
R¹ et R³ sont identiques ou différents et au moins l'un des restes R¹ et R³ représente
a) un reste de formule XI 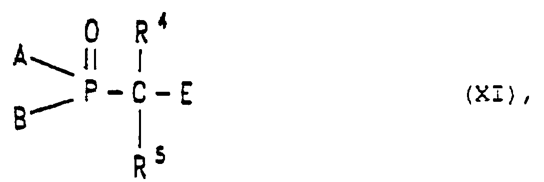 où R⁴ et R⁵ sont identiques ou différents et représentent indépendamment l'un de l'autre
1.1 un atome d'hydrogène,
1.2 un groupe hydroxyle ou
1.3 un groupe alkyle en C₁-C₆,
A et B sont identiques ou différents et représentent indépendamment l'un de l'autre
2.1 un grupe alkyle en C₁-C₄,
2.2 un groupe alkoxy en C₁-C₆,
2.3 un groupe hydroxyle ou
2.4 un groupe benzyloxy,
E représente une liaison covalente ou un groupe alkyle linéaire ou ramifié ayant 1 à 5 atomes de carbone, et
b) dans le cas où l'un des restes R¹ ou R³ possède la signification donnée au point a), l'autre reste R¹ ou R³ représente
1) un atome d'hydrogène,
2) un groupe alkyle en C₁-C₆ linéaire ou ramifié,
3) un groupe alkyle en C₂-C₆ linéaire ou ramifié, la chaîne carbonée étant interrompue par 1 ou 2 atomes d'oxygène et 2 atomes d'oxygène liés l'un à l'autre sont exclus,
4) un groupe alkyle en C₁-C₈ linéaire ou ramifié, substitué par
4.1 un groupe oxo ou
4.2 un ou deux restes hydroxyle,
5) un groupe alcényle en C₂-C₆ linéaire ou ramifié,
6) un groupe benzyle,
7) un groupe benzyle, substitué une à cinq fois par des substituants
7.1 alkyle en C₁-C₄ ou
7.2 alkoxy en C₁-C₄,
8) un groupe cycloalkyle en C₃-C₆ ou
9) un groupe (alkyl en C₁-C₄)-cycloalkyle en C₃-C₆, et
R² représente
1) un atome d'hydrogène,
2) un groupe alkyle en C₁-C₆ linéaire ou ramifié,
3) un groupe alkyle en C₂-C₆ linéaire ou ramifié, la chaîne carbonée étant interrompue par 1 ou 2 atomes d'oxygène et 2 atomes d'oxygène lies l'un à l'autre sont exclus,
4) un groupe benzyle,
5) un groupe benzyle substitué une à cinq fois par des substituants
5.1 alkyle en C₁-C₄ ou
5.2 alkoxy en C₁-C₄,
6) un groupe phényle,
7) un groupe cycloalkyle en C₃-C₆, ou
8) un groupe (alkyl en C₁-C₄)-cycloalkyle en C₃-C₆, et
D représente
1) un atome d'hydrogène ou
2) un atome de fluor, de chlore, de brome ou d'iode,
les composés le [4-(1,3-diméthylxanthin-7-yl)-butyl]phosphonate de diéthyle, l'acide 5-(1,3-diméthoxyxanthin-7-yl)pentylphosphonique, l'acide 4-(1,3-diméthyLxanthin-7-yl)butylphosphonique et l'acide 3-(1,3-diméthylxanthin-7-yl)propyl-phosphonique étant exclus.

2. Composé de formule I selon la revendication 1, **caractérisé en ce que** A et B représentent des groupes
1) hydroxyle,
2) alkoxy en C₂ ou
3) méthyle.

3. Composé de formule I selon la revendication 1, **caractérisé en ce que** R² représente
1) un atome d'hydrogène,
2) un groupe alkyle en C₂-C₆ linéaire ou ramifié, la chaîne carbonée étant interrompue par 1 atome d'oxygène ,
3) un groupe benzyle,
4) un groupe cyclopropyle ou
5) un groupe CH₂-cyclopropyle.

4. Composé de formule I selon l'une ou plusieurs des revendications 1 à 3, **caractérisé en ce que** l'un des restes R¹ ou R³ représente un reste de formule XI et l'autre reste R¹ ou R³ représente
1) un atome d'hydrogène,
2) un groupe alkyle en C₂-C₆ linéaire ou ramifié, la chaîne carbonée étant interrompue par 1 ou 2 atomes d'oxygène et 2 atomes d'oxygène liés l'un à l'autre étant exclus,
3) un groupe benzyle,
4) un groupe CH₂-cyclopropyle.

5. Procédé pour la préparation du composé de formule I selon la revendication 1, **caractérisé en ce qu'**on fait réagir
A) un composé de formule III, 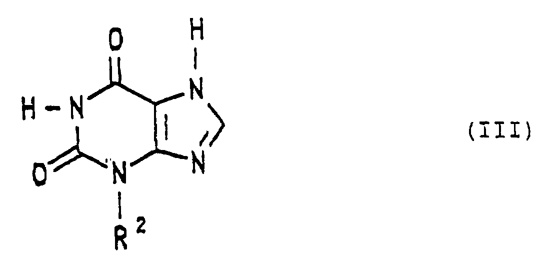 où R² à la formule I possède la signification selon la revendication 1,
en présence d'un agent basique avec un agent d'alkylation de formule II 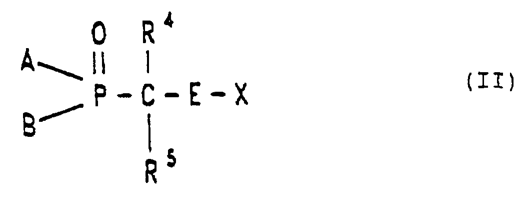 où
X représente un atome de chlore, de brome d'iode ou un reste acide sulfonique et
R⁴, R⁵, A, B, et E possèdent la signification donnée à la formule IX,
pour obtenir un composé de formule I selon la revendication 1, où R¹ représente un atome d'hydrogène et R³ représente un reste de formule XI 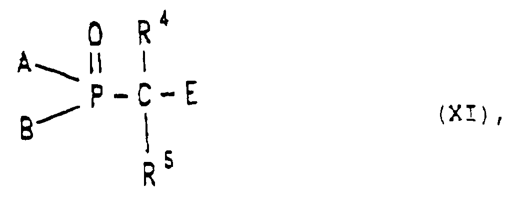 et R² possède la signification donnée à la formule I, ou
B) un composé de formule I préparé selon A) en présence d'un agent basique avec un agent d'alkylation de formule II, X, R⁴, R⁵, A, B et E étant définis comme au procédé A), pour obtenir un composé de formule I selon la revendication 1, R¹ et R³ étant identiques ou différents et représentent un reste de formule XI,
C) un composé de formule I préparé selon A) en présence d'agents basiques avec un composé de formule R⁶-X, où
R⁶ représente
1) un atome d'hydrogène,
2) un groupe alkyle en C₁-C₆ linéaire ou ramifié,
3) un groupe alkyle en C₂-C₆ linéaire ou ramifié, la chaîne carbonée étant interrompue par 1 ou 2 atomes d'oxygène et 2 atomes d'oxygène liés l'un à l'autre sont exclus,
4) un groupe alkyle en C₁-C₆ linéaire ou ramifié, substitué par
4.1 un groupe oxo ou
4.2 un ou deux groupes hydroxyle,
5) un groupe alcényle en C₂-C₆ linéaire ou ramifié,
6) un groupe benzyle,
7) un groupe benzyle, substitué une à cinq fois par des substituants
7.1 alkyle en C₁-C₄ ou
7.2 alkoxy en C₁-C₄,
8) un groupe cycloalkyle en C₃-C₆ ou
9) un groupe (alkyl en C₁-C₄)-cycloalkyle en C₃-C₆, et
X possède la signification donnée au point A), pour obtenir un composé de formule I selon la revendication 1, où
R¹ possède la signification de R⁶, R² est défini selon le composé de formule I et R³ possède la signification de formule II, ou
D) un composé de formule IV 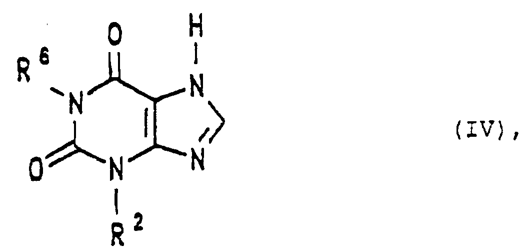 où R⁶ possède la signification donnée au point C) et R² possède la signification donnée à la formule I, en présence d'agents basiques avec un agent d'alkylation de formule II pour obtenir un composé de formule I, où
R¹ possède la signification de R⁶, R² possède la signification donnée à la formule I et R³ possède la signification donnée à la formule XI, ou on fait réagir
E) un composé de formule V 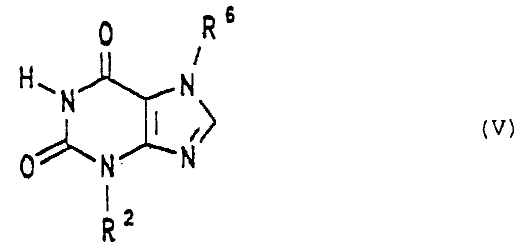 où R⁶ possède la signification donnée au point C) et R² possède la signification donnée à la formule I, en présence d'un agent basique avec un agent d'alkylation de formule II pour obtenir un composé de formule I, où R¹ possède la signification donnée à la formule XI, R² possède la signification donnée à la formule I et R³ possède la signification de R⁶, ou on fait réagir
F) un composé de formule I, au moins un R¹, R² et R³ représente un reste benzyle, alkoxyméthyle ou alkoxyalkoxyméthyle et au moins un R¹ et R³ représente un reste de formule XI, en séparant, sous conditions réductrices ou hydrolytiques, le reste benzyle, alkoxyméthyle ou alkoxyalkoxyméthyle de formule I, ou on fait réagir
G) un composé de formule VI 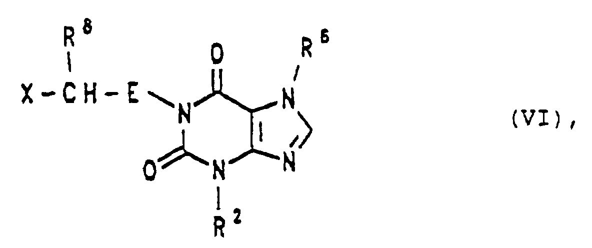 où
R⁶ possède la signification donnée au point C),
R² possède la signification donnée à la formule I,
E possède la signification donnée à la formule XI,
R⁸ représente un atome d'hydrogène ou un groupe alkyle en C₁-C₄ et
X représente un atome de chlore, de brome, d'iode ou un reste ester d'acide sulfonique,
en présence d'une base forte comme l'hydrure de sodium, le butyllithium ou le diisopropylamidure de lithium dans un solvant inerte, avec un composé de formule VIII 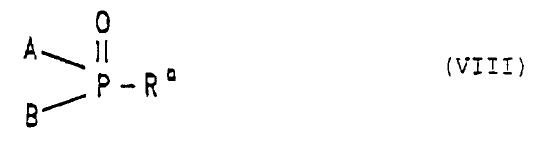 où A et B possèdent la signification donnée à la formule I et R^{a} représente un atome d'hydrogène ou un groupe alkyle en C₁-C₄, pour obtenir un composé de formule I, R¹ ayant la signification donnée à la formule XI, R² possède la signification donnée à la formule I et R³ possède la sifgnification de R⁶, ou on fait réagir
H) un composé de formule VI, où R², R⁶, R⁸, E et X possèdent la signification donnée au point G) avec un composé de formule IX,
P(OR¹⁰)₃ (IX),
où P est un atome de phosphore, O est un atome d'oxygène et R¹⁰ représente un groupe alkyle en C₁-C₄, pour donner un composé de formule I, où R¹ possède la signification donnée à la formule XI, R² possède la signification donnée à la formule I et R³ possède la signification de R⁶, ou on fait transforme
I) un composé de formule I, où R¹, R², R³, E et D possèdent la signification donnée à la formule I et A et B représentent un groupe alkyle en C₁-C₄, ou alkoxy en C₁-C₄, à l'aide d'agents tels que des acides minéraux ou des halogénures de silyle pour obtenir les dérivés de l'acide phosphonique correspondants de formule I, ou on transforme
K) un composé de formule I, où R¹, R², R³, E et D possèdent la signification donnée à la formule I et A et B représentent un groupe benzyloxy, sélectivement, en l'hémi-ester de l'acide phosphonique, ou on halogène
L) un composé de formule I, où R¹, R², R³, E, A et B possèdent la signification donnée à la formule I et D représente un atome d'hydrogène, en obtenant un composé correspondant de formule I, où D représente un atome de fluor, de chlore, de brome cu d'iode, ou
M) le composé de formule I préparé selon les procédés A) à L) est soit isolé sous forme libre, soit, en cas de présence de groupes acides ou basiques, éventuellement est transformé en sels tolérés sur le plan physiologique.

6. Produit thérapeutique contenant un composé de formule I selon une ou plusieurs des revendications 1 à 4, les composés le [4-(1,3-diméthylxanthin-7-yl)-butyl]-phosphonate de diéthyle et l'acide 4-(1,3-diméthylxanthin-7-yl)butyl-phosphonique étant inclus.

7. Utilisation d'au moins un composé de formule I selon une ou plusieurs des revendications 1 à 4, les composés le [4-(1,3-diméthylxanthin-7-yl)-butyl]-phosphonate de diéthyle et l'acide 4-(1,3-diméthylxanthin-7-yl)butylphosphonique étant inclus, pour la préparation d'un produit thérapeutique pour la prévention ou la thérapie de l'athrophie musculaire, de la dystrophie musculaire, de la cachexie, de la septicémie, du choc septique, du choc endotoxinique, du syndrome de réponse d'inflammation systémique, du syndrome de détresse respiratoire de l'adulte, de la malarie cérébrale, de la pneumonie chronique, de la sarcoïdose pulmonaire, des dommages provoqués par des ré-perfusions, de la formation de cicatrices, des inflammations intestinales, de la colite ulcéreuse, de la cachexie, suite aux infections, du SIDA, du cancer, du traumatisme et d'autres maladies associées à une perte élevée en protéines, aux troubles d'irrigation sanguine périphérique, de maladies associées aux troubles d'adhésion de leucocytes, ainsi que de maladies associées à une production élevée ou irrégulière du facteur de necrose de tumeur, telles que l'arthrite rheumatoïde, la spondylite rheumatoïde, l'ostéoarthrite et autres maladies arthritiques.

8. Procédé pour la préparation d'un produit thérapeutique selon la revendication 6, **caractérisé en ce qu'**on met en forme d'administration appropriée au moins un composé de formule I selon la revendication 1 et/ou au moins un sel toléré sur le plan physiologique du composé de formule I et/ou éventuellement d'une forme stéréoisomère du composé de formule I et/ou un composé obtenu selon le procédé de la revendication 5, les composés le [4-(1,3-diméthylxanthin-7-yl)-butyl]-phosphonate de diéthyle et l'acide 4-(1,3-diméthylxanthin-7-yl)-butyl-phosphonique étant inclus, avec des adjuvants et véhicules tolérés sur le plan physiologique et éventuellement avec d'autres matières d'addition et/ou d'autres principes actifs.
